# EUROPÄISCHE PATENTSCHRIFT

(11) **EP 0 749 466 B1**
(45) Veröffentlichungstag und Bekanntmachung des Hinweises auf die Patenterteilung: **12.11.1997**
(21) Anmeldenummer: 95911272.3
(22) Anmeldetag: 27.02.1995
(51) Int. Cl.: C09K 19/38

(54) **NEUE POLYMERISIERBARE FLÜSSIGKRISTALLINE VERBINDUNGEN**
NOVEL POLYMERISABLE LIQUID-CRYSTALLINE COMPOUNDS
NOUVEAUX COMPOSES DE CRISTAUX LIQUIDES POLYMERISABLES

(30) Priorität: 11.03.1994 DE 4408171
(43) Veröffentlichungstag der Anmeldung: 27.12.1996
(73) Patentinhaber: BASF AKTIENGESELLSCHAFT, 67056 Ludwigshafen (DE)
(72) Erfinder: SIEMENSMEYER, Karl, D-67227 Frankenthal (DE); ETZBACH, Karl-Heinz, D-67227 Frankenthal (DE); DELAVIER, Paul, D-67061 Ludwigshafen (DE); MEYER, Frank, D-67063 Ludwigshafen (DE)
(86) Internationale Anmeldenummer: EP9500707
(87) Internationale Veröffentlichungsnummer: WO9524454

(56) Entgegenhaltungen:
- EP-A- 0 331 233
- EP-A- 0 451 905
- EP-A- 0 512 308
- WO-A-93/22397
- DE-A- 2 831 909
- STN International, File CA, Chemical abstract, volume 121, no. 18, 31 Oct 1994, abstract no. 217783, JP, A2, 06018864, (SATO, KOICHI ET AL) 28 Jan 1994
- STN International, File CA, Chemical abstract, volume 119, no.26, 27 Dec 1993, (Columbus Ohio, US),Geibel, Kurt et al:"In situ photopolymerized, oriented liquid-crystalline diacrylates with high thermal conductivities". Abstract no. 271752 Adv. Mater. (Weinheim, Fed. Repub. Ger.) (1993), 5(2), 107-9
- STN International, File CA, Chemical abstract, volume 116, no.16, 20 Apr 1992, (Columbus Ohio, US),Hikmet, R.A.M. et al: "Anisotropic polymerization shrinkage behavior of liquid-crystalline diacrylates". Abstract no. 153029 Polymer (1992), 33(1), 89-95
- Angew. Chem., Band 99, 1987, Von Heino Finkelmann, "FlBssigkristalline Polymere" Seite 840 - Seite 848, Siehe Tabelle 2 Verbindung (22)

## Beschreibung

Wie für formanisotrope Medien bekannt, können beim Erwärmen flüssigkristalline Phasen, sogenannte Mesophasen, auftreten. Die einzelnen Phasen unterscheiden sich durch die räumliche Anordnung der Molekülschwerpunkte einerseits sowie durch die Molekülanordnung hinsichtlich der Längsachsen andererseits (G.W. Gray, P.A. Winsor, Liquid Crystals and Plastic Crystals, Ellis Horwood Limited, Chichester 1974). Die nematisch flüssigkristalline Phase zeichnet sich dadurch aus, daß lediglich eine Orientierungsfernordnung durch Parallellagerung der Moleküllängsachsen existiert. Unter der Voraussetzung, daß die die nematische Phase aufbauenden Moleküle chiral sind, entsteht eine sogenannte cholesterische Phase, bei der die Längsachsen der Moleküle eine zu ihnen senkrechte, helixartige Überstruktur ausbilden (H. Baessler, Festkörperprobleme XI, 1971). Der chirale Molekülteil kann sowohl im flüssigkristallinen Molekül selbst vorhanden sein als auch als Dotierstoff zur nematischen Phase gegeben werden, wobei die cholesterische Phase induziert wird. Dieses Phänomen wurde zuerst an Cholesterolderivaten untersucht (z.B. H. Baessler, M.M. Labes, J. Chem. Phys.. 52, 631 (1970); H. Baessler, T.M. Laronge, M.M. Labes, J. Chem. Phys., 51 799 (1969); H. Finkelmann, H. Stegemeyer, Z. Naturforschg. 28a, 799 (1973); H. Stegemeyer, K.J. Mainusch, Naturwiss., 58, 599 (1971), H. Finkelmann, H. Stegemeyer, Ber. Bunsenges. Phys. Chem. 78, 869 (1974)).

Die cholesterische Phase hat bemerkenswerte optische Eigenschaften: eine hohe optische Rotation sowie einen ausgeprägten Zirkulardichroismus, der durch Selektivreflexion von zirkular polarisiertem Licht innerhalb der cholesterischen Schicht entsteht. Die je nach Blickwinkel unterschiedlich erscheinenden Farben sind abhängig von der Ganghöhe der helixartigen Überstruktur, die ihrerseits vom Verdrillungsvermögen der chiralen Komponente abhängt. Dabei kann insbesondere durch Änderung der Konzentration eines chiralen Dotierstoffes die Ganghöhe und damit der Wellenlängenbereich des selektiv reflektierten Lichtes einer cholesterischen Schicht variiert werden. Solche cholesterischen Systeme bieten für eine praktische Anwendung interessante Möglichkeiten. So kann durch Einbau chiraler Molekülteile in mesogene Acrylsäureester und Orientierung in der cholesterischen Phase, z.B. nach der Photovernetzung, ein stabiles, farbiges Netzwerk hergestellt werden, dessen Konzentration an chiraler Komponente dann aber nicht mehr verändert werden kann (G. Galli, M. Laus, A. Angelon, Makromol. Chemie, 187, 289 (1986)). Durch Zumischen von nichtvernetzbaren chiralen Verbindungen zu nematischen Acrylsäureestern kann durch Photovernetzung ein farbiges Polymer hergestellt werden, welches noch hohe Anteile löslicher Komponenten enthält (I. Heyndricks, D.J. Broer, Mol. Cryst. Liq. Cryst. 203, 113 (1991)). Weiterhin kann durch statistische Hydrosilylierung von Gemischen aus Cholesterolderivaten und acrylathaltigen Mesogenen mit definierten zyklischen Siloxanen und anschließende Photopolymerisation ein cholesterisches Netzwerk gewonnen werden, bei dem die chirale Komponente einen Anteil von bis zu 50 % an dem eingesetzten Material haben kann; diese Polymerisate enthalten jedoch noch deutliche Mengen löslicher Anteile (F.H. Kreuzer, R. Maurer, Ch. Müller-Rees, J. Stohrer, Vortrag Nr. 7, 22. Freiburger Arbeitstagung Flüssigkristalle, Freiburg, 1993).

In der Anmeldung DE-OS-35 35 547 wird ein Verfahren beschrieben, bei dem eine Mischung cholesterolhaltiger Monoacrylate über eine Photovernetzung zu cholesterischen Schichten verarbeitet werden kann. Allerdings beträgt der Gesamtanteil der chiralen Komponente in der Mischung ca. 94 %. Als reines Seitenkettenpolymer ist ein solches Material zwar mechanisch nicht sehr stabil, eine Erhöhung der Stabilität kann aber durch hochvernetzende Verdünnungsmittel erreicht werden.

In EP-A 331 233 werden Mischungen flüssigkristalliner Verbindungen beschrieben, die symmetrisch mit aliphatischen Seitenketten substituiert sind.

Neben oben beschriebenen nematischen und cholesterischen Netzwerken sind auch smektische Netzwerke bekannt, welche insbesondere durch Photopolymerisation/Photovernetzung von smektisch flüssigkristallinen Materialien in der smektisch flüssigkristallinen Phase hergestellt werden. Die hierfür verwendeten Materialien sind in der Regel symmetrische, flüssigkristalline Bisacrylate, wie sie z.B. D.J. Broer und R.A.M. Hikmet, Makromol. Chem., 190, 3201-3215 (1989) beschrieben haben. Diese Materialien weisen aber sehr hohe Klärtemperaturen von > 120°C auf, so daß die Gefahr einer thermischen Polymerisation gegeben ist. Durch Zumischen chiraler Materialien können beim Vorliegen einer S_{c}-Phase piezoelektrische Eigenschaften erzielt werden (R.A.M. Hikmet, Macromolecules 25, S. 5759, 1992).

Aufgabe der vorliegenden Erfindung war die Herstellung neuer polymerisierbarer nematisch flüssigkristalliner Materialien, die allein oder in Mischungen mit anderen polymerisierbaren nematischen Flüssigkristallen breite nematische Phasenbereiche und Klärtemperaturen unterhalb 120°C aufweisen und die unterhalb von 120°C verarbeitet werden können.

Diese Aufgabe wird erfindungsgemäß durch die flüssigkristallinen Mischungen gelöst, die mindestens zwei verschiedene Verbindungen der allgemeinen Formel I 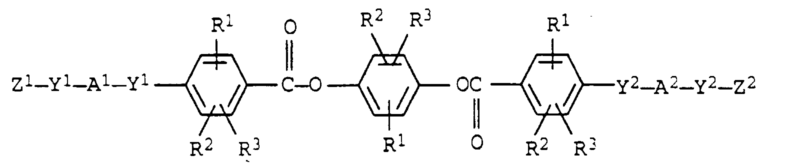 enthalten, in der die Reste
- Z¹, Z²: unabhängig voneinander eine polymerisierbare Gruppe,
- Y¹, Y²: jeweils unabhängig voneinander eine direkte Bindung, -O-, -COO-, -OCO- oder -S-,
- A¹, A²: unabhängig voneinander einen Spacer und
- R¹ , R² und R³: unabhängig voneinander Wasserstoff, C₁- bis C₂₀-Alkyl, C₁- bis C₂₀-Alkoxy, C₁- bis C₂₀-Alkoxycarbonyl, C₁- bis C₂₀-Monoalkylaminocarbonyl, Formyl, C₁- bis C₂₀-Alkylcarbonyl, Fluor, Chlor, Brom, Cyan, C₁- bis C₂₀-Alkylcarbonyloxy, C₁- bis C₂₀-Alkylcarbonylamino, Hydroxy oder Nitro bedeuten, mit der Maßgabe, daß bei mindestens einer der Substanzen I die Gruppen -A¹-Y¹- und -A²-Y²⁻ verschiedene O-alkylen-Reste sind.

Bevorzugte Gruppen Z¹ und Z² sind solche, die durch einen photochemischen Initiierungsschritt polymerisiert werden können, also insbesondere Gruppen der Struktur: CH₂=CH-, CH₂=CCl-, CH₂=C(CH₃)-oder 4-Vinylphenylyl. Bevorzugt sind CH₂=CH-, CH₂=CCl- und CH₂=C(CH₃)-, wobei CH₂=CH- und CH₂=C(CH₃)- besonders bevorzugt sind.

Für Y¹ und Y² sind neben einer direkten Bedeutung insbesondere Ether- und Estergruppen zu nennen. Als Spacer A¹ und A² können alle für diesen Zweck bekannten Gruppen verwendet werden. Üblicherweise sind die Spacer über Ester- oder Ethergruppen oder eine direkte Bindung mit Z verknüpft. Die Spacer enthalten in der Regel 0 bis 30, vorzugsweise 2 bis 12 C-Atome und können in der Kette z.B. durch O, S, NH oder NCH₃ unterbrochen sein. Als Substituenten für die Spacerkette kommen dabei noch Fluor, Chlor, Brom, Cyan, Methyl oder Ethyl in Betracht. Repräsentative Spacer sind beispielsweise (CH₂)ₚ, (CH₂CH₂O)_{q}CH₂CH₂, (CH₂CH₂S)_{q}CH₂CH₂, (CH₂CH₂NH)_{q}CH₂CH₂, 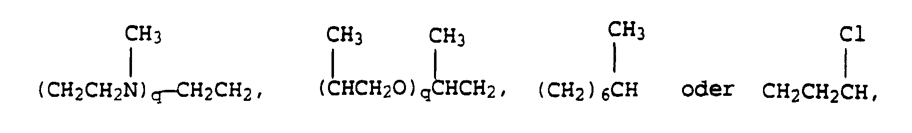 wobei q 1 bis 3 und p 1 bis 12 sind.

Die Reste R¹, R² und R³ in den Mischungskomponenten der Formel I können Wasserstoff oder Substituenten der in Anspruch 1 angegebenen Art sein. Bevorzugt sind solche Reste, die die Ausbildung von smektischen Phasen unterdrücken und die von nematischen Phasen fördern. Vorzugsweise ist einer der Reste R Wasserstoff und insbesondere sollen zwei Reste R Wasserstoff sein. Von den genannten Resten sind Chlor, Brom, Cyan, Fluor, Hydroxy, Methyl, Ethyl, Methoxy, Ethoxy, Methoxycarbonyl, Formyl, Acetyl und Acetoxy sowie längerkettige mit ≧ 8 C-Atomen bevorzugt.

Der Molekülteil 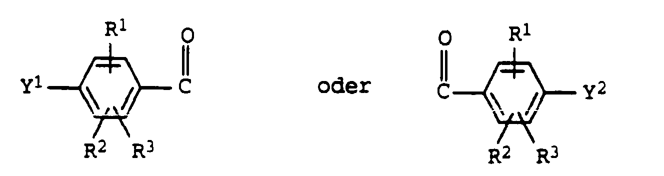 der Verbindungen in den Mischungen gemäß der Formel I in Anspruch 1 hat unabhängig voneinander bevorzugt eine der folgenden Strukturen: 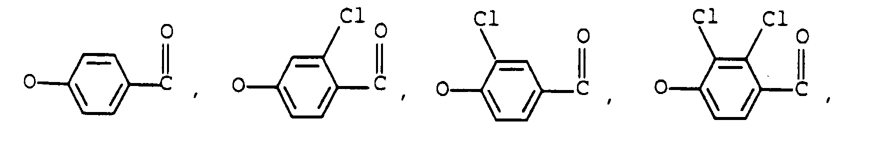 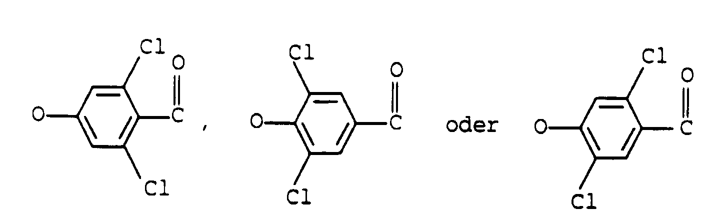 oder analog mit F, Br, CH₃, OCH₃, CHO, COCH₃, OCOCH₃ oder CN anstelle von Cl, wobei die Substituenten auch gemischt vorliegen können. Ferner sind die Strukturen 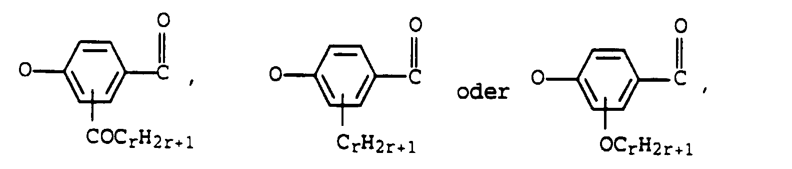 zu nennen, bei denen r 2 bis 20, vorzugsweise 8 bis 15, bedeutet.

Der Molekülteil 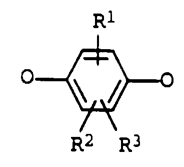 in Formel I entspricht vorzugsweise den Formeln: 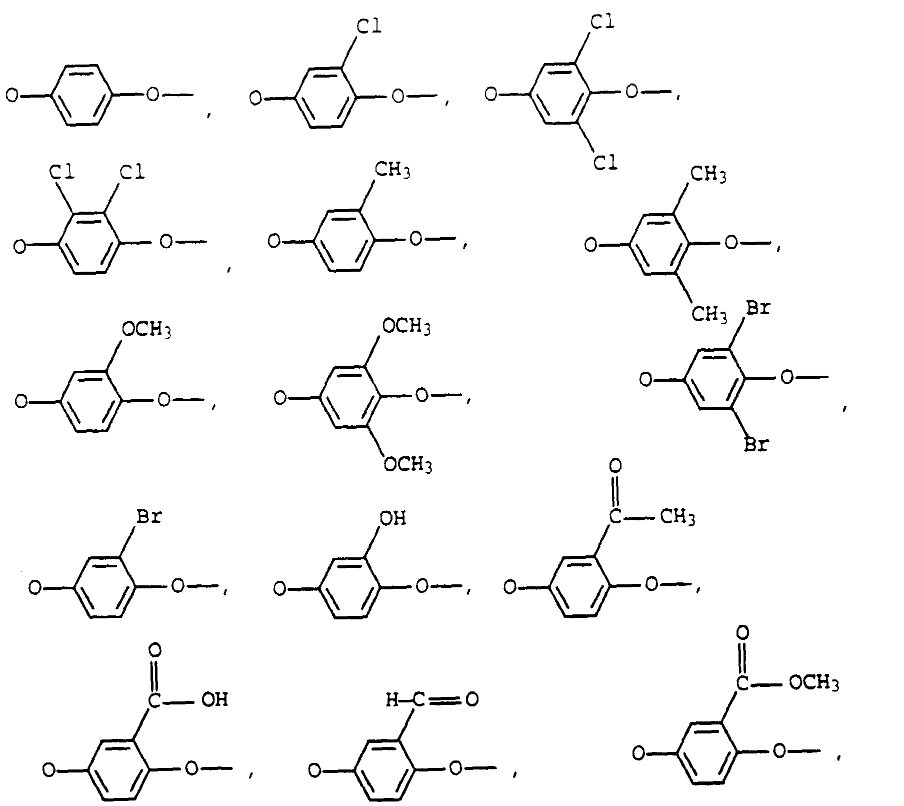 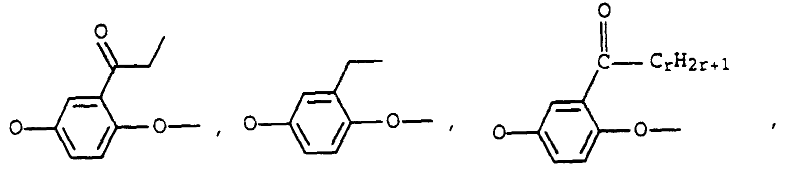 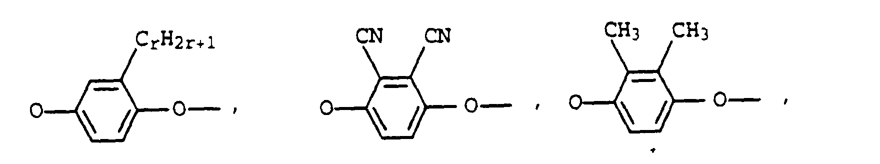 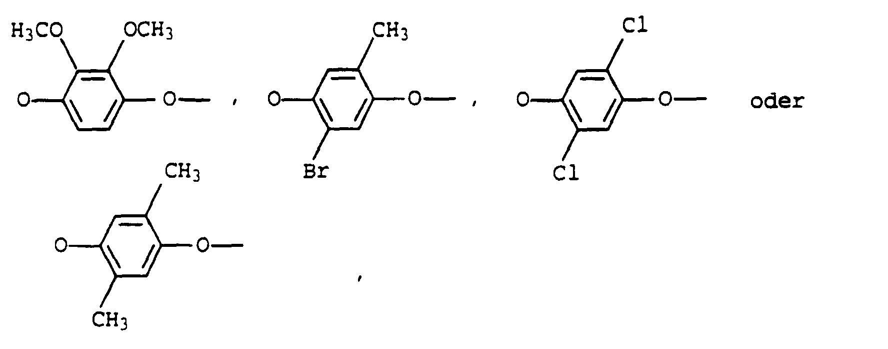 wobei r die Zahlen 2 bis 20, vorzugsweise 8 bis 15, bedeutet.

Besonders gut als Komponenten für die erfindungsgemäßen Mischungen eignen sich die neuen Verbindungen gemäß Anspruch 9. Durch das überraschend unterschiedliche Kristallisationsverhalten der Einzelkomponenten wird der flüssigkristalline Zustandsbereich der Mischungen gemäß Anspruch 1 deutlich erweitert.

Die Herstellung der Mischungen sowie der Verbindungen der Formel T ist nach an sich bekannten Methoden möglich. Einzelheiten der Herstellung können den Beispielen entnommen werden. Die Verbindungen der Formel I sind flüssigkristallin und können in Abhängigkeit von der Struktur smektische oder nematische Phasen ausbilden. Die erfindungsgemäßen Verbindungen und Mischungen sind für alle Zwecke geeignet, bei denen man üblicherweise flüssigkristalline Verbindungen verwendet.

Die erfindungsgemäßen Mischungen und Verbindungen weisen allein, in Mischungen untereinander oder mit anderen flüssigkristallinen Verbindungen Phasenstrukturen wie niedermolekulare Flüssigkristalle auf, lassen sich jedoch durch radikalische oder ionische Polymerisationsverfahren in hochvernetzte Polymere mit eingefrorener flüssigkristalliner Ordnungsstruktur überführen.

Zur Einstellung gewünschter Eigenschaften der Mischungen kann es zweckmäßig sein, mehr als zwei Verbindungen der Formel I oder auch Kombinationen erfindungsgemäßer Mischungen mit anderen polymerisierbaren Flüssigkristallen zu verwenden, wobei solche Mischungen durch mechanisches Mischen hergestellt werden können. Das Anpassen der Phasenzustandsbereiche ist insbesondere auch durch Zusatz von nicht flüssigkristallinen polymerisierbaren Komponenten, sogenannten Reaktivverdünnern, wie beispielsweise Hexandioldiacrylat oder Bisphenol-A-diacrylat möglich. Der Zusatz von Reaktionsverdünnern hat insbesondere auf die Fließviskosität einen günstigen Einfluß. Die erfindungsgemäßen Mischungen eignen sich insbesondere als Orientierungsschichten für flüssigkristalline Materialien, als photovernetzbare Kleber, als Monomere zur Herstellung flüssigkristalliner Netzwerke, als Basismaterial zur Herstellung von chiral dotierbaren polymerisierbaren Flüssigkristallsystemen, als polymerisierbare Matrixmonomere für polymer dispergierte Displays, als Basismaterial für polymerisierbare, flüssigkristalline Materialien für optische Bauelemente wie z.B. Polarisatoren, Verzögerungsplatten, Linsen und, chiral dotiert, als Basismaterial für Farbeffekt- und Piezomaterialien.

Die Erfindung betrifft weiterhin neue Verbindungen der Formel I, in der R¹ bis R³, Z¹, Z², Y¹, Y², A¹ und A² die angegebene Bedeutung haben und die Gruppen -A¹-Y¹- und Y²-A² nicht gleichzeitig gleiche -O-alkylen-Reste sind.

### Beispiele

Im folgenden seien einige, in den Beispielen durchgängig benutzte Abkürzungen aufgeführt:
- k: kristalline Phase
- n: nematische Phase
- ch: cholesterische Phase
- s: smektische Phase (nicht näher charakterisiert)
- i: isotrope Phase

Die Phasenumwandlungstemperaturen wurden polarisationsmikroskopisch bestimmt. Die Temperaturkontrolle erfolgte in einem Mettler Mikroskopheiztisch FP 80/82.

Die als Komponenten der Mischungen verwendeten Monomeren weisen alle polymerisationsfähige Gruppen auf. Die Polymerisation kann bei diesen Materialien photochemisch, durch übliche Radikalbildner oder auch thermisch gestartet werden. Bei der Herstellung der Mischungen ist daher darauf zu achten, daß noch keine Polymerisation eintritt.

Allgemeine Vorschrift zur Herstellung der nematischen oder chiral nematischen (cholesterischen) Mischungen:

Die Mischungskomponenten werden in Methylenchlorid gelöst, sodaß eine isotrope Lösung entsteht. Das Methylenchlorid wird dann im Vakuum bei ungefähr 40 bis 60°C entfernt.

### Beispiel 1

### Herstellung von 1-[4'-(4"-Acryloxybutyloxy)-benzoyloxy]-4-[4'-(2"-acryloxyethyloxy)-benzoyloxy]-benzol

4-(4'-Hydroxybutyloxy)-benzoesäure (1):
   Zu einer Lösung von 4-Hydroxybenzoe-säureethylester (249 g; 1,5 mol), Kaliumiodid (3 g) und Kaliumcarbonat (248 g; 1,8 mol) in Dimethylformamid (2 l) wird 4-Chlorbutylacetat (276,6 g; 1,8 mol) gegeben und 11 h bei 90°C gerührt. Die Reaktionsmischung wird auf 5 l Eiswasser gegeben, der ausgefallene Niederschlag abgesaugt und mit 4 bis 5 l Eiswasser gewaschen. Das Rohprodukt wird in 3 l Ethanol gelöst, mit Kaliumhydroxid (400 g) versetzt und 3 h unter Rückfluß erhitzt. Die Reaktionsmischung wird auf 6 l Eiswasser gegeben, mit konzentrierter Salzsäure sauer gestellt und der Niederschlag abfiltriert. Der Niederschlag wird mit Wasser neutral gewaschen und anschließend getrocknet. Ausbeute: 282.1 g (89 %).
4-(4'-Acryloxybutyloxyl-benzoesäure (2):
   Eine Lösung von (1) (282 g; 1,34 mol), frisch destillierte Acrylsäure (230 ml; 3,35 mol), Hydrochinon (1,9 g) und p-Toluolsulfonsäure (23,7 g) in 1,1,1-Trichlorethan (1,1 l) wird 10 h unter Rückfluß erhitzt. Die Reaktionsmischung wird auf 60 bis 70°C abgekühlt, in 2,5 1 Petrolether eingerührt und der Niederschlag abfiltriert. Nach Waschen mit Petrolether wird der Niederschlag bei Raumtemperatur im Vakuum 24 h getrocknet. Ausbeute: 299.3 g (84 %).
4- 4-[4'-(4-Acryloxybutyloxy)-benzoyloxy]-phenol (3) :
   Oxalylchlorid (10 ml) wird bei 0°C zu (2) (5 g; 19 mmol) gegeben und solange gerührt, bis die Gasentwicklung abklingt (20 min). Das überschüssige Oxalylchlorid wird im Wasserstrahlvakuum abdestilliert, das Säurechlorid in Toluol (10 ml) aufgenommen und bei 0°C zu einer Lösung von Hydrochinon (10,5 g; 95 mmol) in Pyridin (10 ml) und Toluol (5 ml) gegeben und anschließend 24 h bei Raumtemperatur gerührt. Die Reaktionsmischung wird dann auf Wasser (200 ml) gegeben und zweimal mit Dichlormethan (je 50 ml) extrahiert. Die organischen Extrakte werden mit Calciumchlorid getrocknet, das Filtrat mit Hydrochinon (50 mg) versetzt und im Wasserstrahlvakuum eingeengt. Der Rückstand wird an Kieselgel (Toluol/Essigester (5 : 1) chromatographiert und ergibt 4,5 g (66 %) (3).
1[4'-(4"-Acryloxybutyloxy)-benzoyloxy]-4-[4'(2"-acryloxyethyloxy)-benzoyloxy]-benzol (4):
   Oxalylchlorid (10 ml) wird bei 0°C zu 4-(2'-Acryloxyethyloxy)-benzoesäure (4,3 g; 16,3 mmol) gegeben und solange gerührt, bis die Gasentwicklung abklingt (30 min). Das überschüssige Oxalylchlorid wird im Wasserstrahlvakuum abdestilliert, das Säurechlorid in Toluol (10 ml) aufgenommen und bei 0°C zu einer Lösung von (3) (5,8 g; 16,3 mmol) in Pyridin (10 ml) und Toluol (5 ml) gegeben und anschließend 13 h bei Raumtemperatur gerührt. Die Reaktionsmischung wird dann auf Wasser (200 ml) gegeben und zweimal mit Dichlormethan (je 50 ml) extrahiert. Die organischen Extrakte werden mit Calciumchlorid getrocknet, das Filtrat mit Hydrochinon (50 mg) versetzt und im Wasserstrahlvakuum eingeengt. Der Rückstand wird an Kieselgel (Toluol/Essigester 5 : 1) chromatographiert und ergibt 7,8 g (83 %) (4).

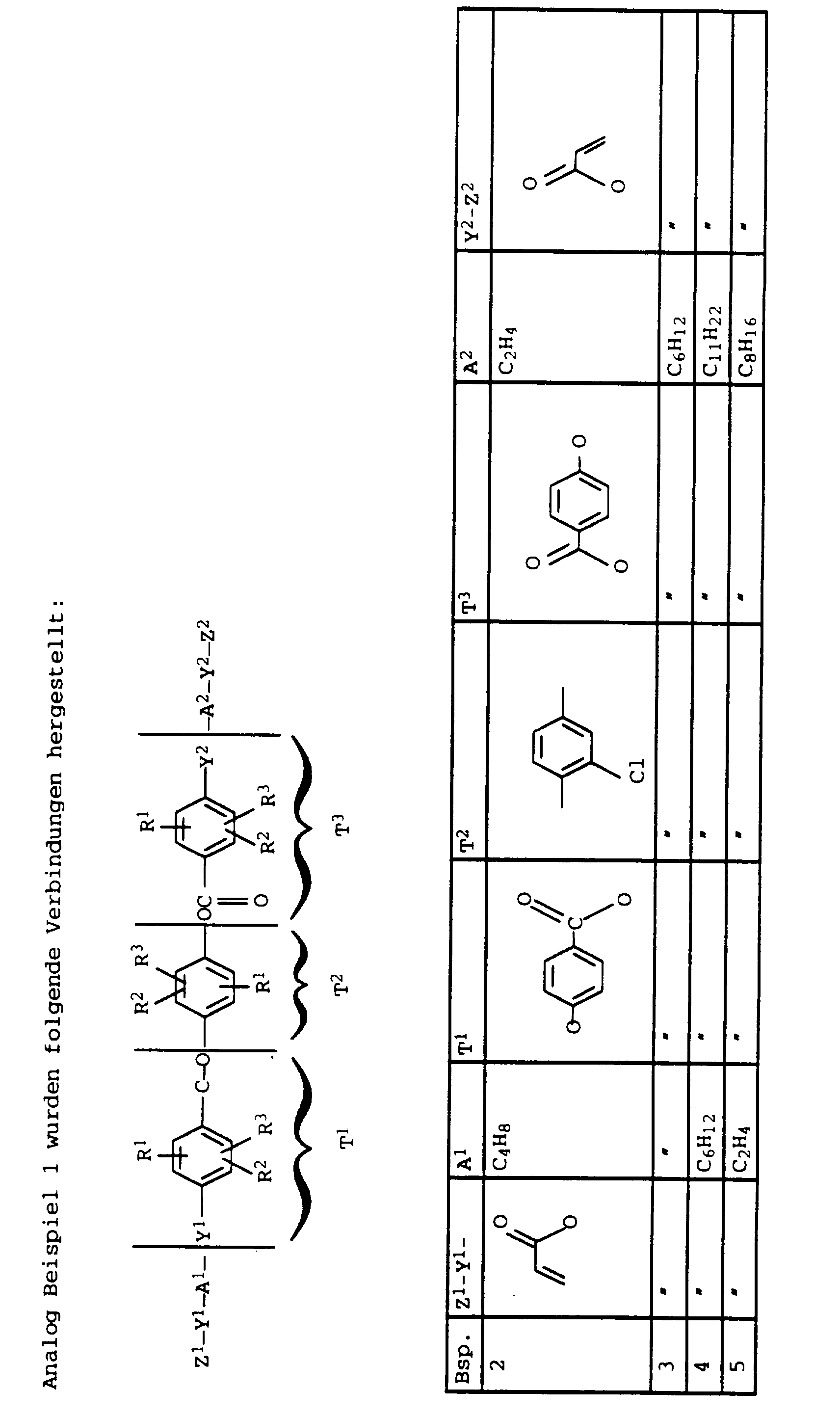

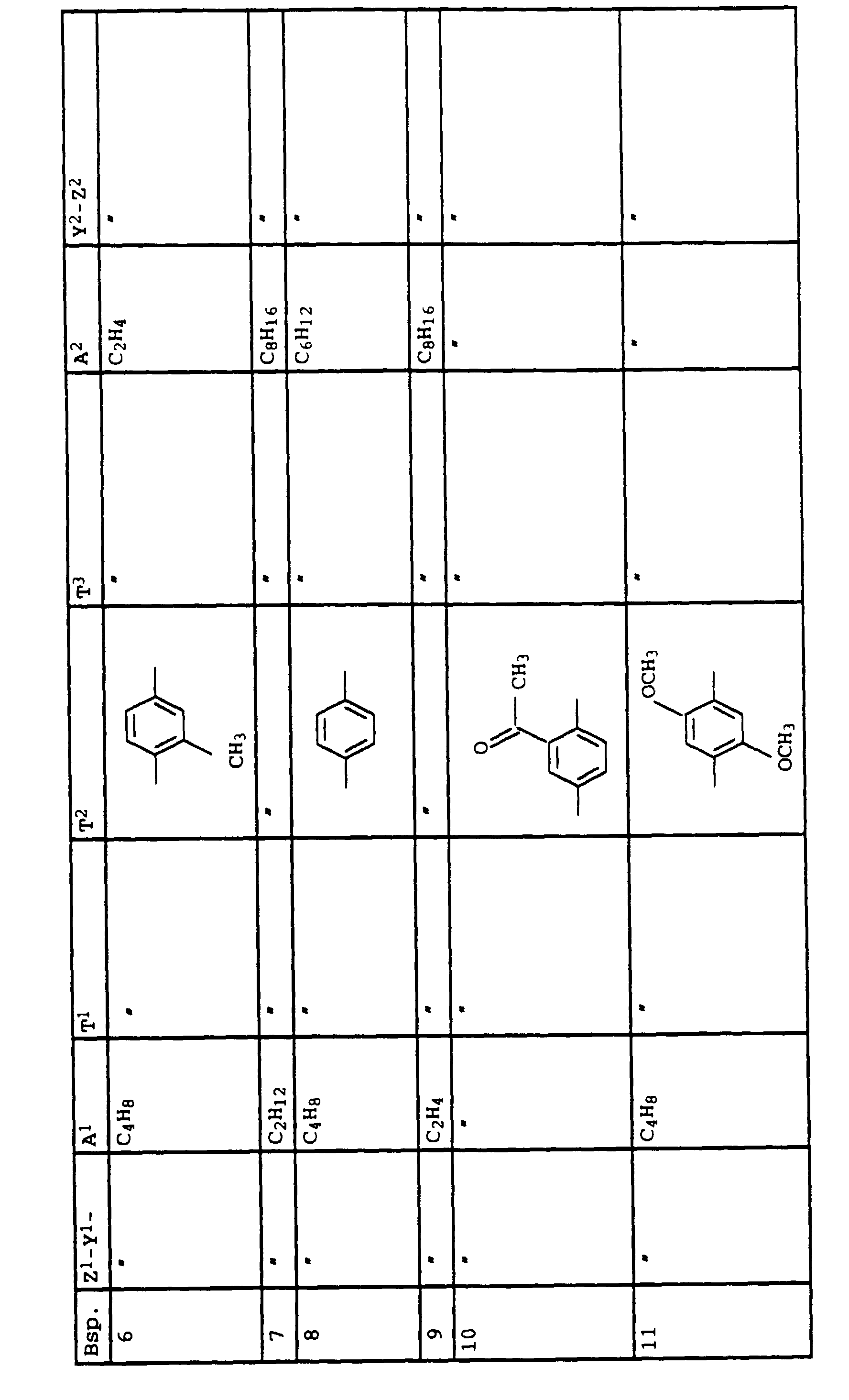

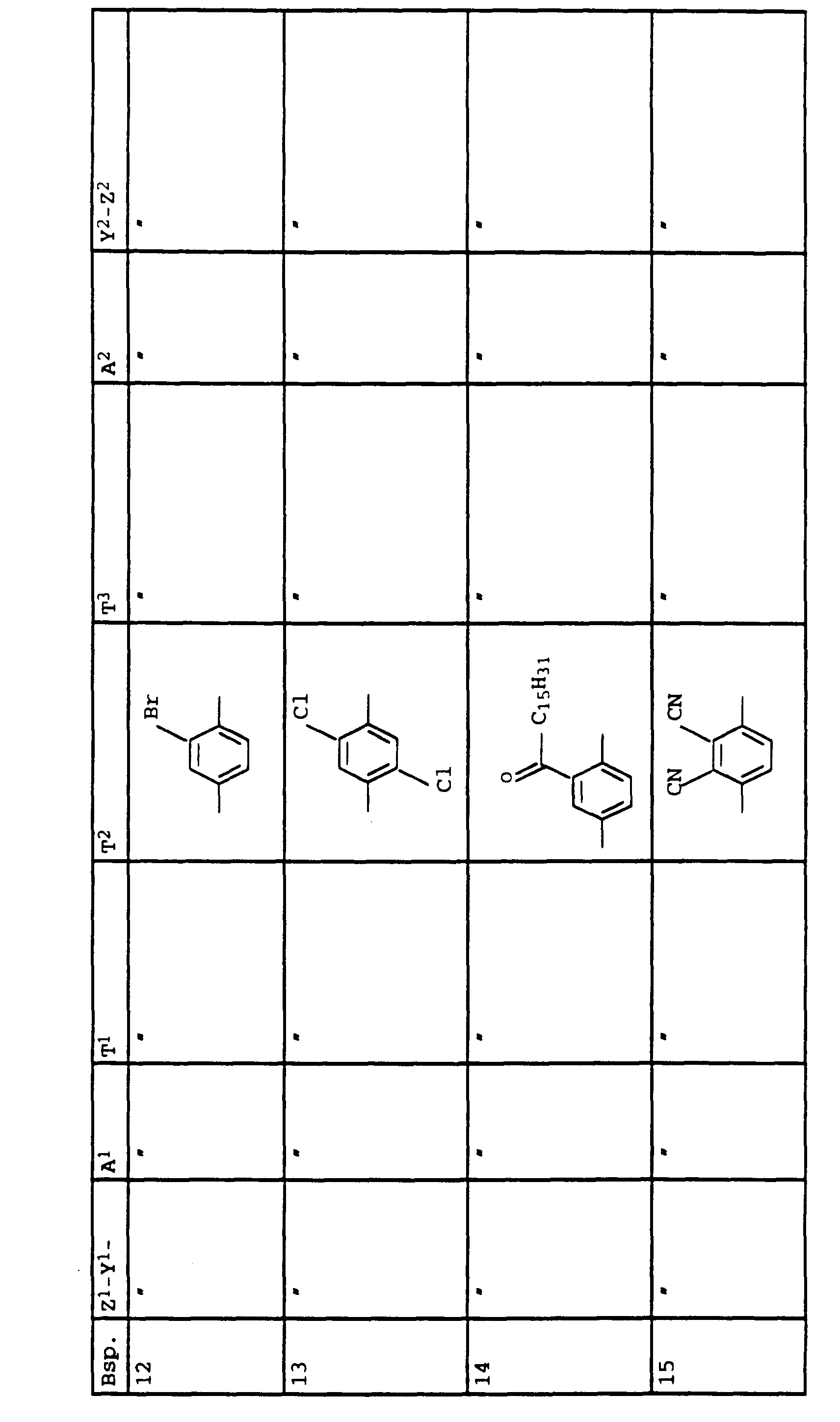

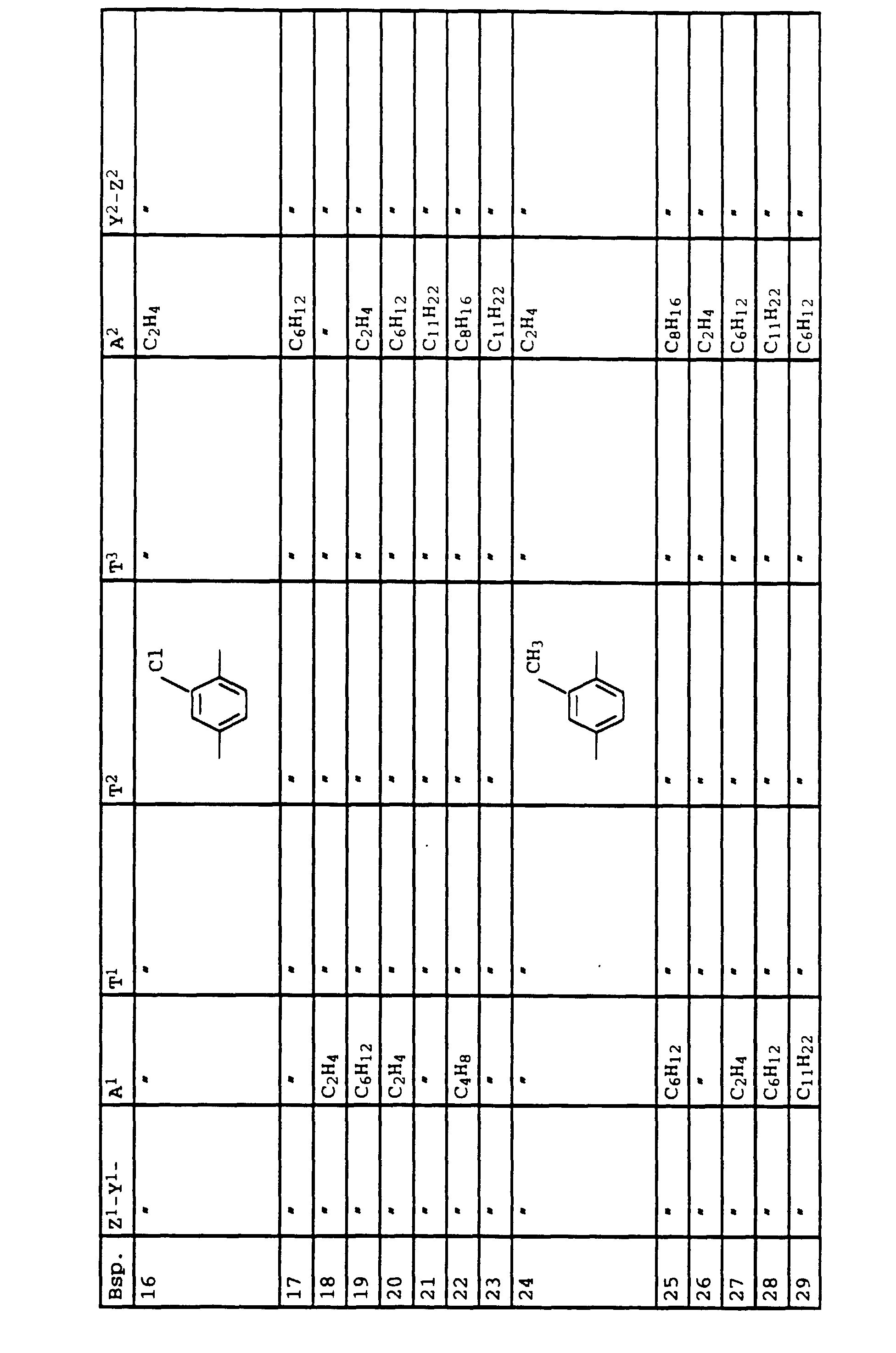

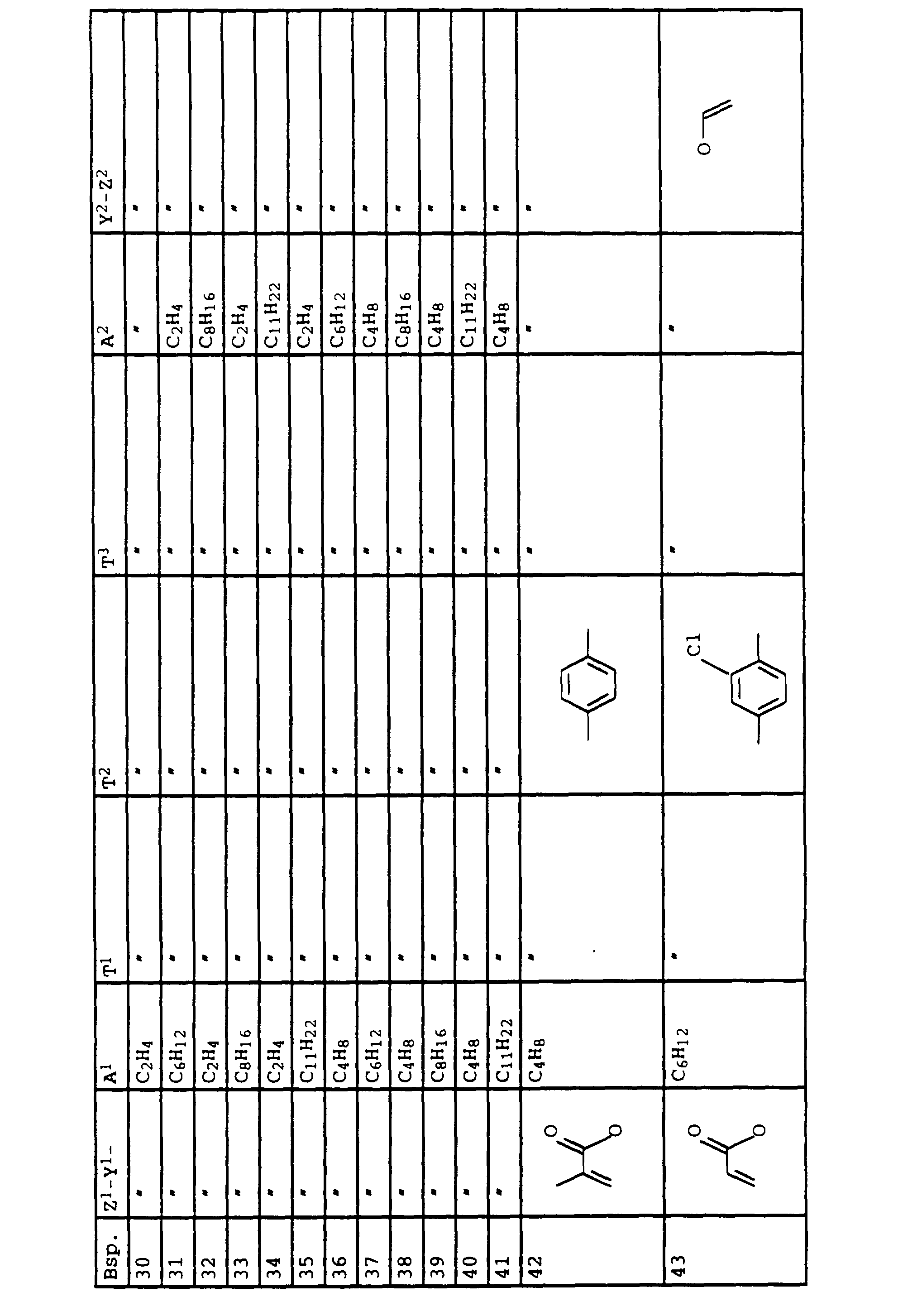

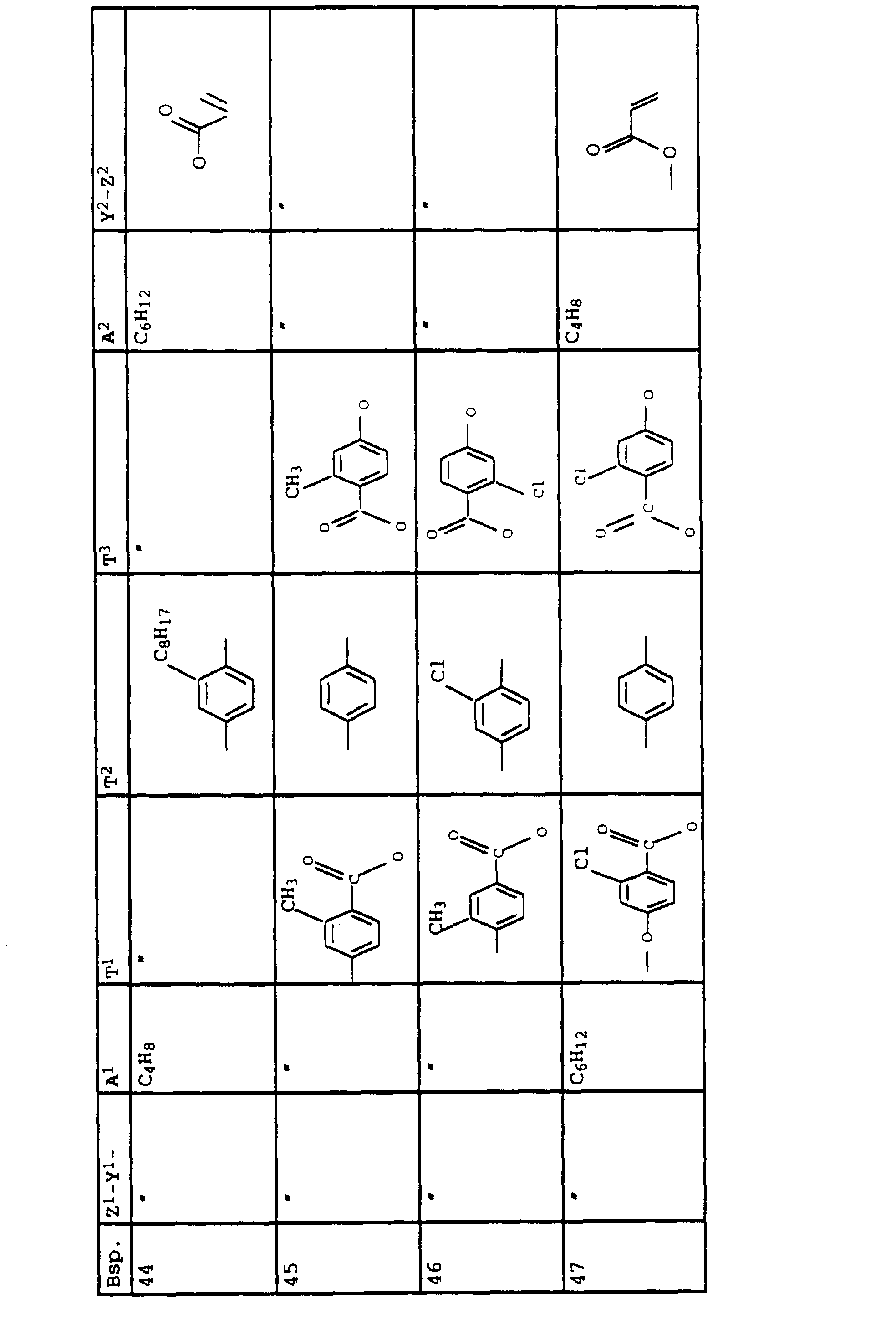

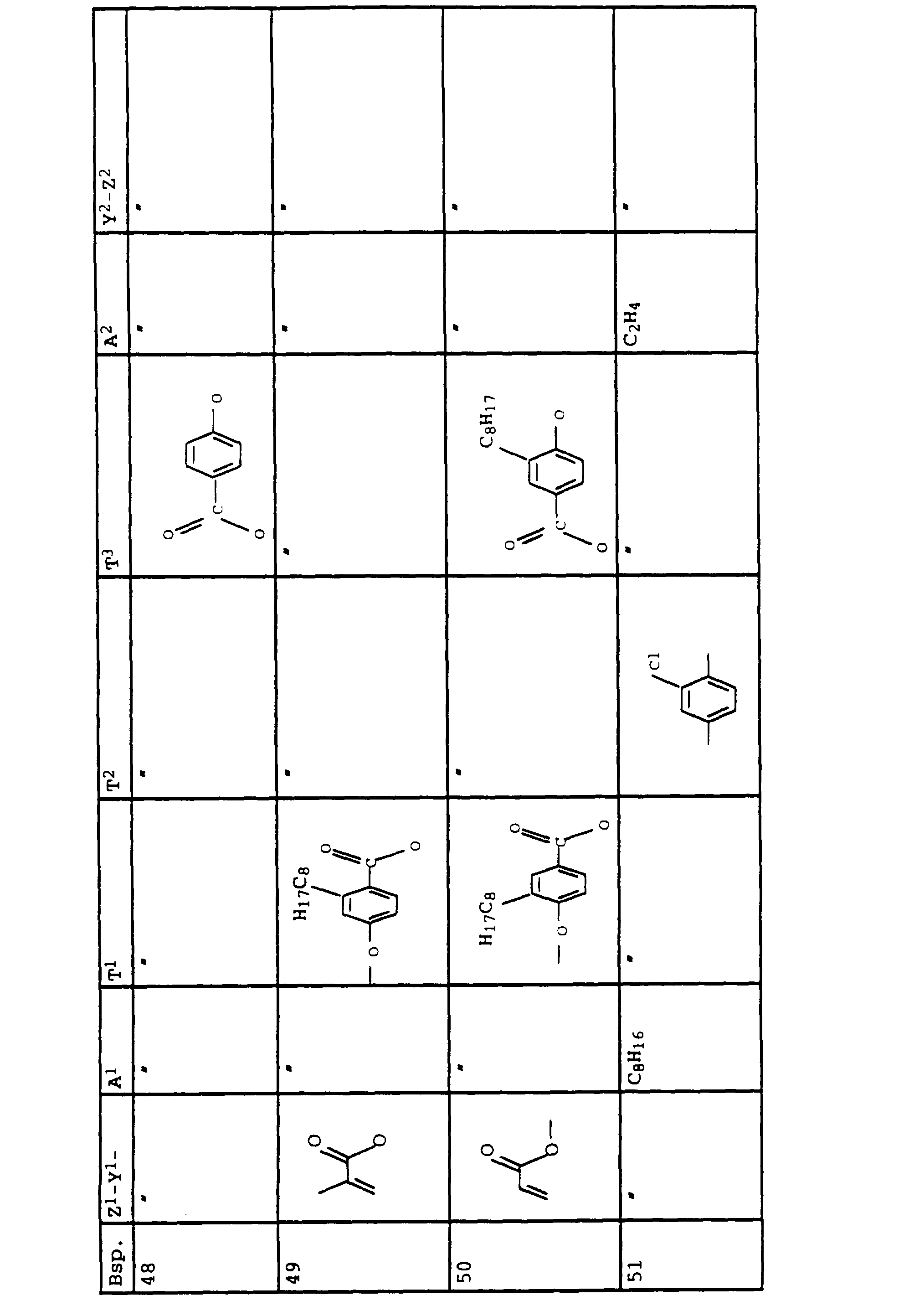

### Beispiel 52

### Mischung 1:

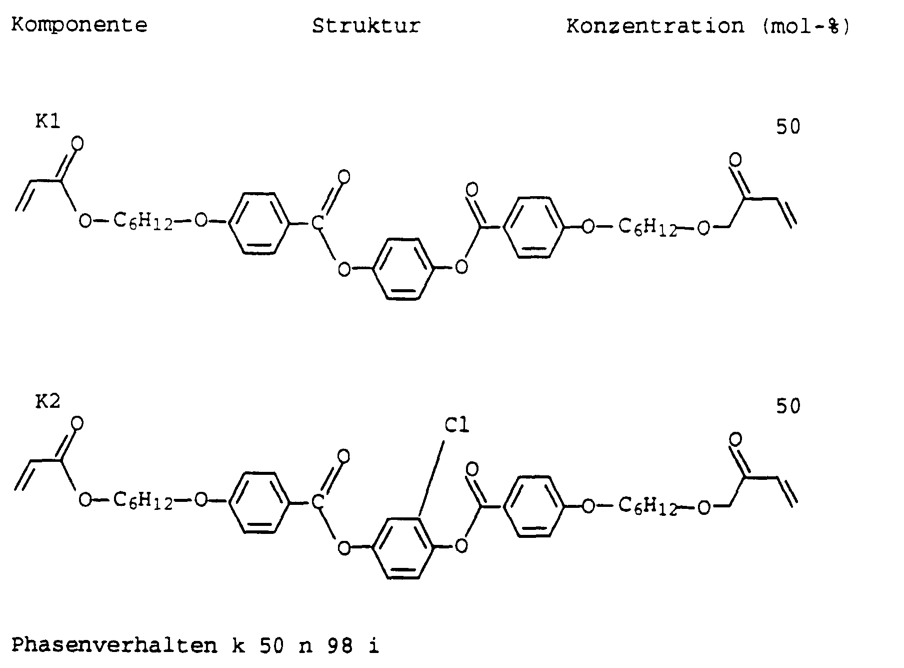

### Beispiel 53

### Mischung 2:

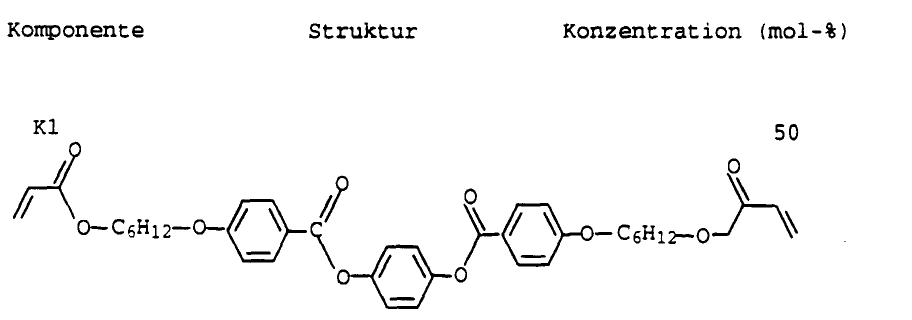

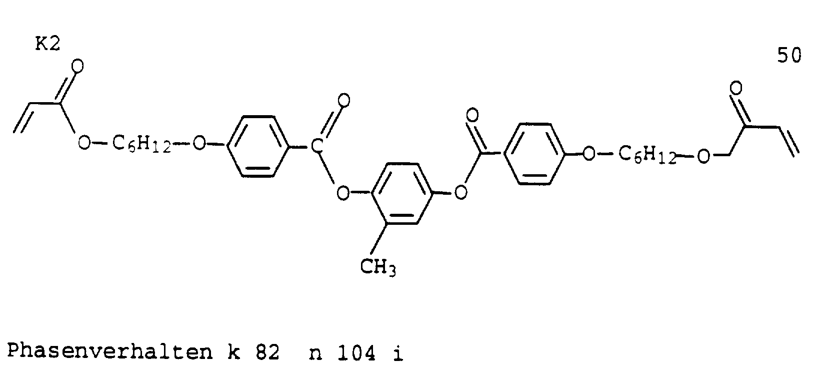

### Beispiel 54

### Mischung 3:

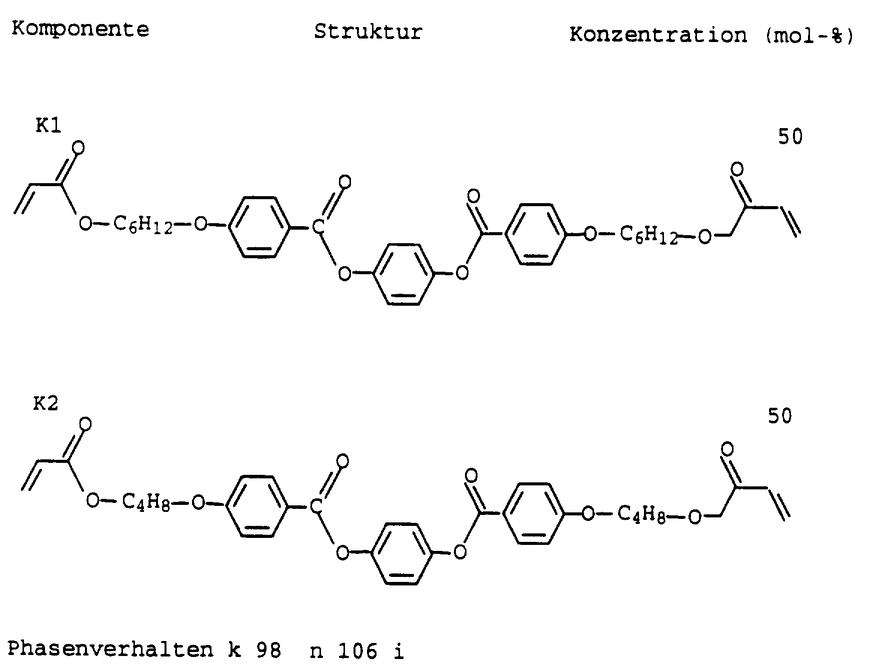

### Beispiel 55

### Mischung 4:

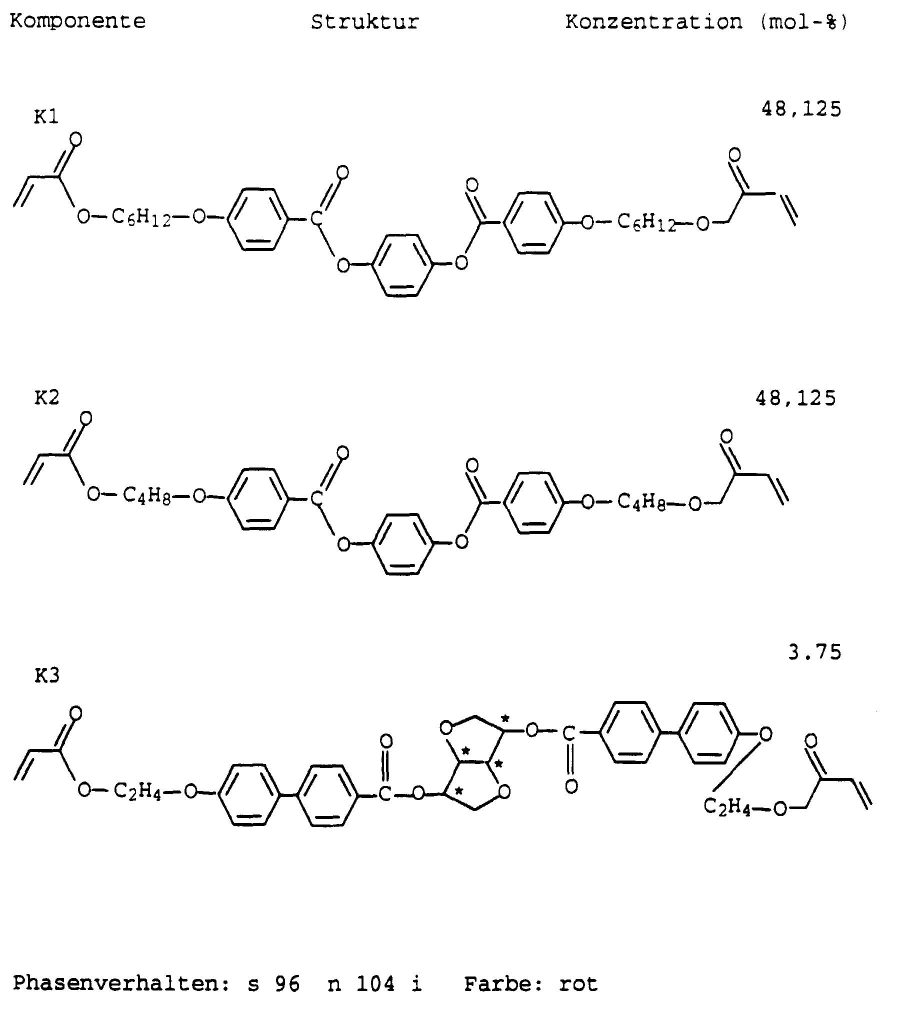

### Beispiel 56

### Mischung 5:

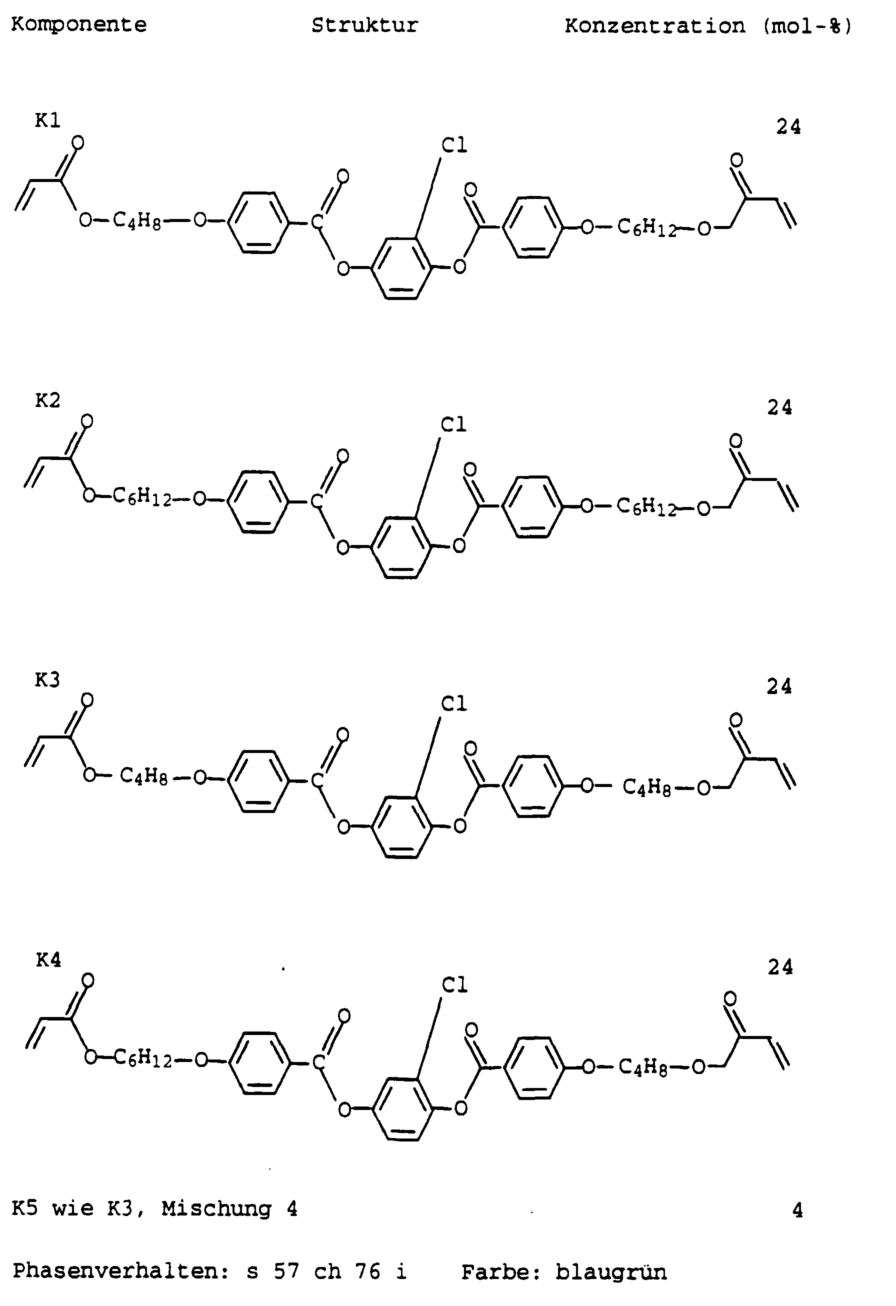

### Beispiel 57

### Mischung 6:

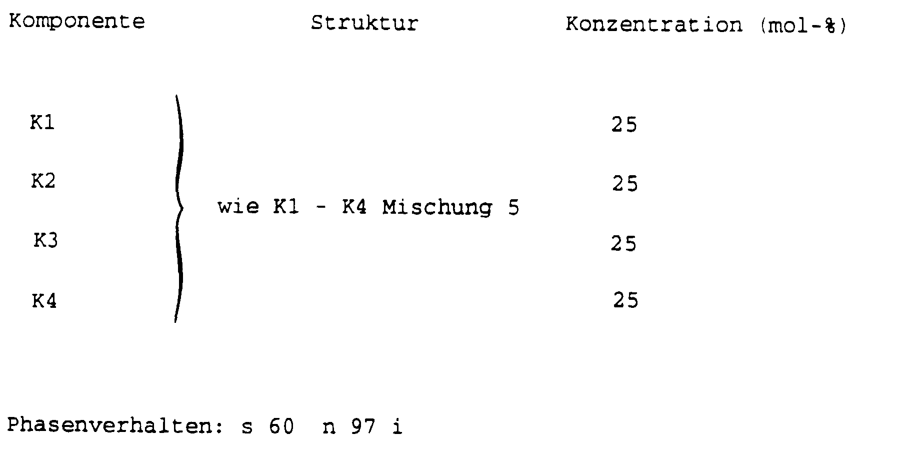

### Beispiel 58

### Mischung 7:

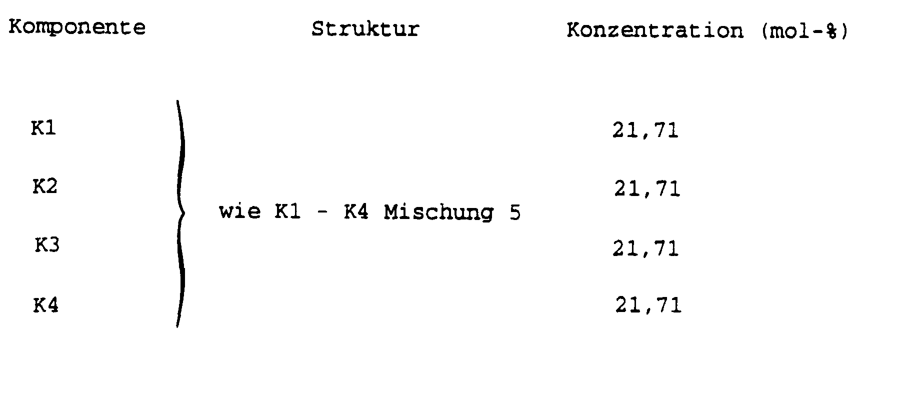

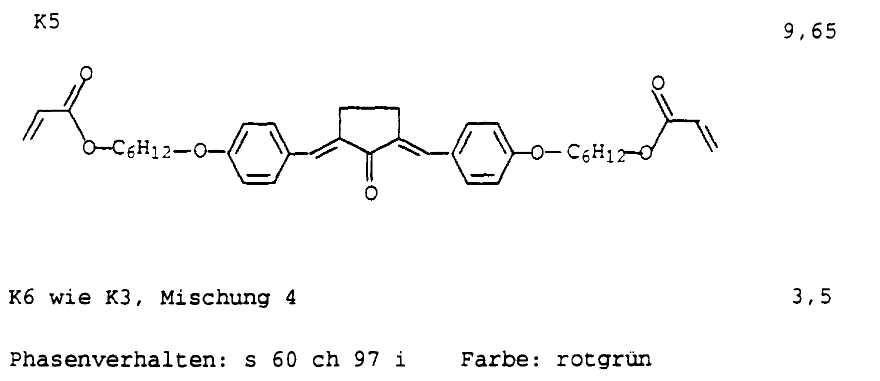

### Beispiel 59

### Mischung 8:

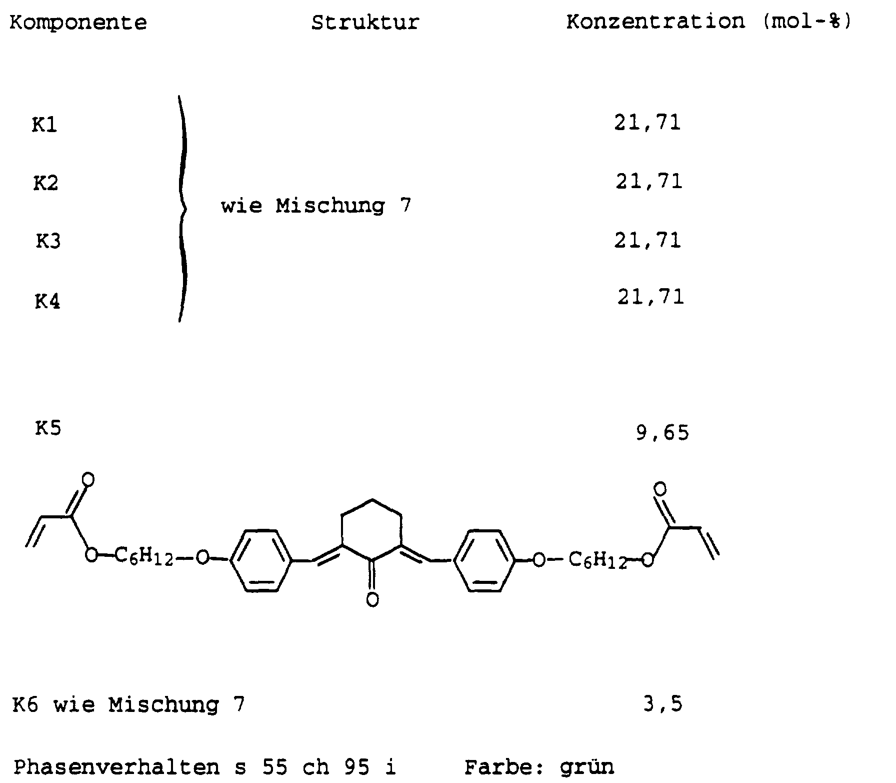

### Beispiel 60

### Mischung 9:

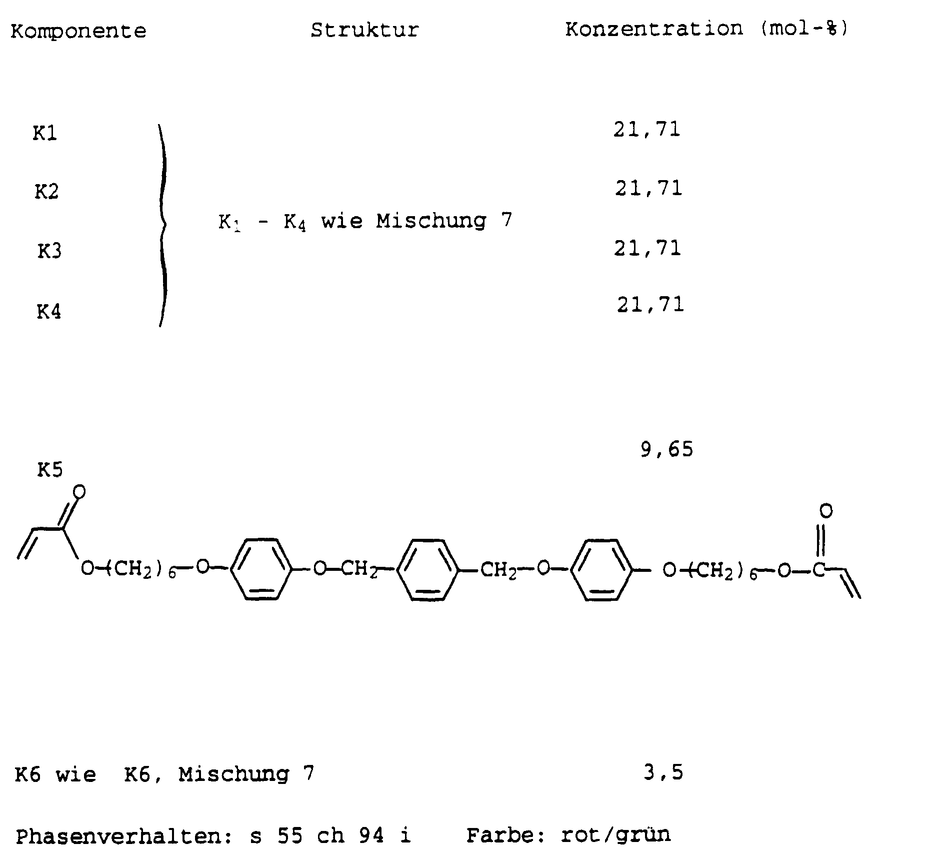

### Beispiel 61

### Mischung 10:

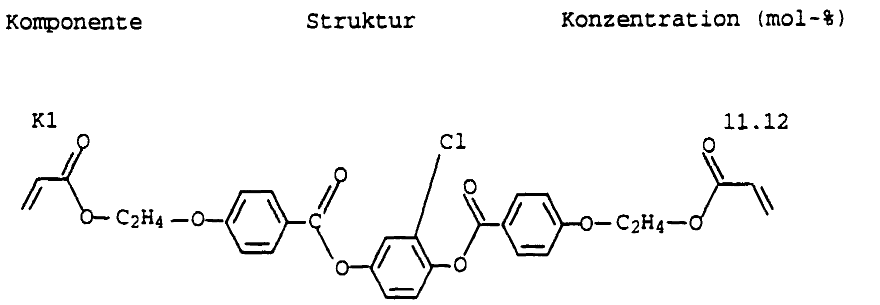

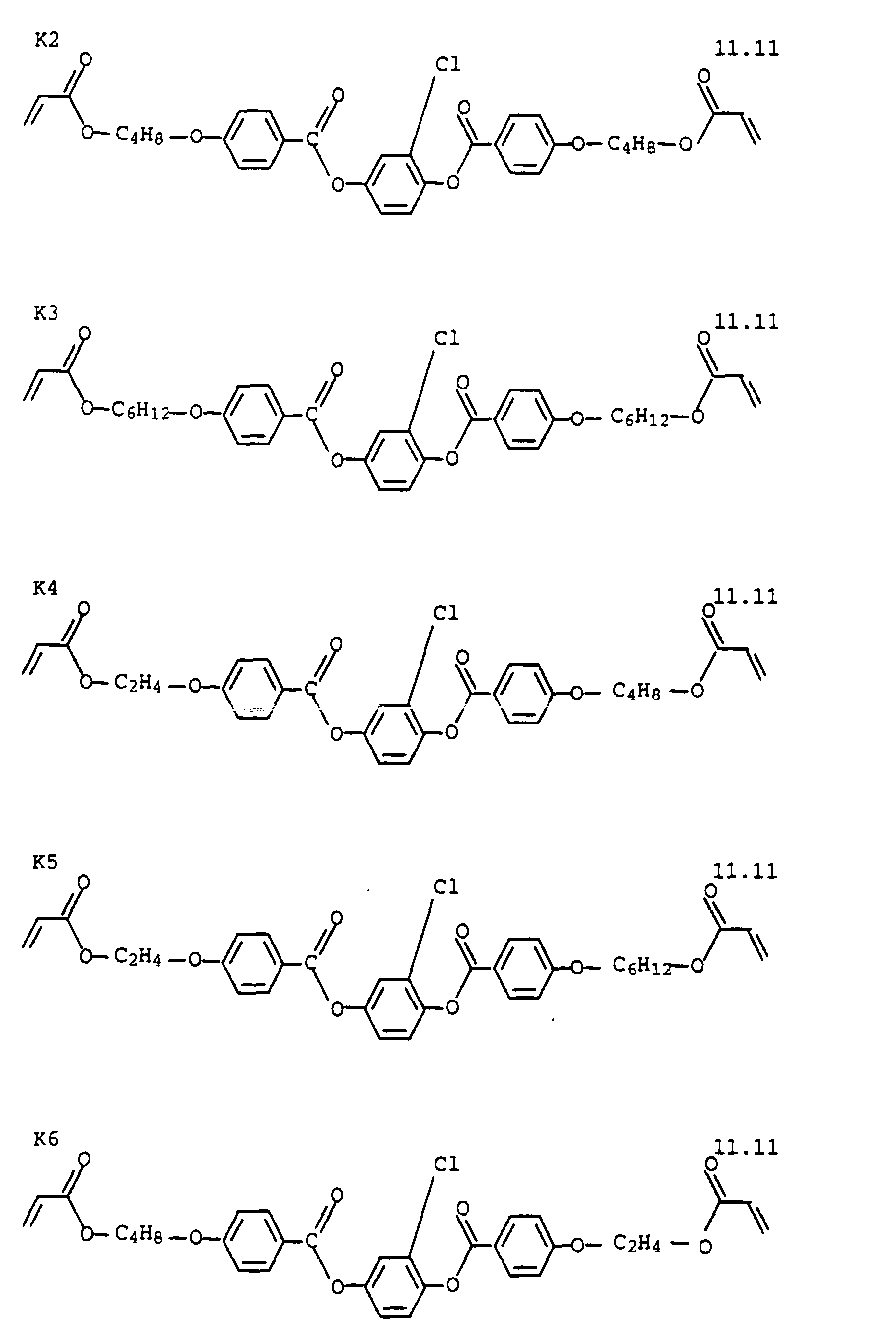

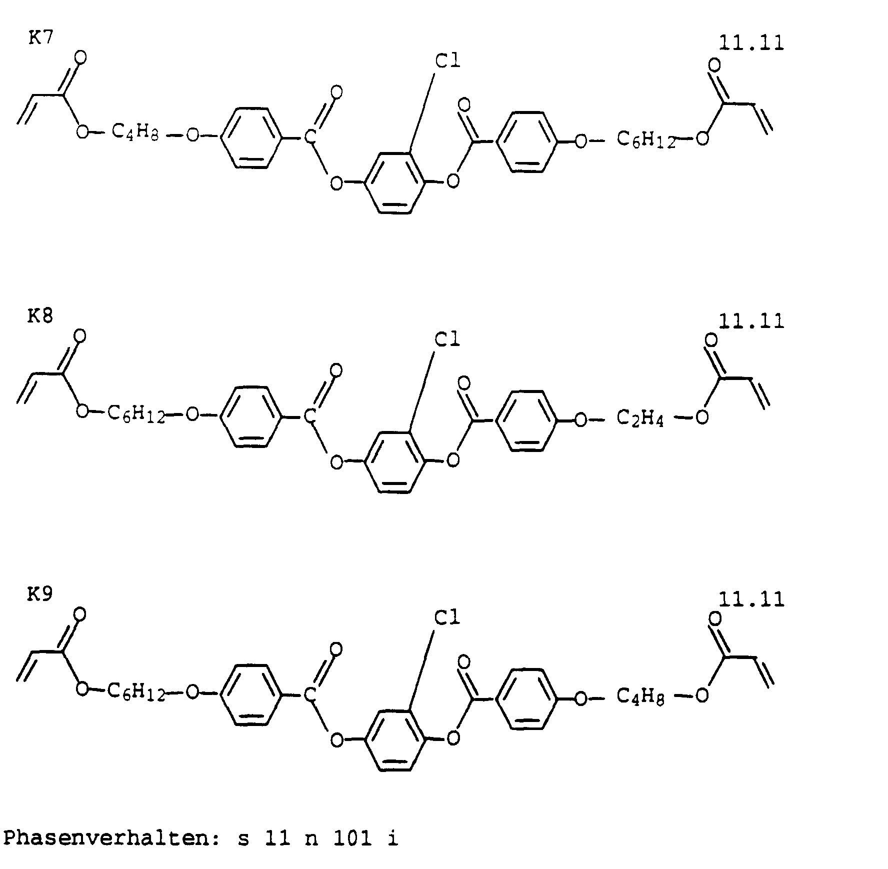

### Beispiel 62

### Mischung 11:

Komponenten der Mischung 10 in einer Konzentration von jeweils 10.8 %.

K6 aus Mischung 7 in einer Konzentration von 1,99 %.
Phasenverhalten: s 34 ch 92 i farblos

### Beispiel 63

### Mischung 12:

Komponenten der Mischung 10 in einer Konzentration von jeweils 10,78 %

K6 aus Mischung 7 in einer Konzentration von 2,98 %.
Phasenverhalten: s 44 ch 90 i Farbe: rot

### Beispiel 64

### Mischung 13:

Komponenten der Mischung 10 in einer Konzentration von jeweils 10,72 mol-%.

K6 aus Mischung 7 in einer Konzentration von 3,52 mol-%.
Phasenverhalten: s 51 n 80 i Farbe: grün

### Beispiel 65

### Mischung 14:

Komponenten der Mischung 10 in einer Konzentration von jeweils 10,67 mol-%.

K 6 aus Mischung 7 in einer Konzentration von 3,97 mol-%.
Phasenverhalten: s 65 ch 75 i Farbe: blaugrün

### Beispiel 66

### Mischung 15:

Komponenten der Mischung 10 in einer Konzentration von jeweils 9,61 mol-%. 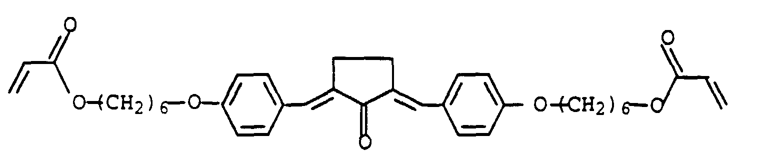 in einer Konzentration von 10 mol-%

K 6 aus Mischung 7 in einer Konzentration von 3,51 mol-%.
Phasenverhalten: s 63 ch 96 i Farbe: grün

### Beispiel 67

### Mischung 16:

Komponenten der Mischung 10 in einer Konzentration von jeweils 8,5 mol-%. 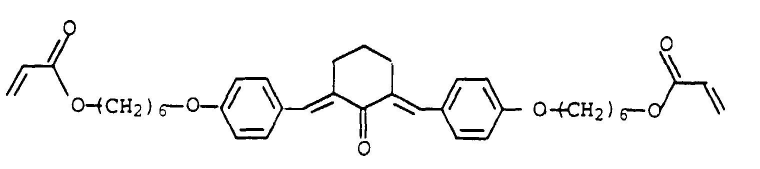 in einer Konzentration von 10 mol-%.

K6 aus Mischung 7 in einer Konzentration von 3,5 mol-%
Phasenverhalten: s 51 n 87 i Farbe: grün

### Beispiel 68

### Mischung 17

Komponenten der Mischung 10 in einer Konzentration von jeweils 9,61 mol-%. 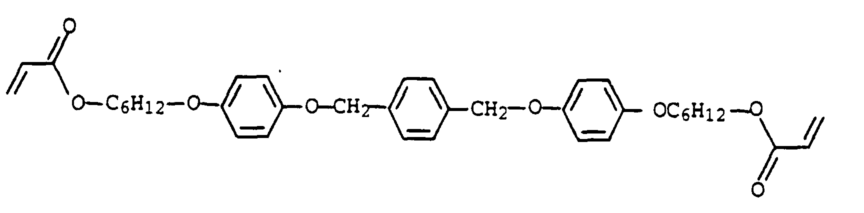 in einer Konzentration von 10 mol-%

K6 aus Mischung 7 in einer Konzentration von 3,51 mol-%.
Phasenverhalten: s 63 ch 78 i Farbe: grün

### Beispiel 69

### Mischung 18

Komponenten K1 bis K9 der Mischung 10 in einer Konzentration von jeweils 10,67 mol-%. 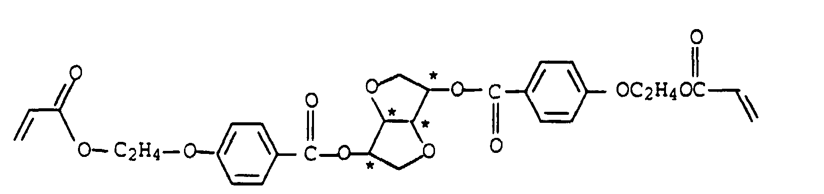 in einer Konzentration von 3,97 mol-%.
Phasenverhalten: ch 91 i farblos

### Beispiel 70

### Mischung 19:

Komponenten der Mischung 10 in einer Konzentration von jeweils 9,61 mol-%.

Hexandioldiacrylat in einer Konzentration von 10 mol-%.

K6 aus Mischung 7 in einer Konzentration von 3,51 mol-%
Phasenverhalten: s 50 ch 70 i Farbe: grün

### Beispiel 72

### Mischung 20:

Komponenten der Mischung 10 in einer Konzentration von jeweils 8,5 mol-%.

Hexandioldiacrylat in einer Konzentration von 20 mol-%.

K6 aus Mischung 7 in einer Konzentration von 3,5 mol-%.
Phasenverhalten: s 46 ch 61 i Farbe: grün

### Beispiel 73

### Mischung 21

Komponenten der Mischung 10 in einer Konzentration von jeweils 8,5 mol-% 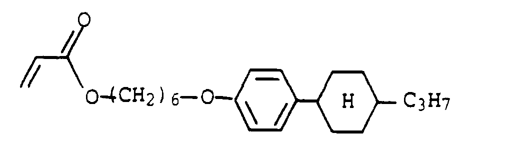 in einer Konzentration von 20 mol-%.

K6 aus Mischung 7 in einer Konzentration von 3,5 mol-%
Phasenverhalten s 56 ch 80 i Farbe: grün bis rot

### Beispiel 74

### Mischung 22

Komponenten der Mischung 10 in einer Konzentration von jeweils 8,5 mol-%. 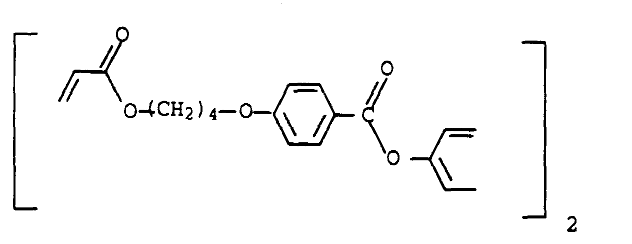 in einer Konzentration von 20 mol-%.

K6 aus Mischung 7 in einer Konzentration von 3,5 mol-%.
Phasenverhalten: s 50 ch 82 i Farbe: grün

### Beispiel 75

### Mischung 23

Komponenten der Mischung 10 in einer Konzentration von jeweils 8,5 mol-%. 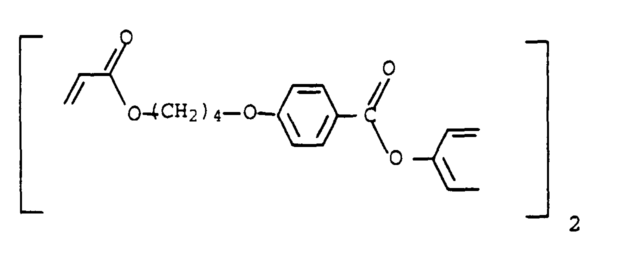 in einer Konzentration von 20 mol-%.

K 6 aus Mischung 7 in einer Konzentration von 3,5 mol-%.
Phasenverhalten s 49 ch 80,5 i

### Beispiel 76

### Mischung 24

Komponenten der Mischung 10 in einer Konzentration von jeweils 8,5 mol-% 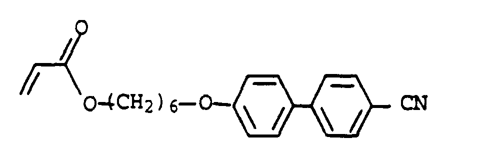 in einer Konzentration von 20 mol-%.

K6 aus Mischung 7 in einer Konzentration von 3,5 mol-%.
Phasenverhalten: s 53 ch 84 i Farbe: grün

### Beispiel 77

### Mischung 25

Komponenten der Mischung 10 in einer Konzentration von jeweils 10.1 mol-%

K10 aus Mischung 18 in einer Konzentration von 9,1 mol-%.
Phasenverhalten: ch 89 i Farbe: blau

### Beispiel 78

### Mischung 26

Komponenten K1 bis K9 der Mischung 10 in einer Konzentration von jeweils 10,55 % 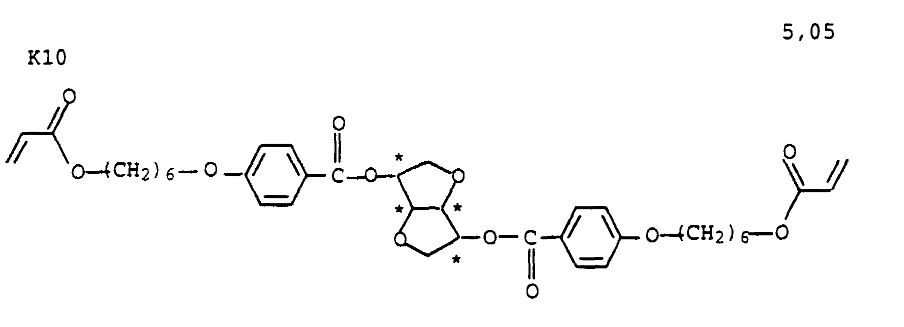 Phasenverhalten: ch 91 i Farbe: rot

### Beispiel 79

### Mischung 27

Komponente K1 bis K9 der Mischung 10 in einer Konzentration von 10,33 %

Komponente K10 aus Mischung 26 in einer Konzentration von 7,03 %
Phasenverhalten: ch 90 i Farbe: blau

### Beispiel 80

### Mischung 28

Komponenten K1 bis K9 der Mischung 10 in einer Konzentration von 6,51 mol-%

Styrol in einer Konzentration von 41,41 mol-%
Phasenverhalten: ch 61-74 i

### Beispiel 81

### Mischung 29

Komponenten K1 bis K9 der Mischung 10 in einer Konzentration von 5,22 mol-%

Styrol in einer Konzentration von 53,02 mol-%
Phasenverhalten: ch 50-69 i

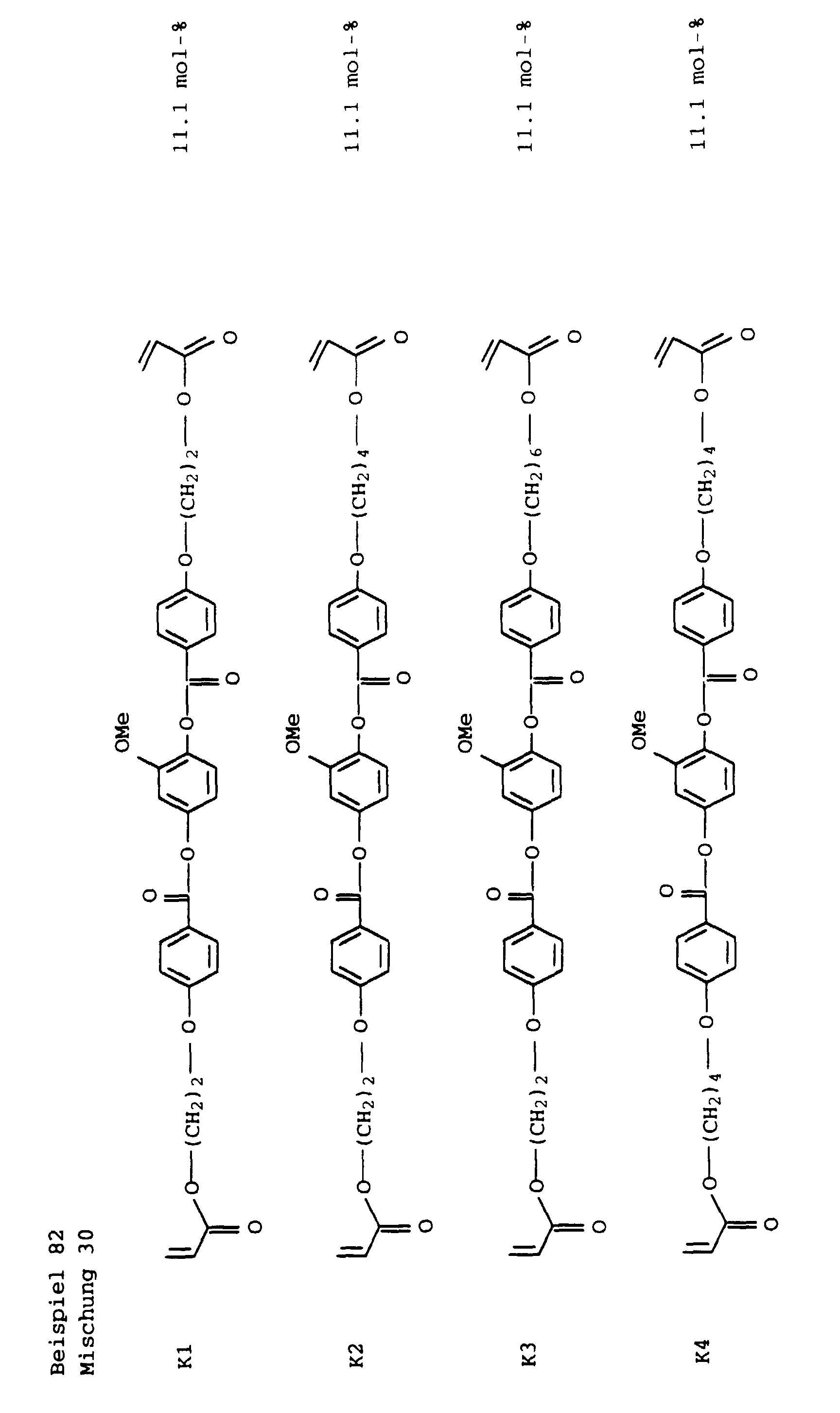

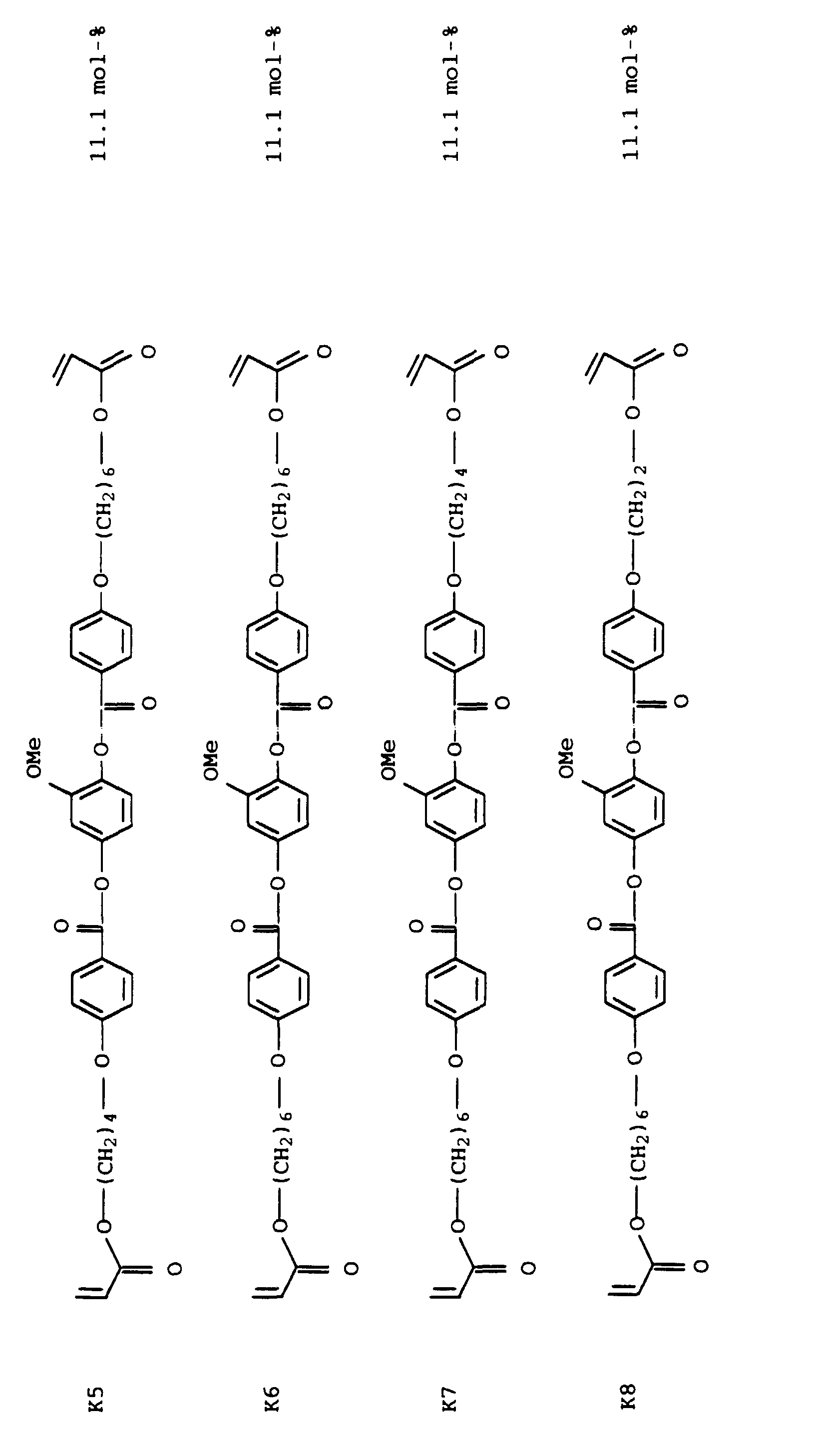

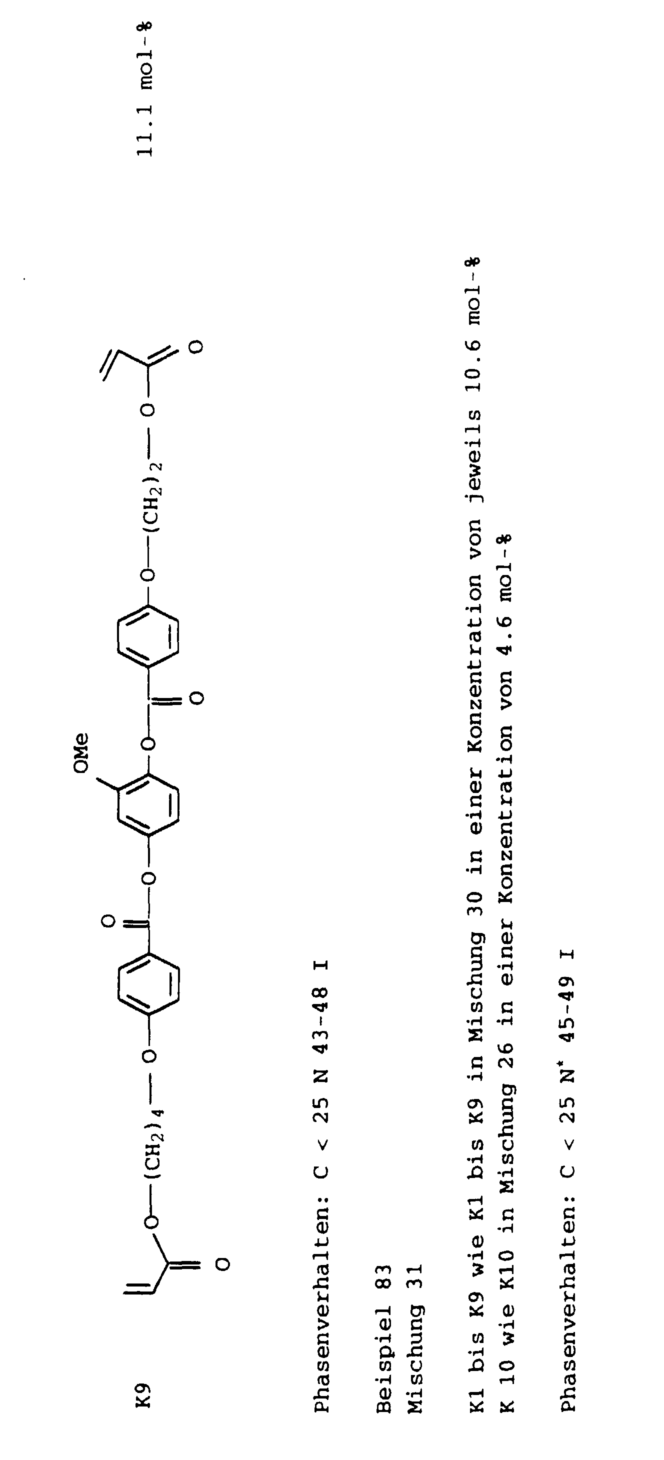

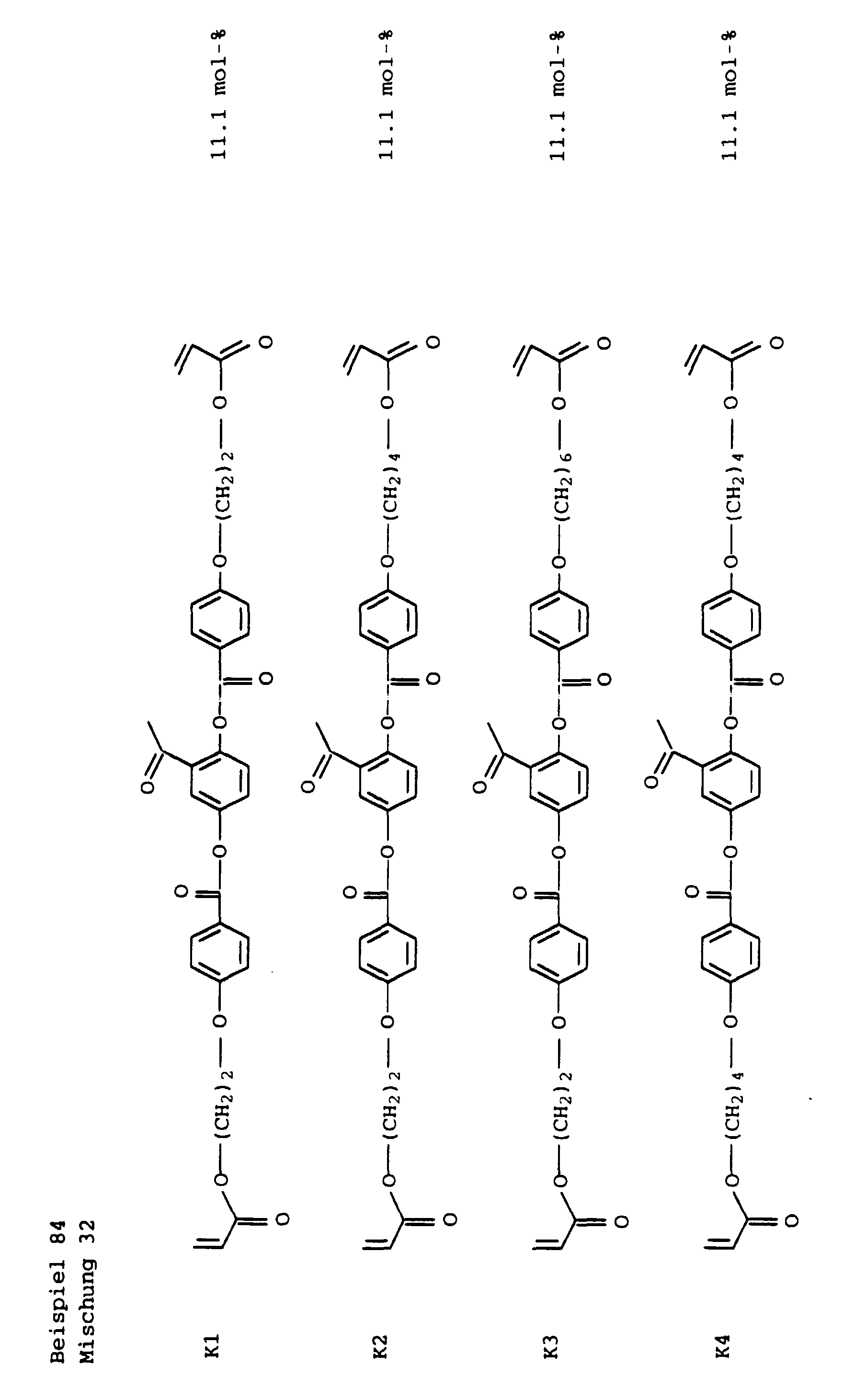

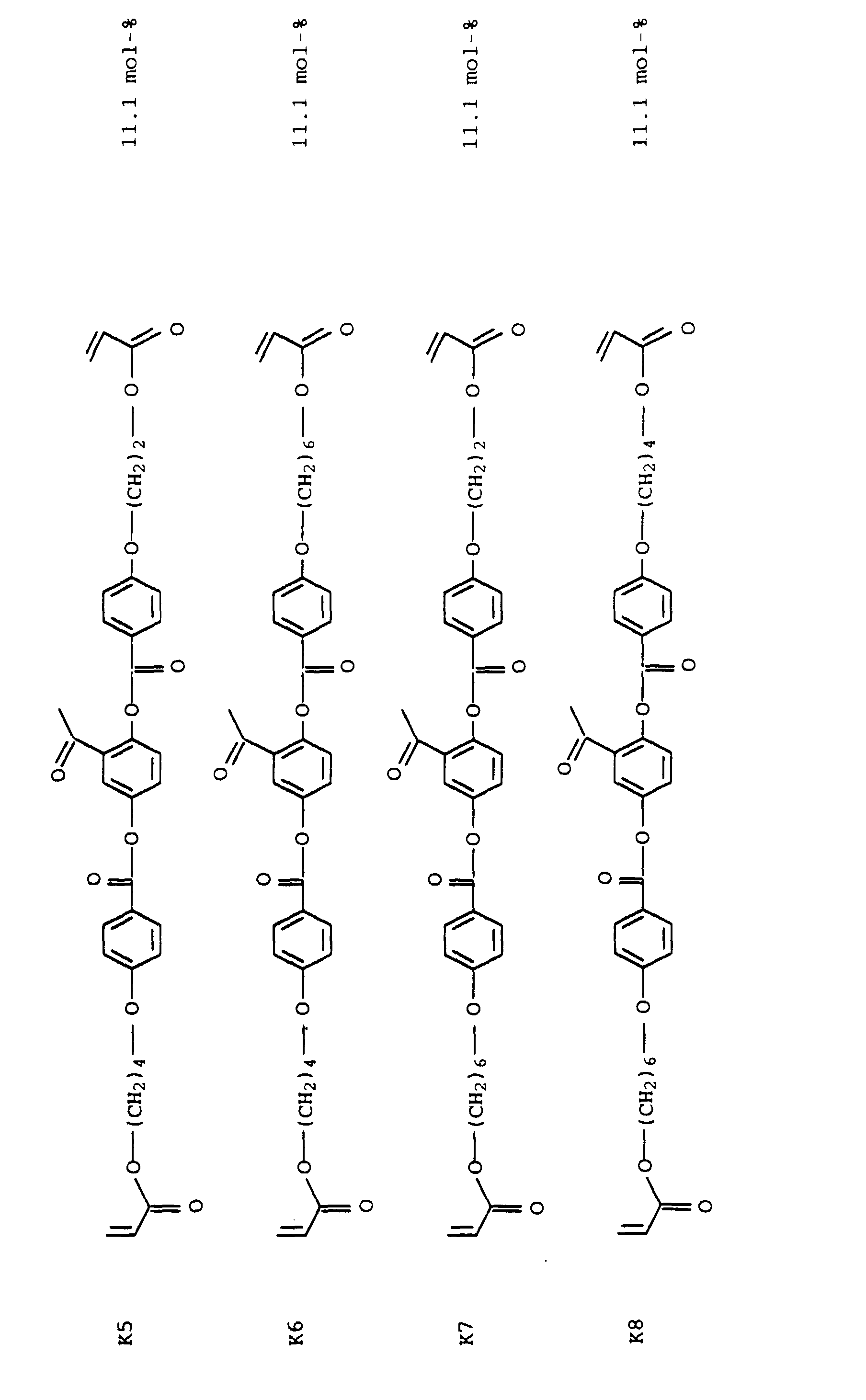

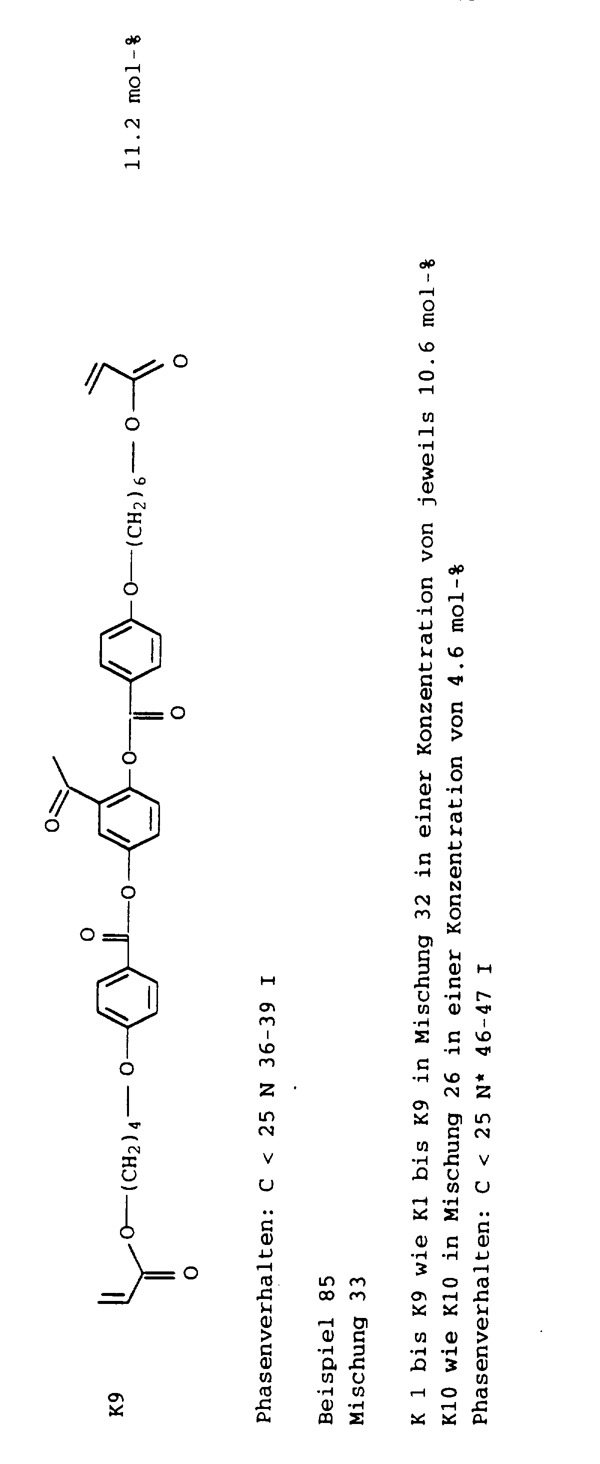

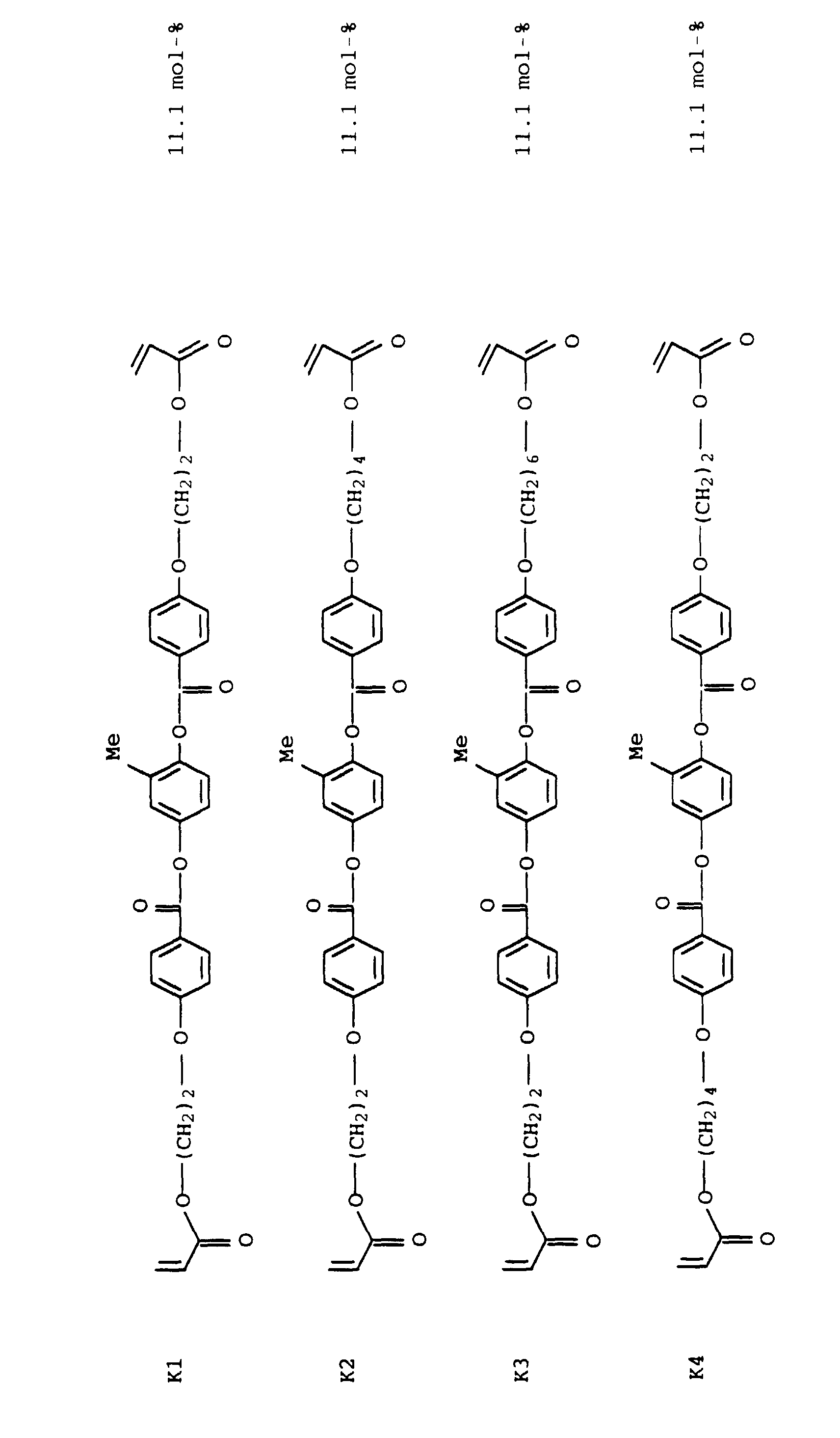

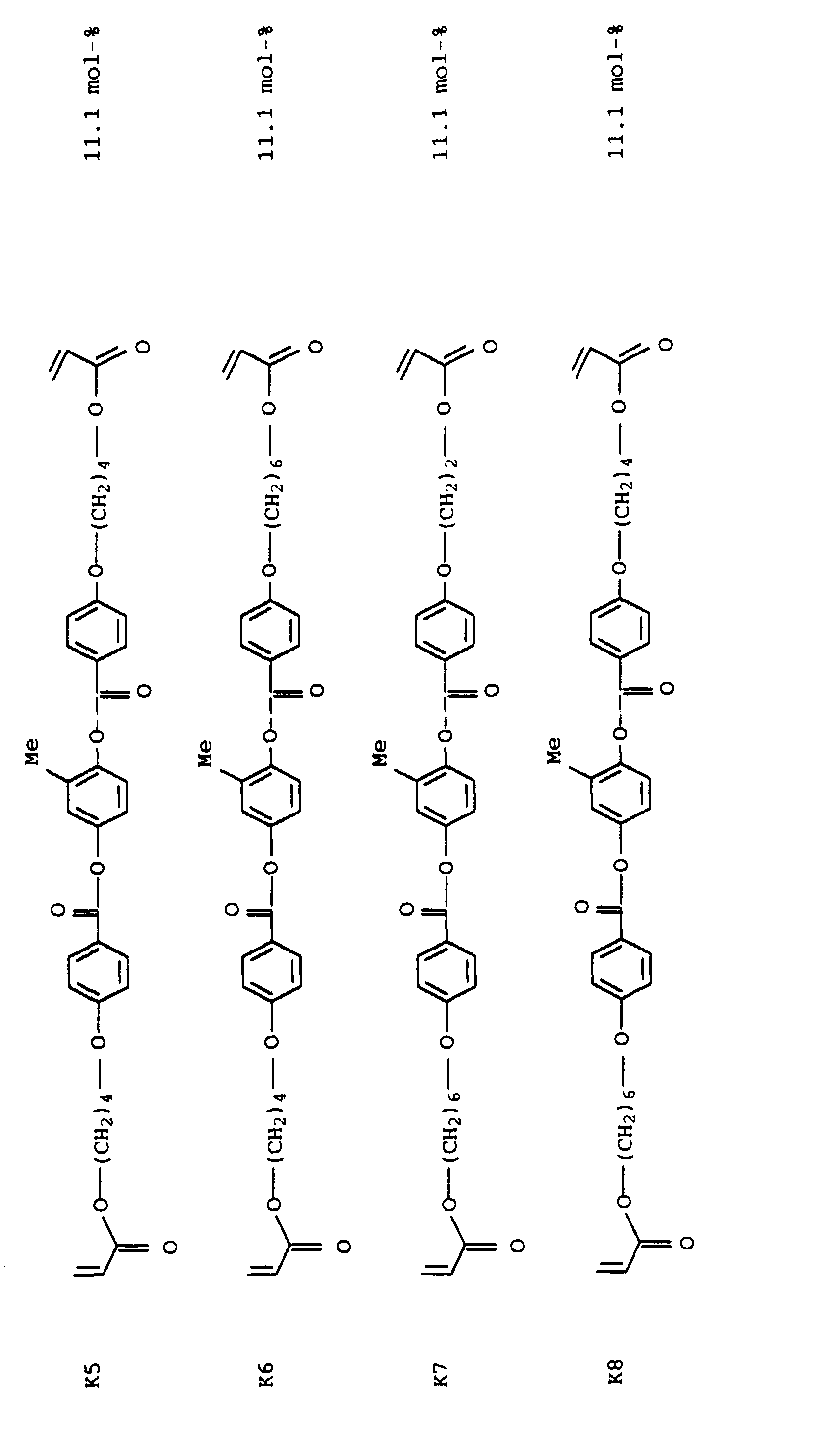

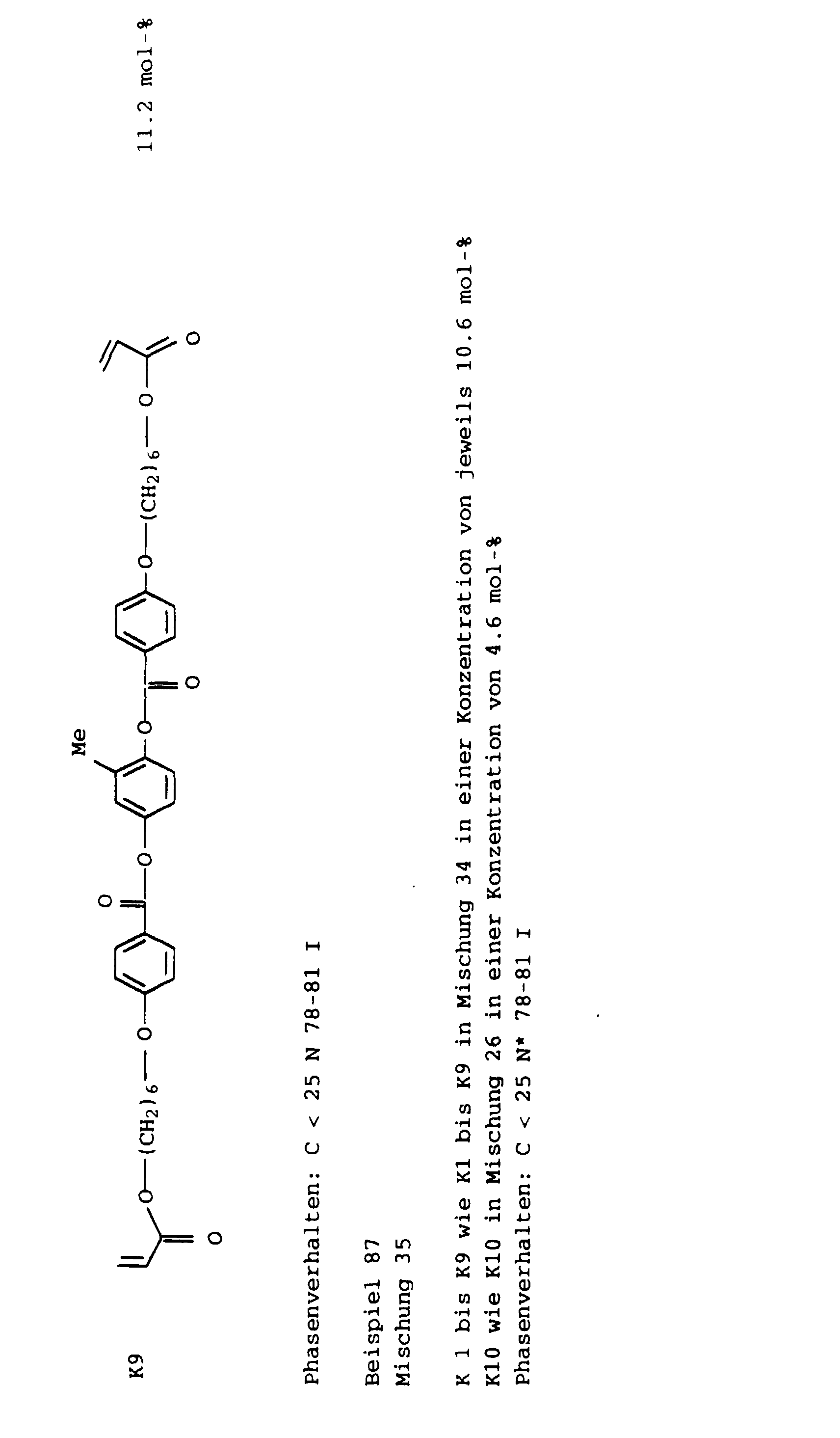

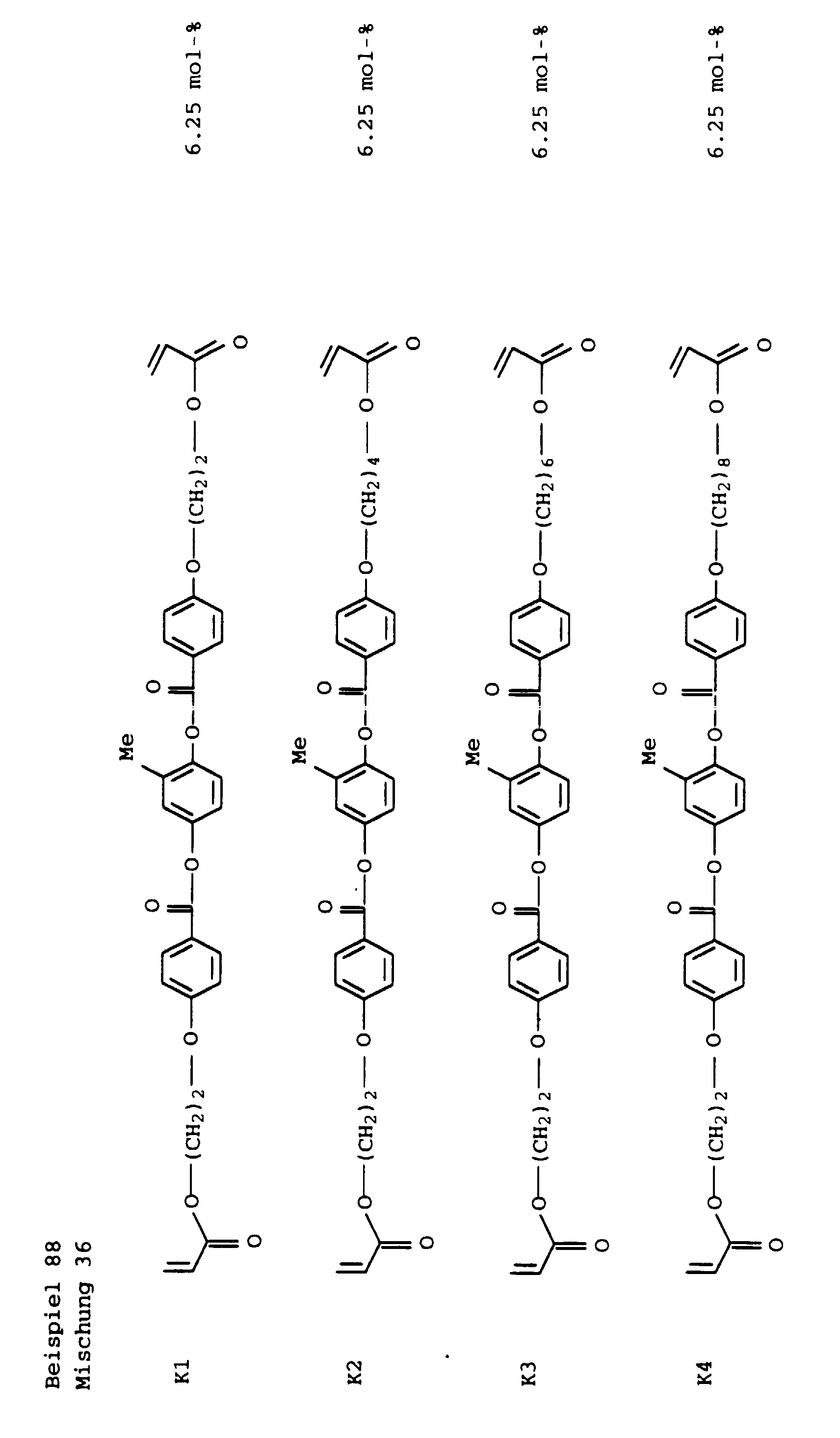

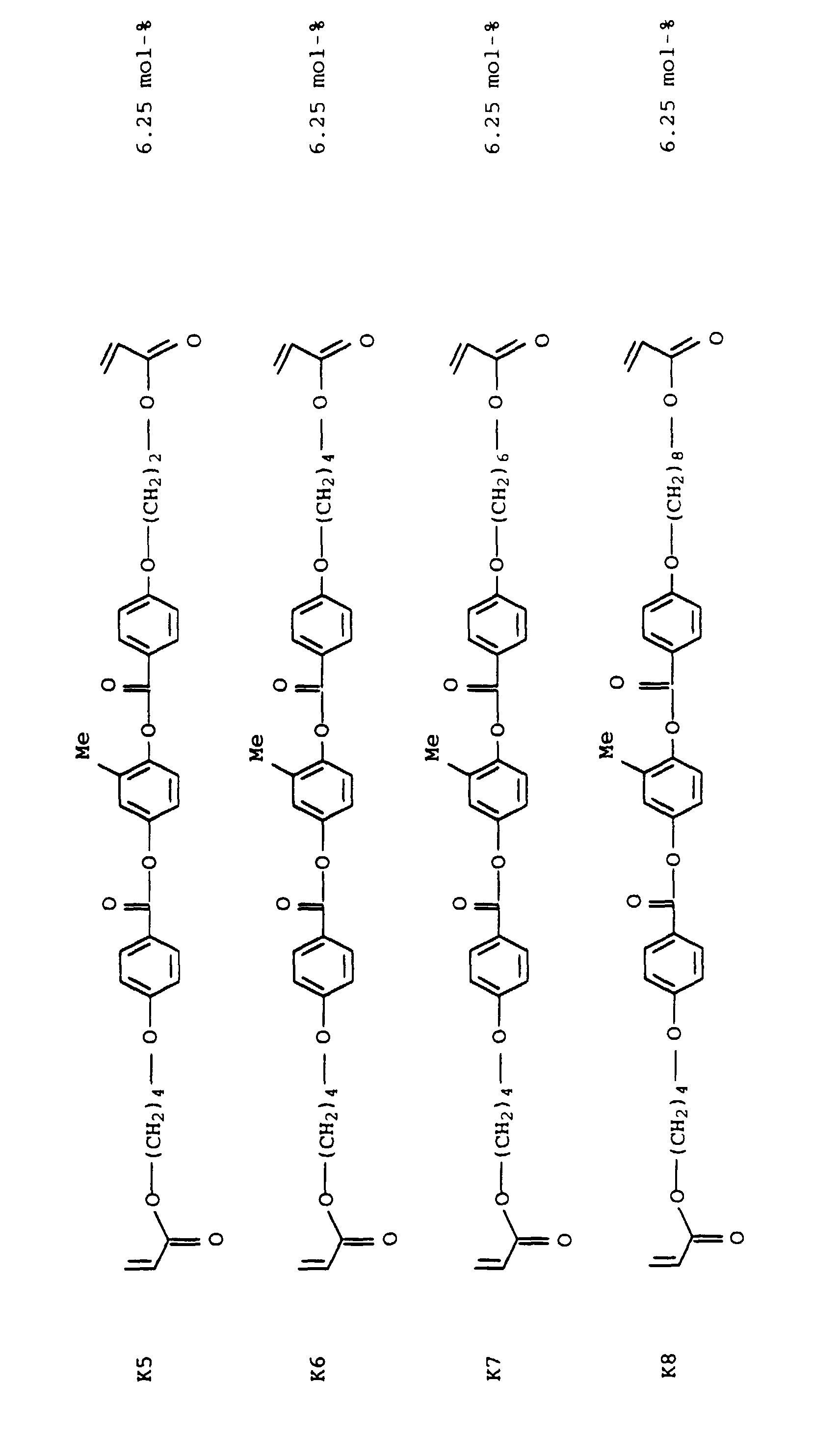

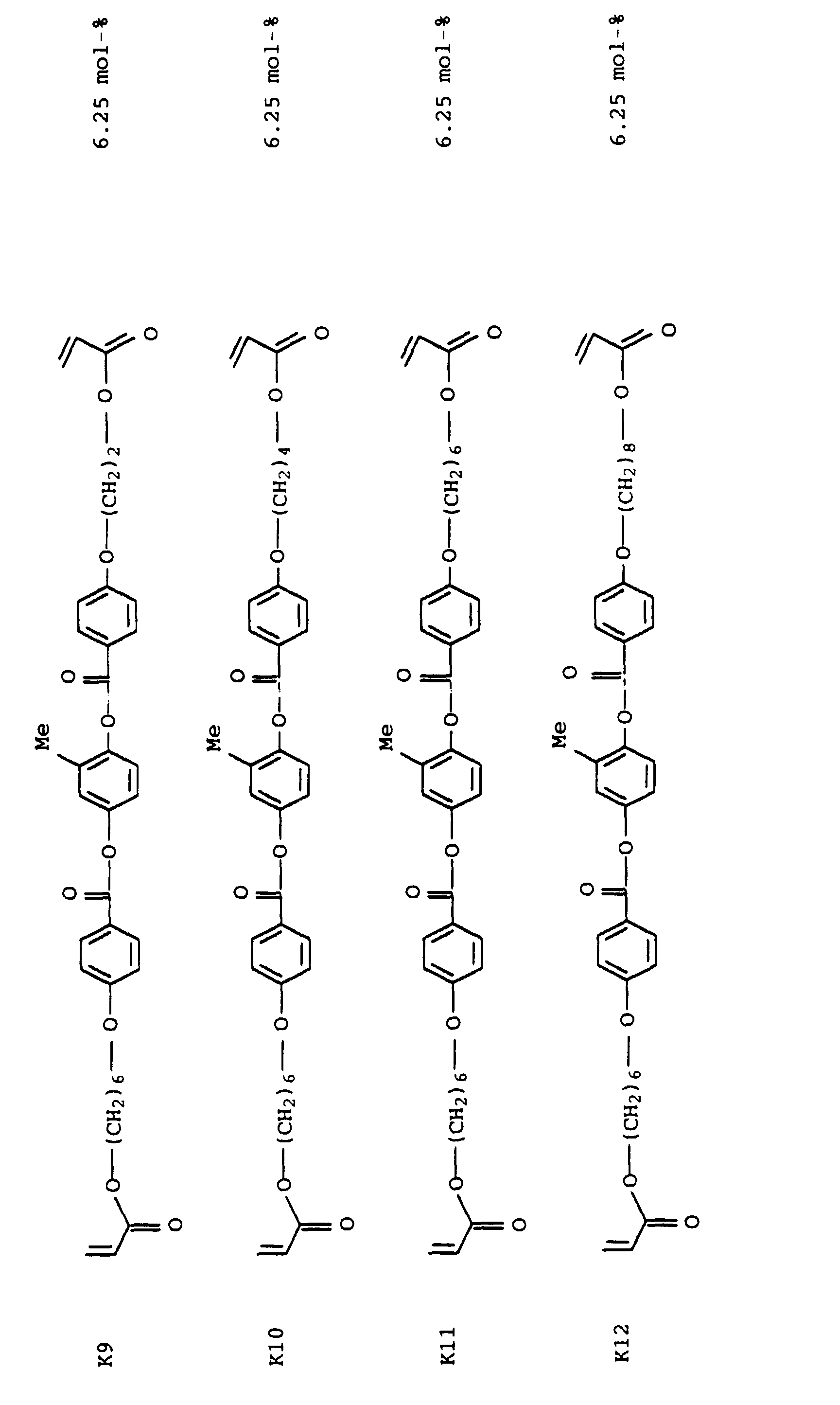

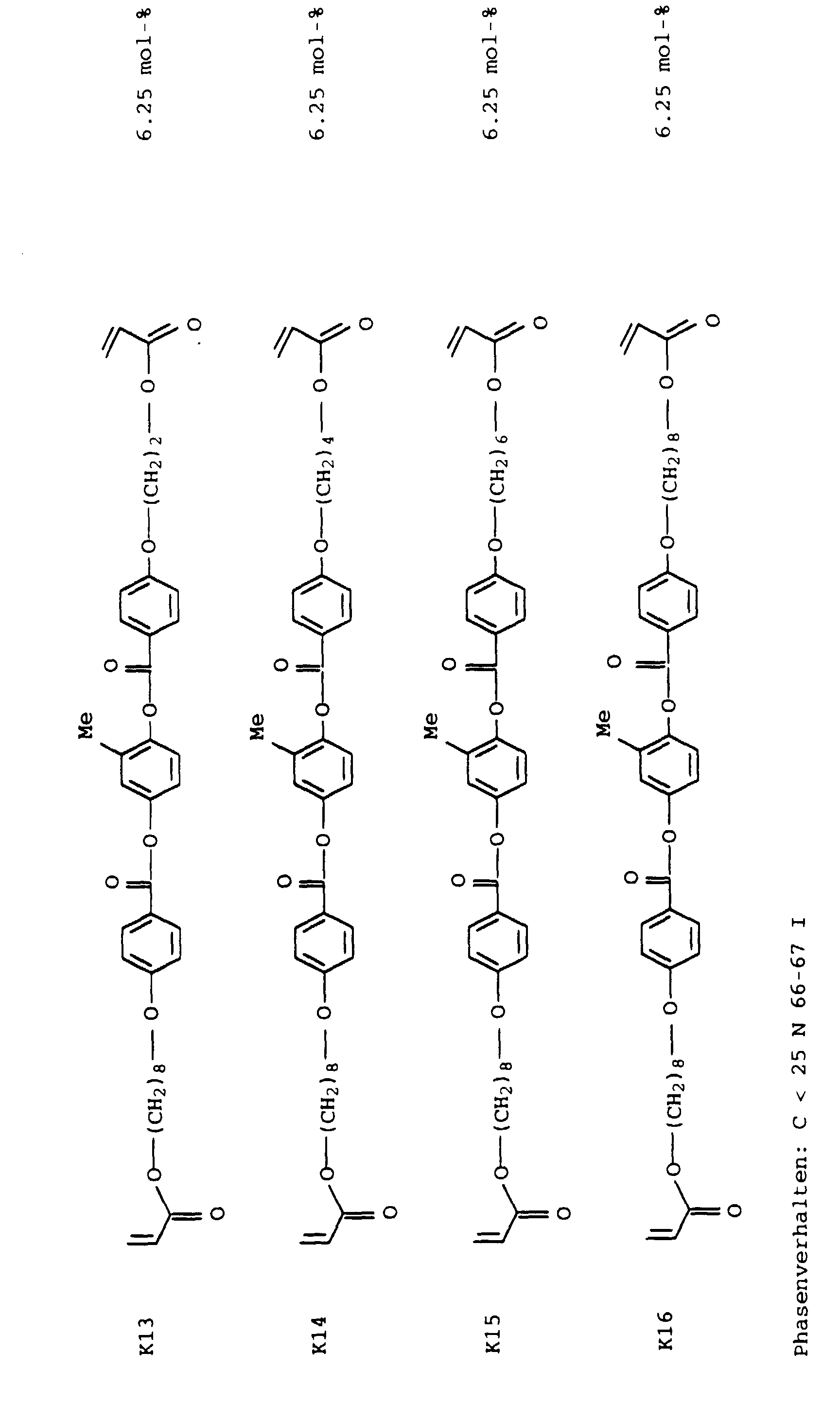

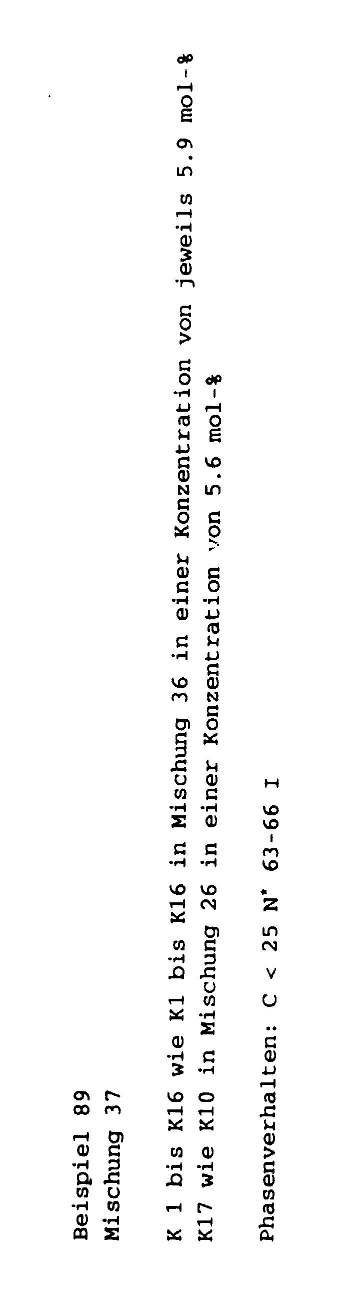

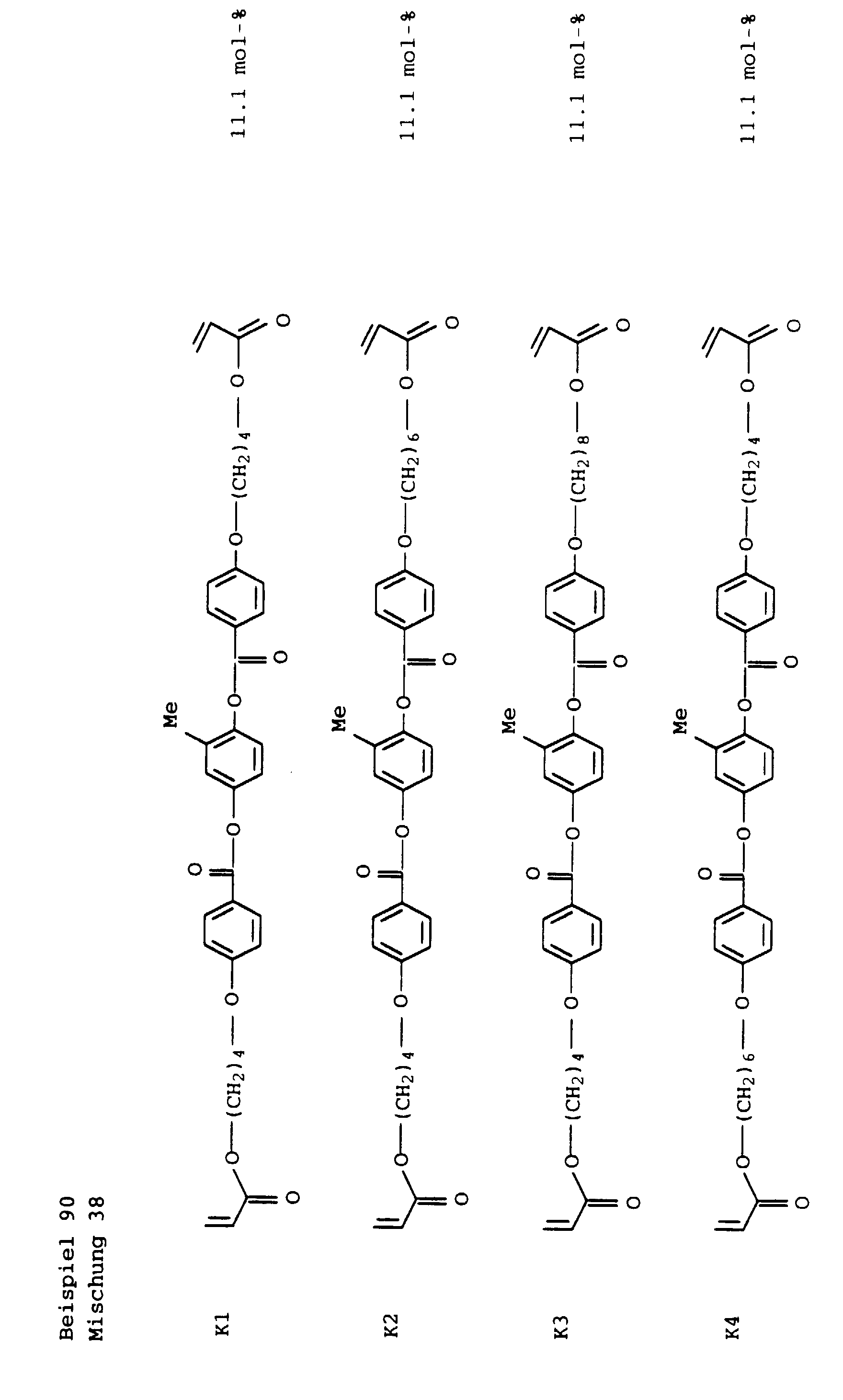

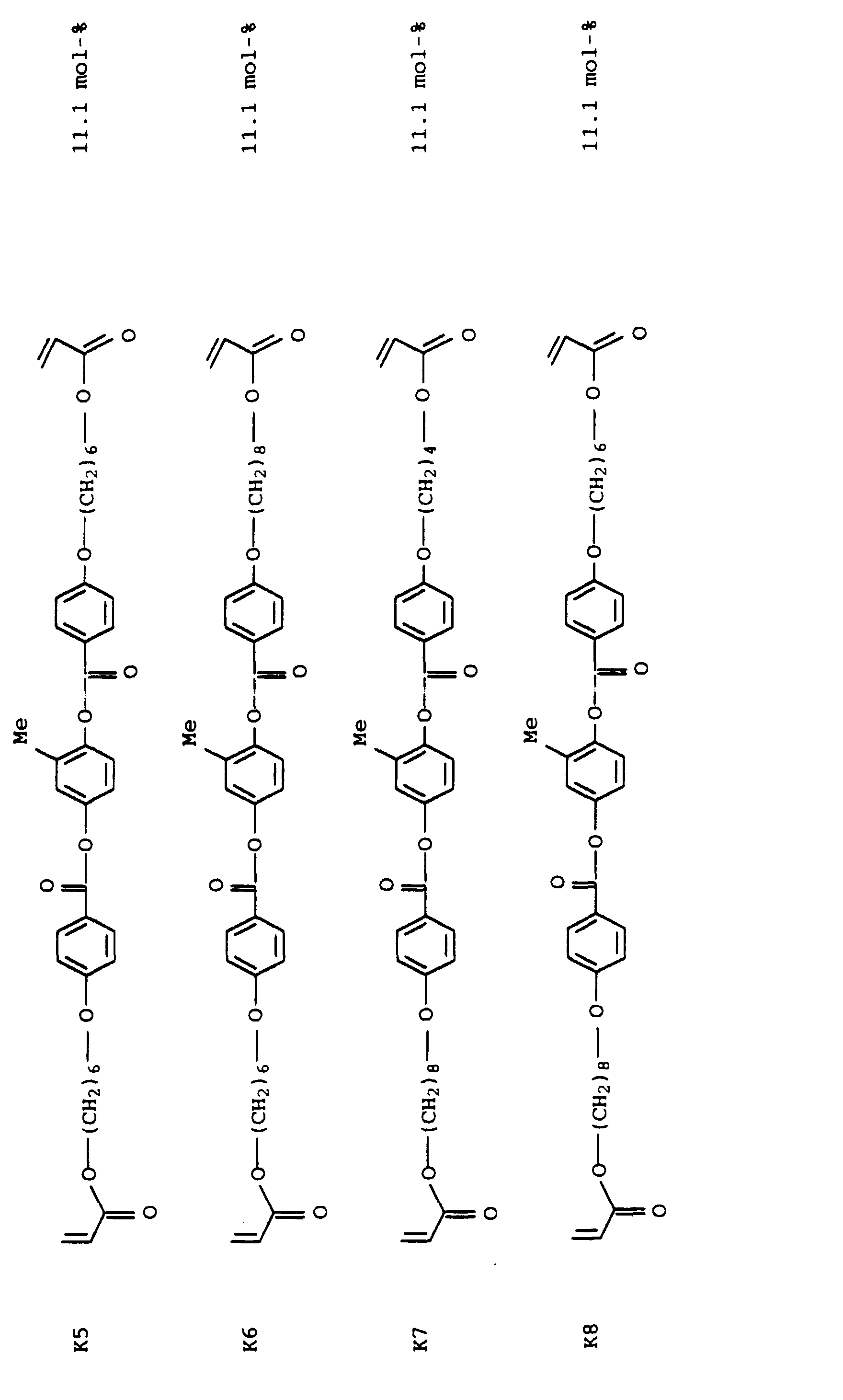

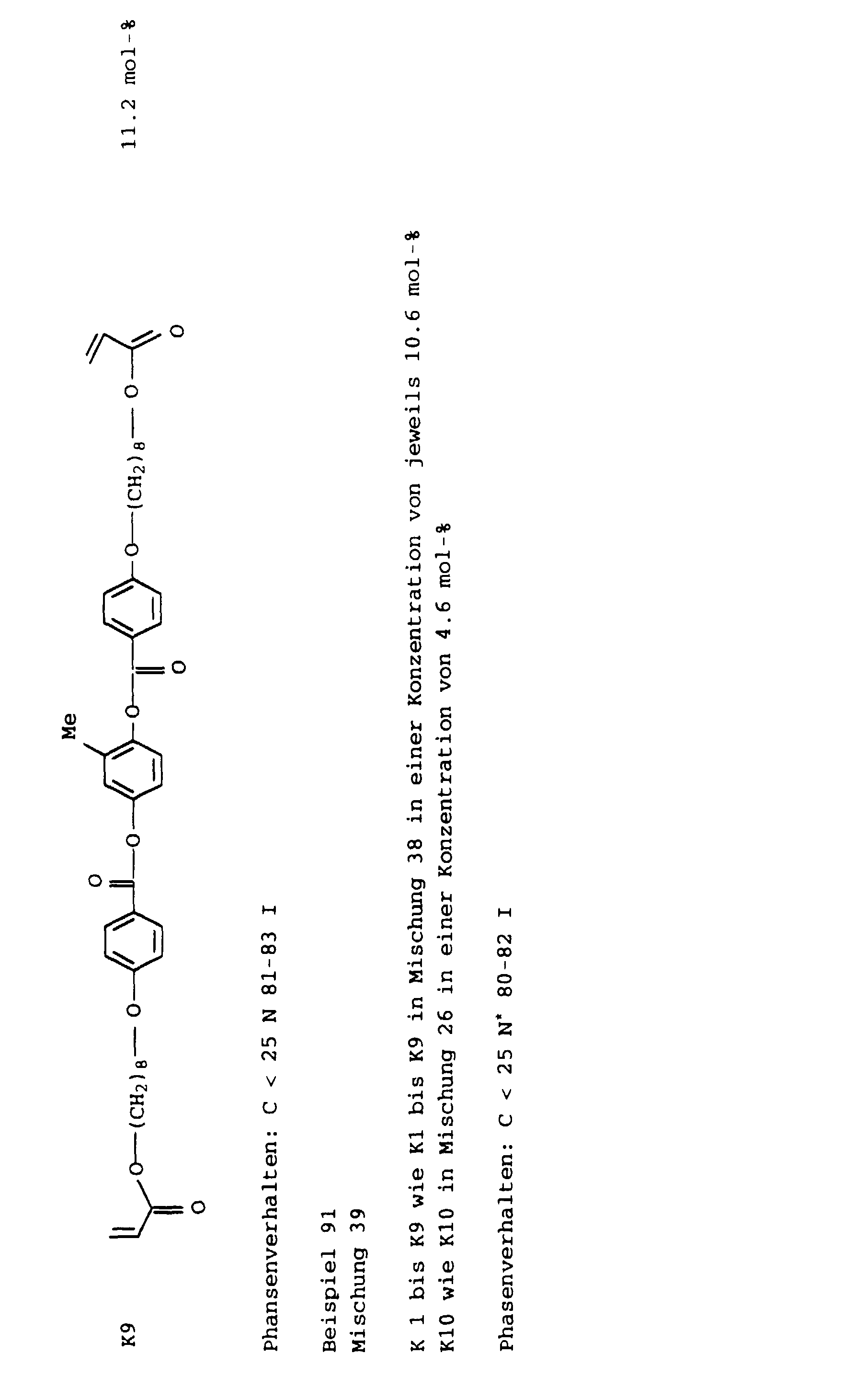

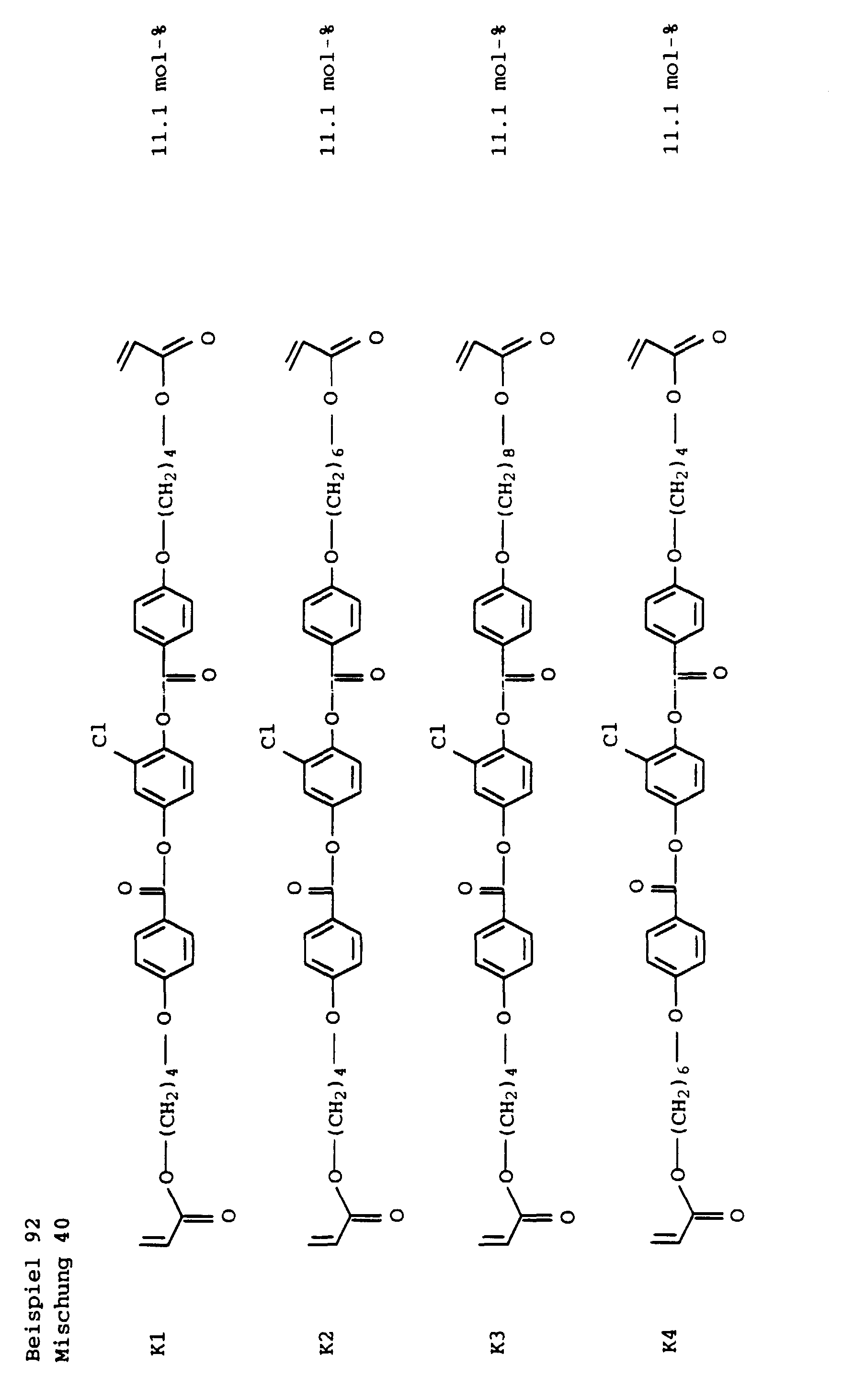

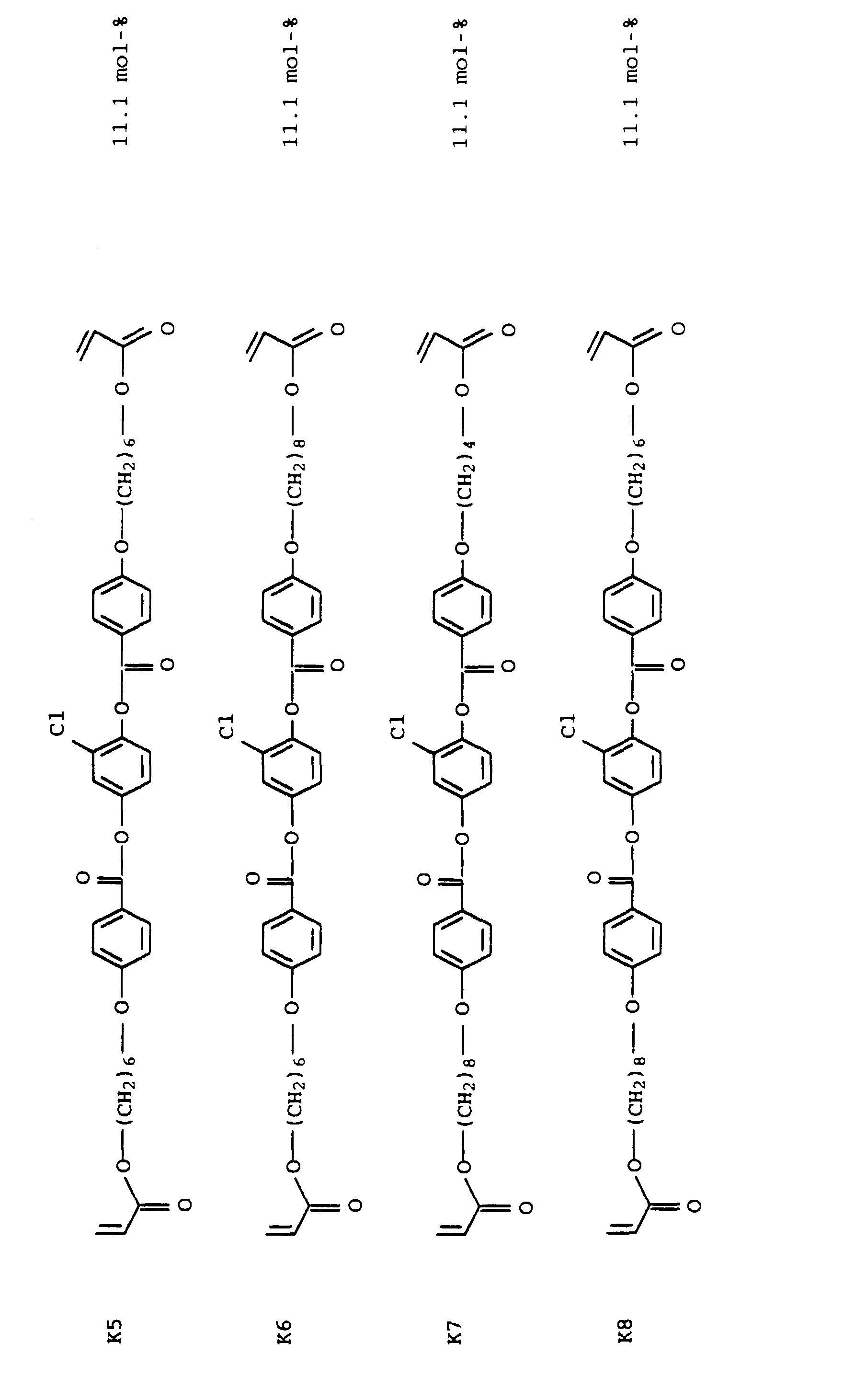

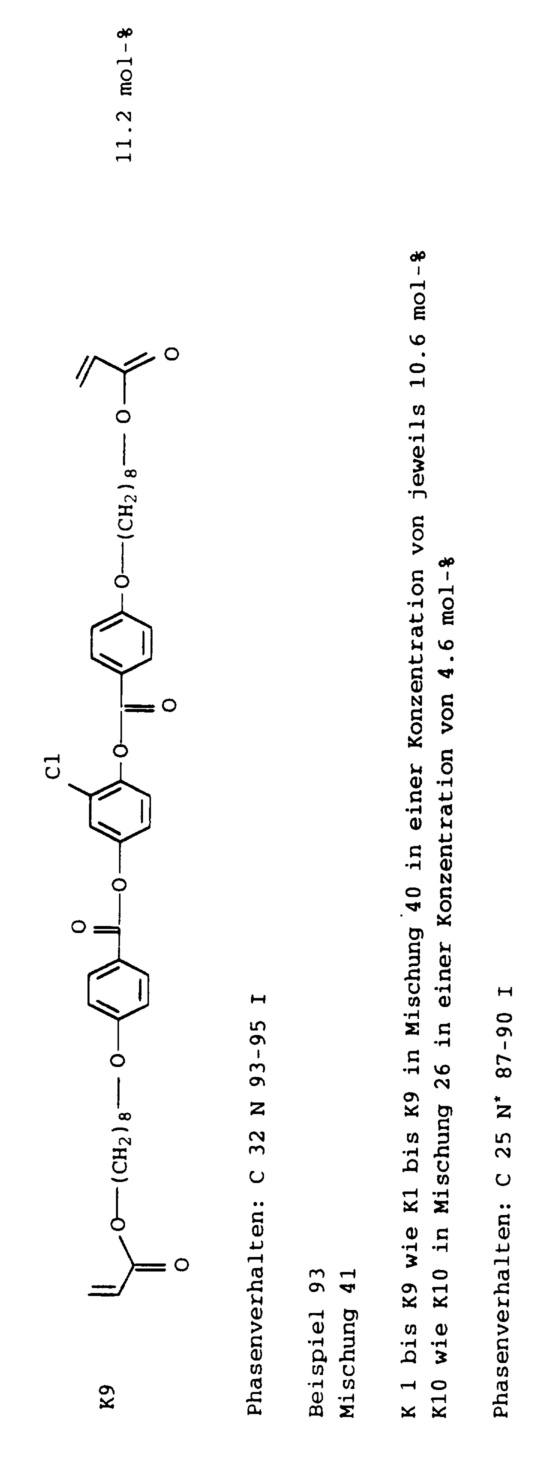

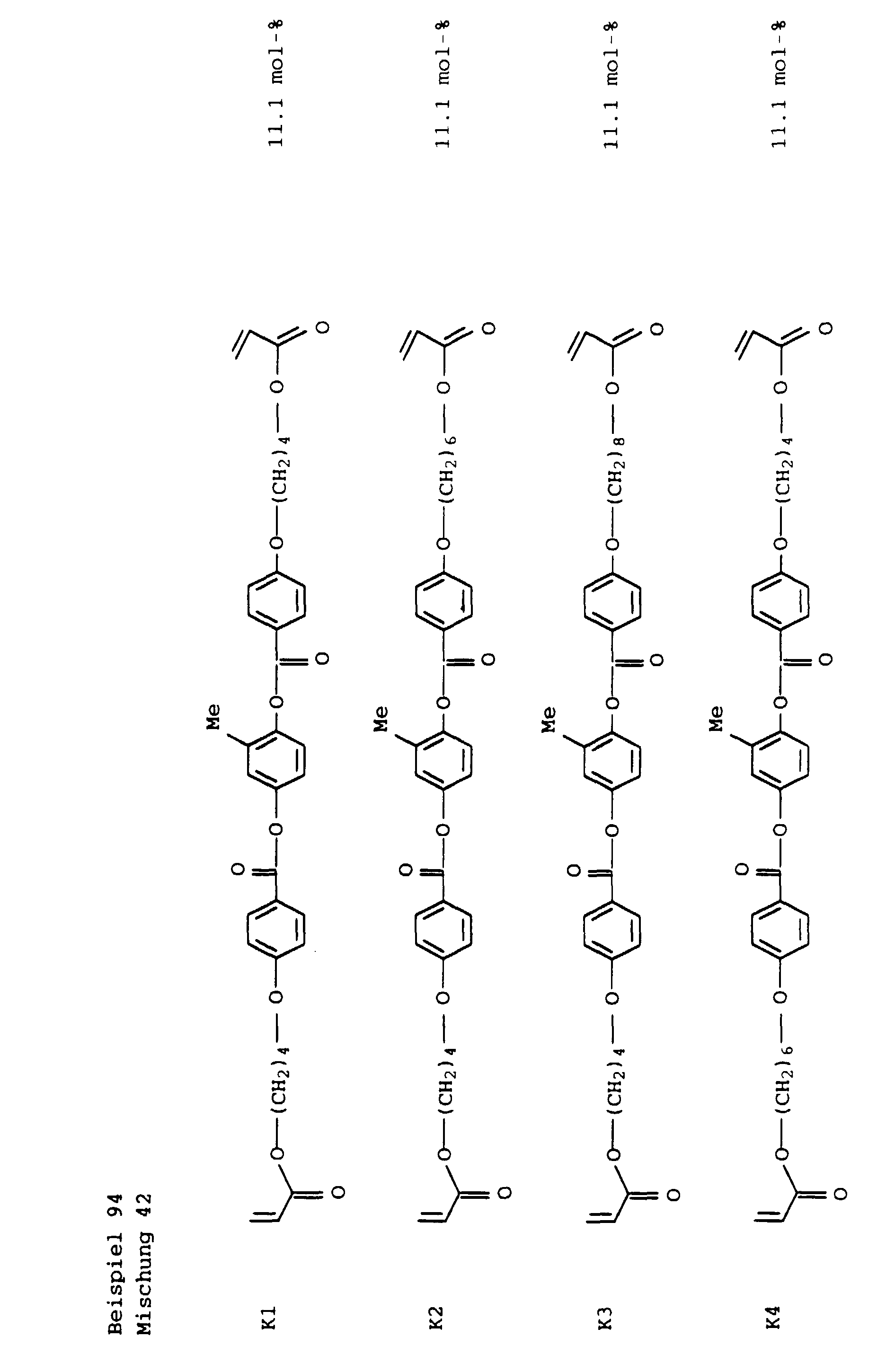

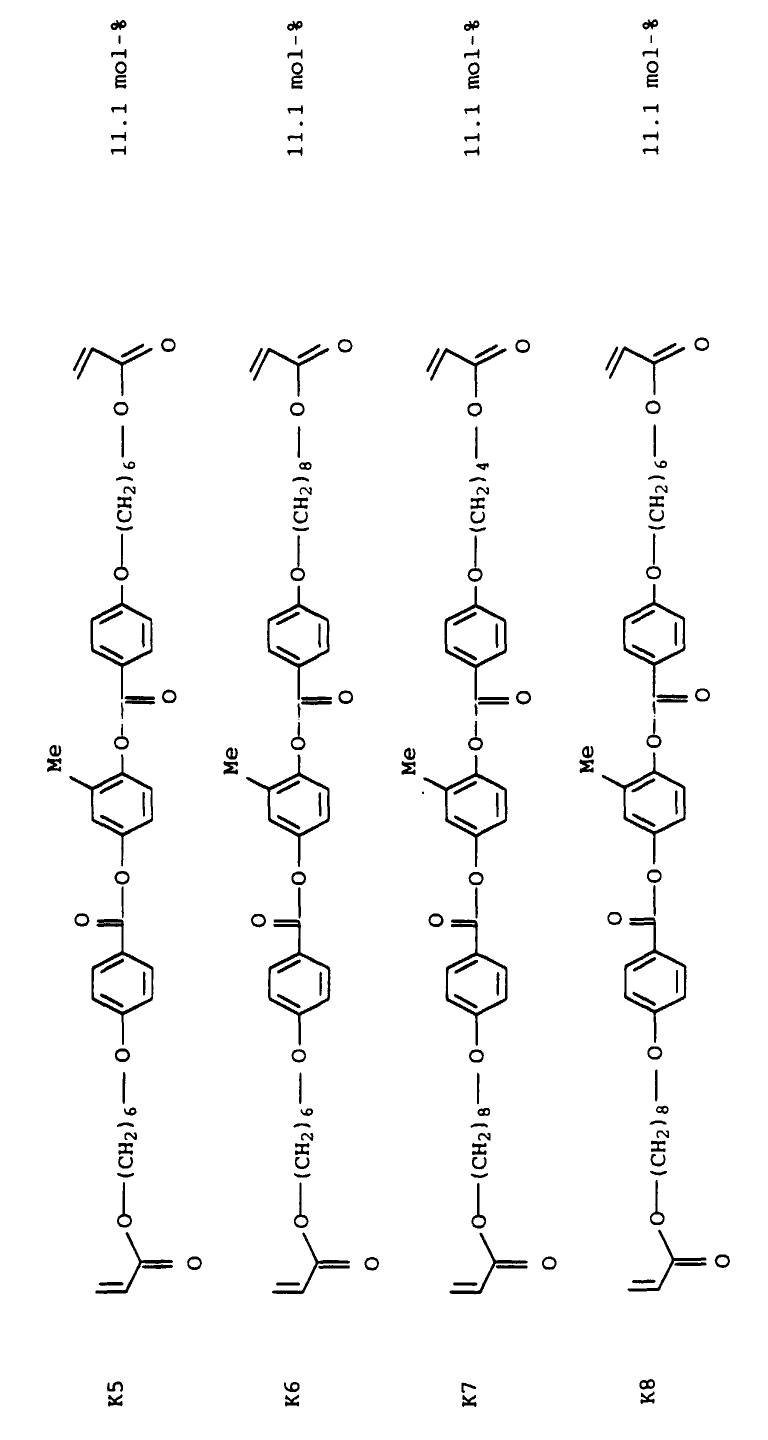

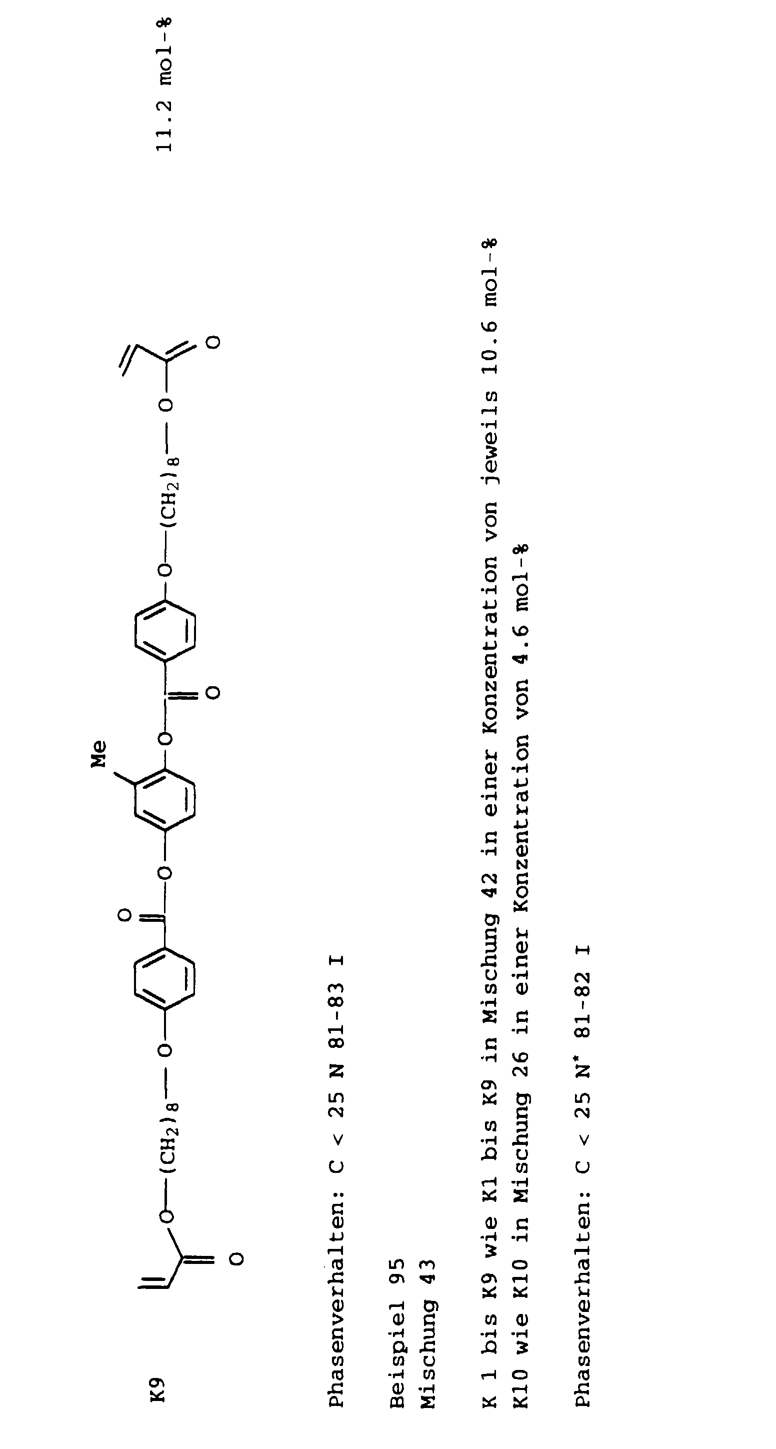

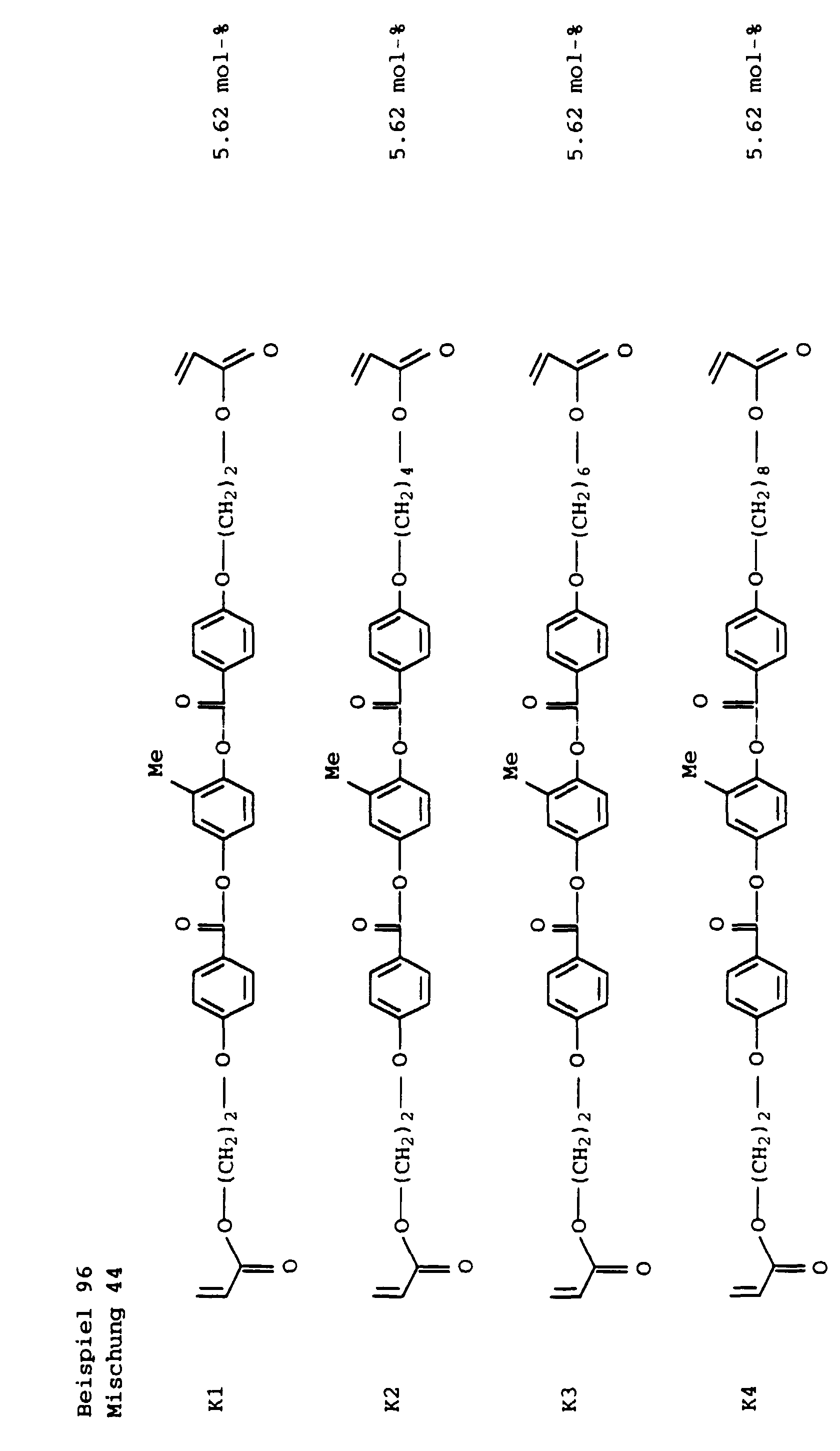

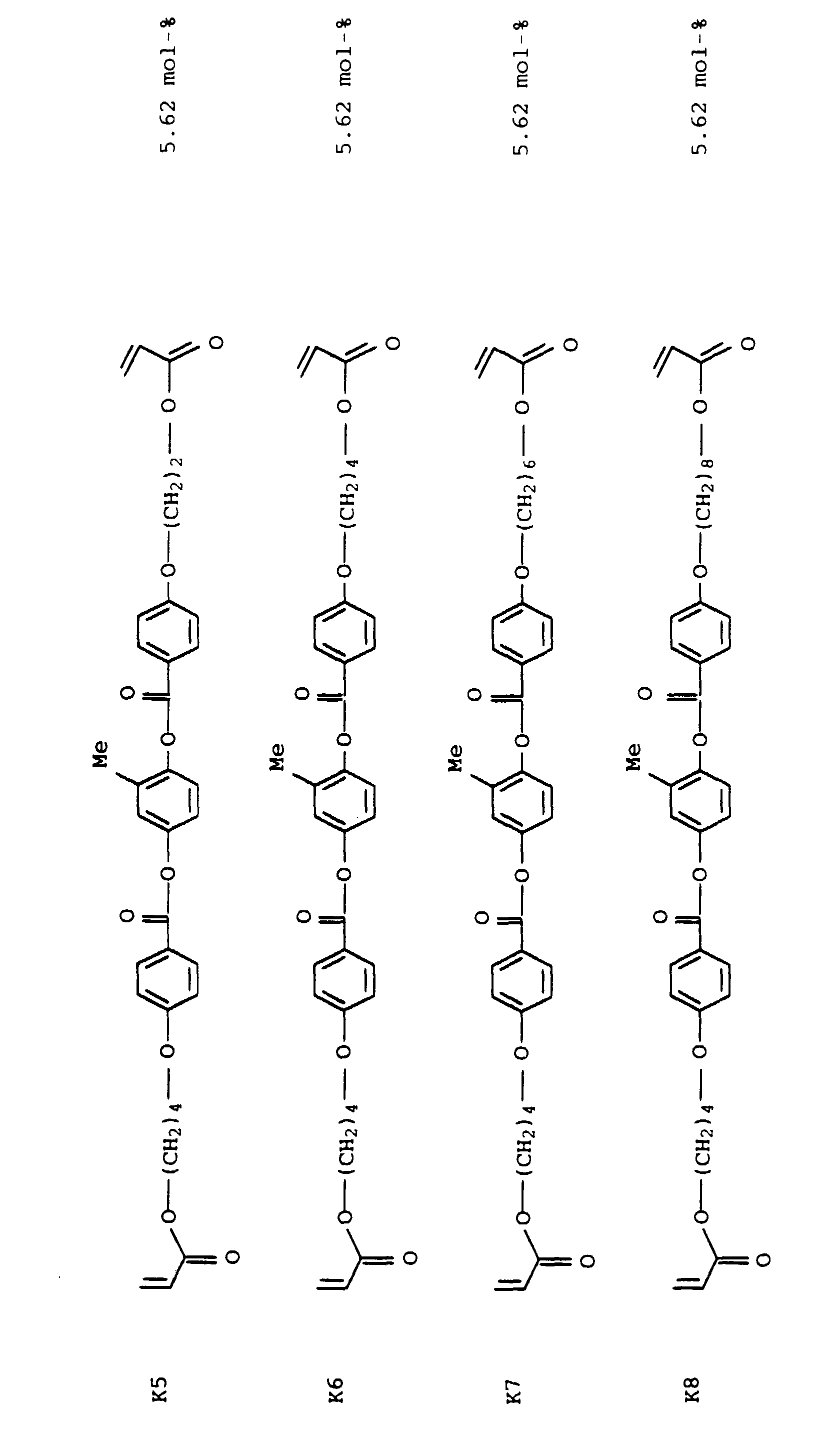

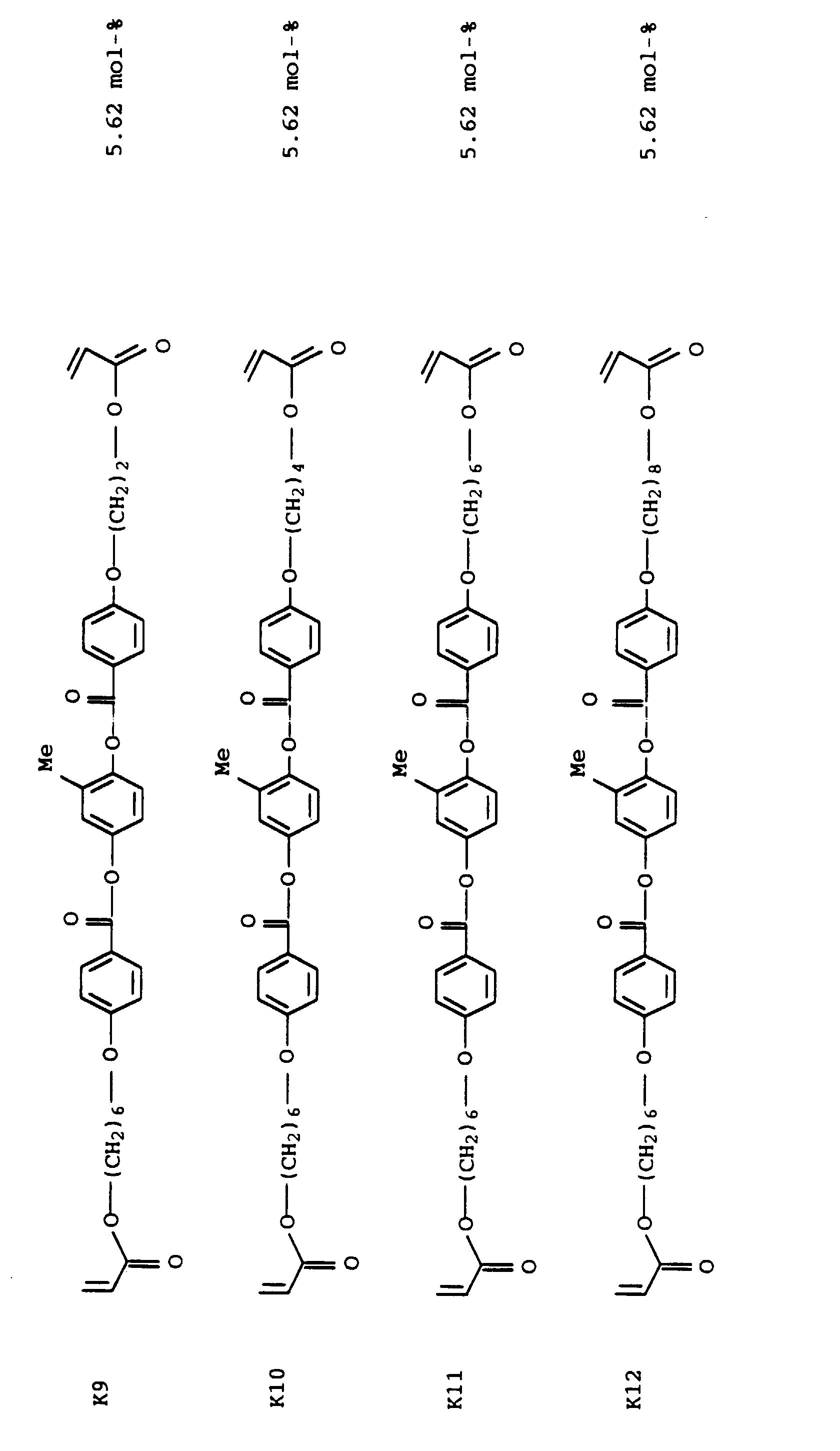

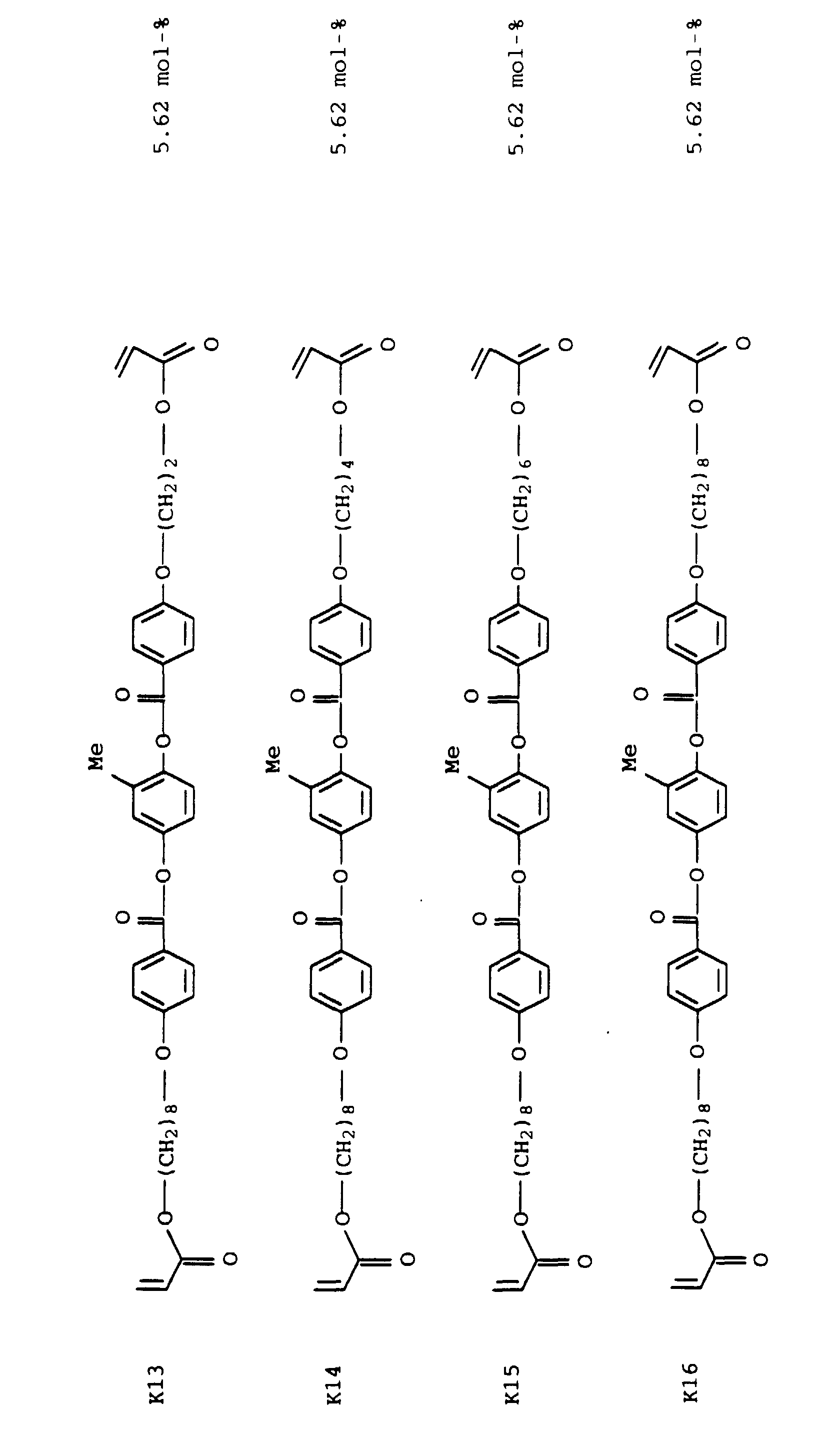

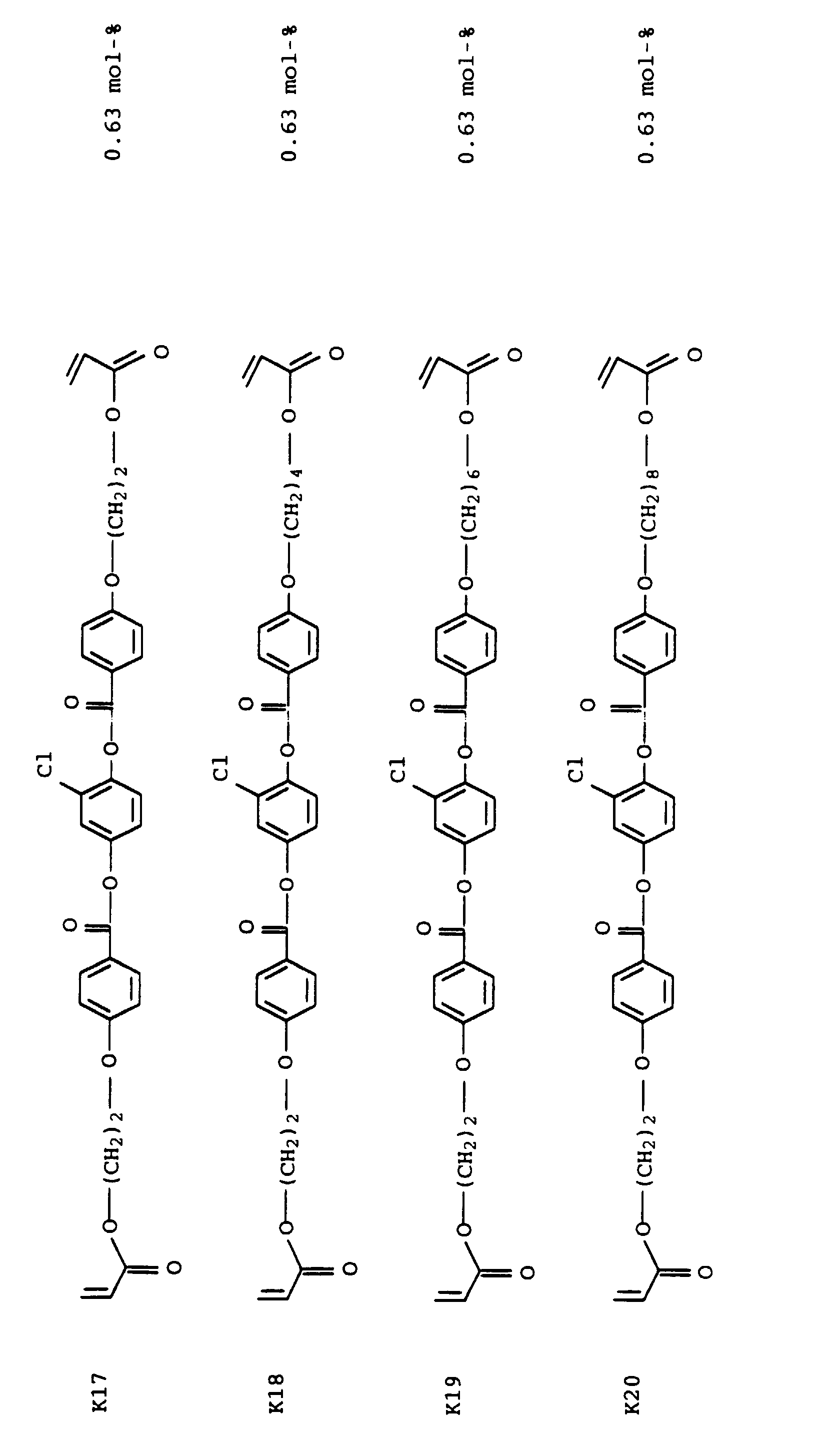

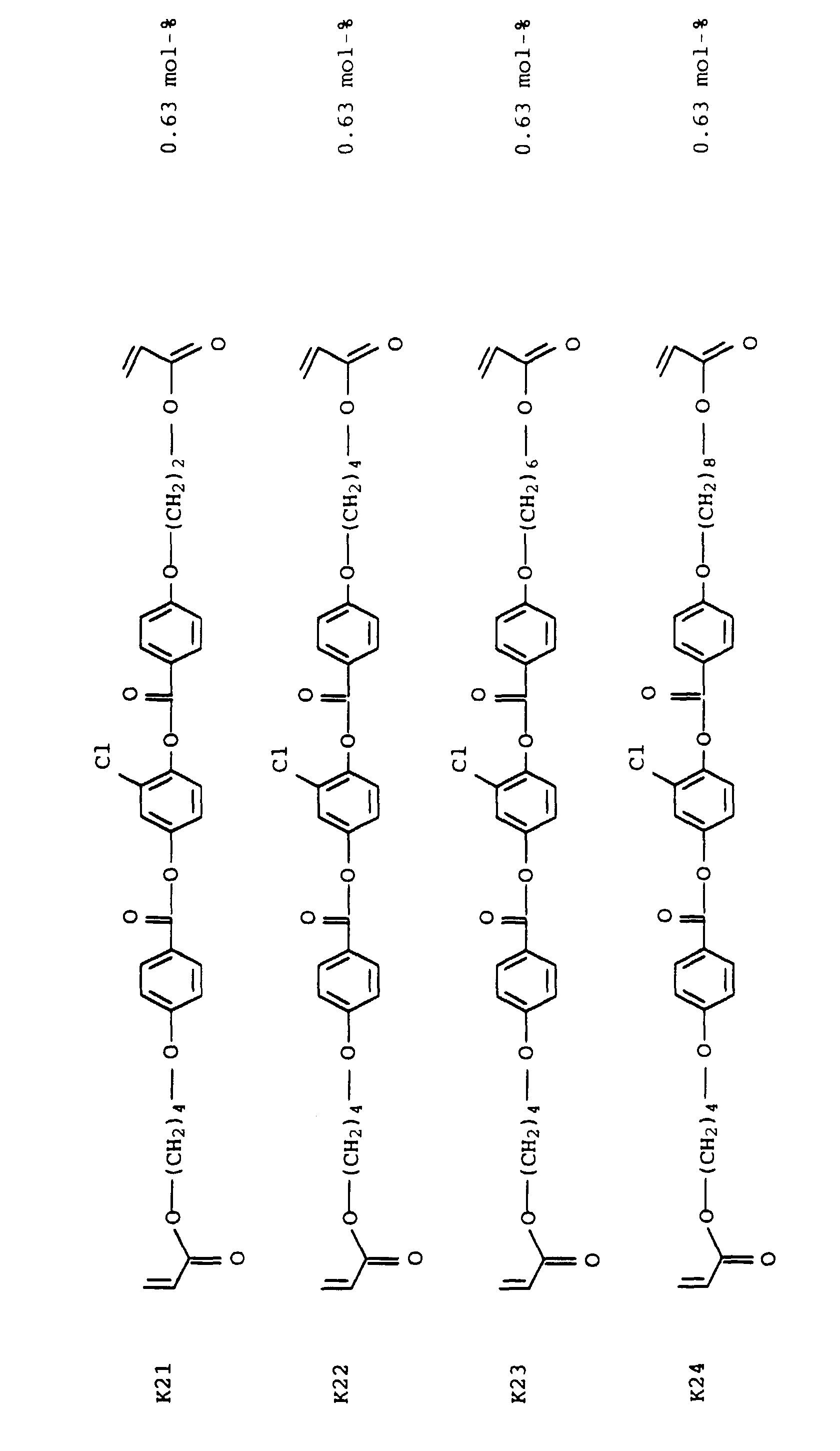

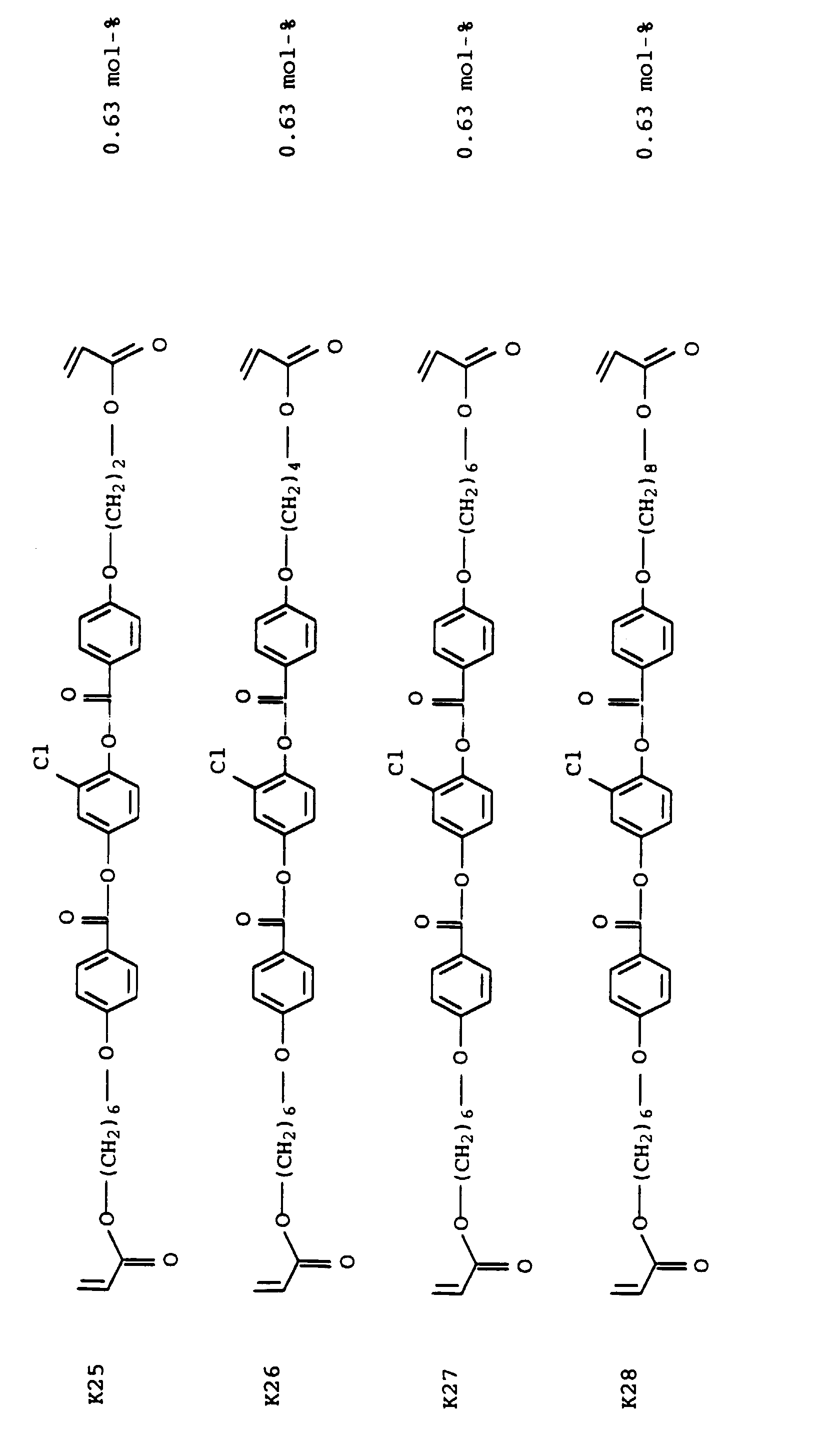

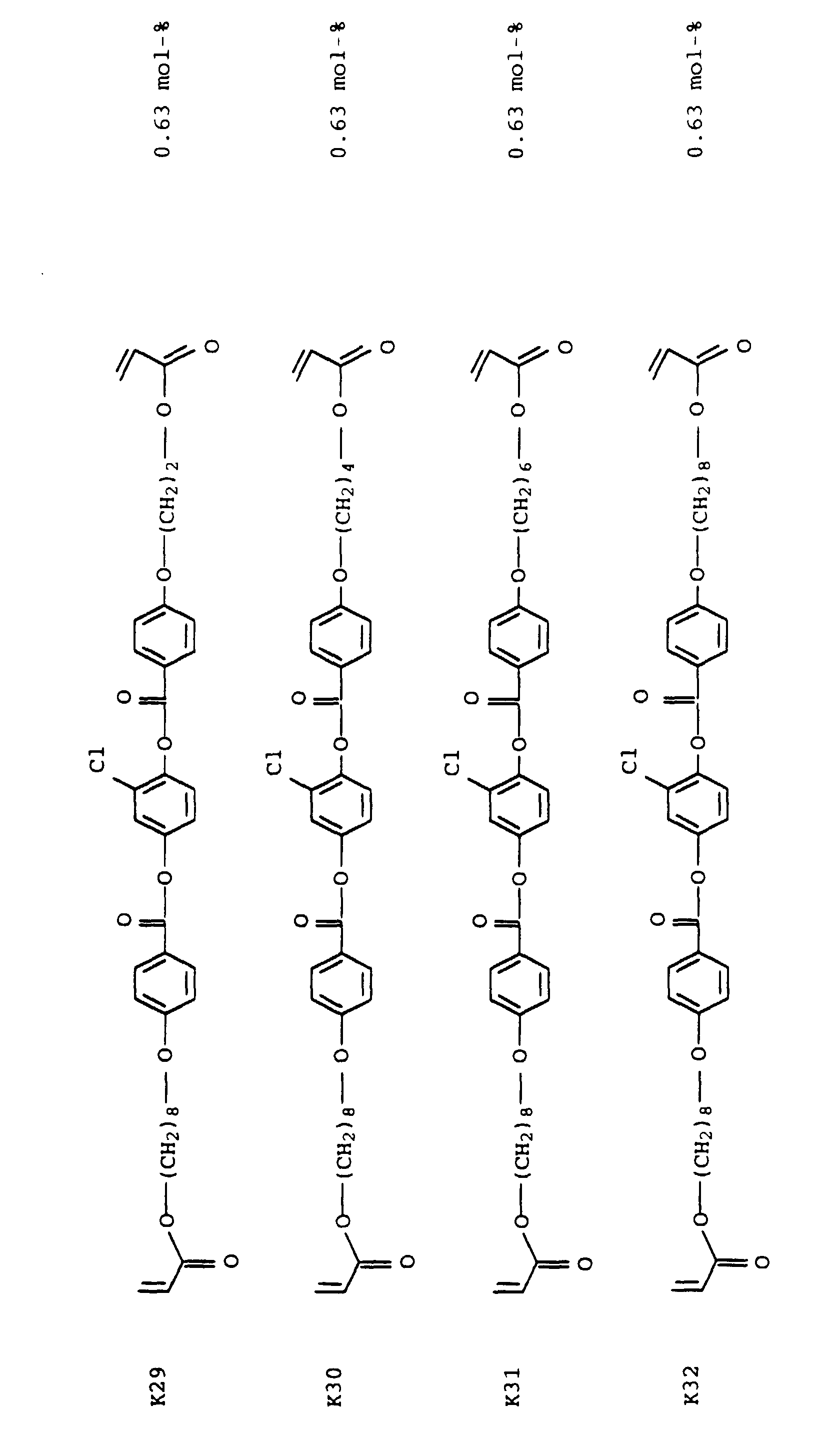

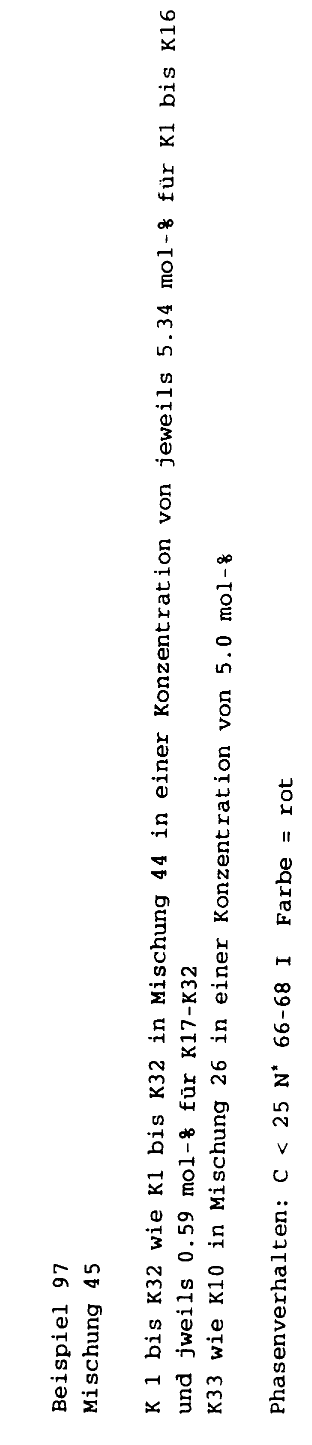

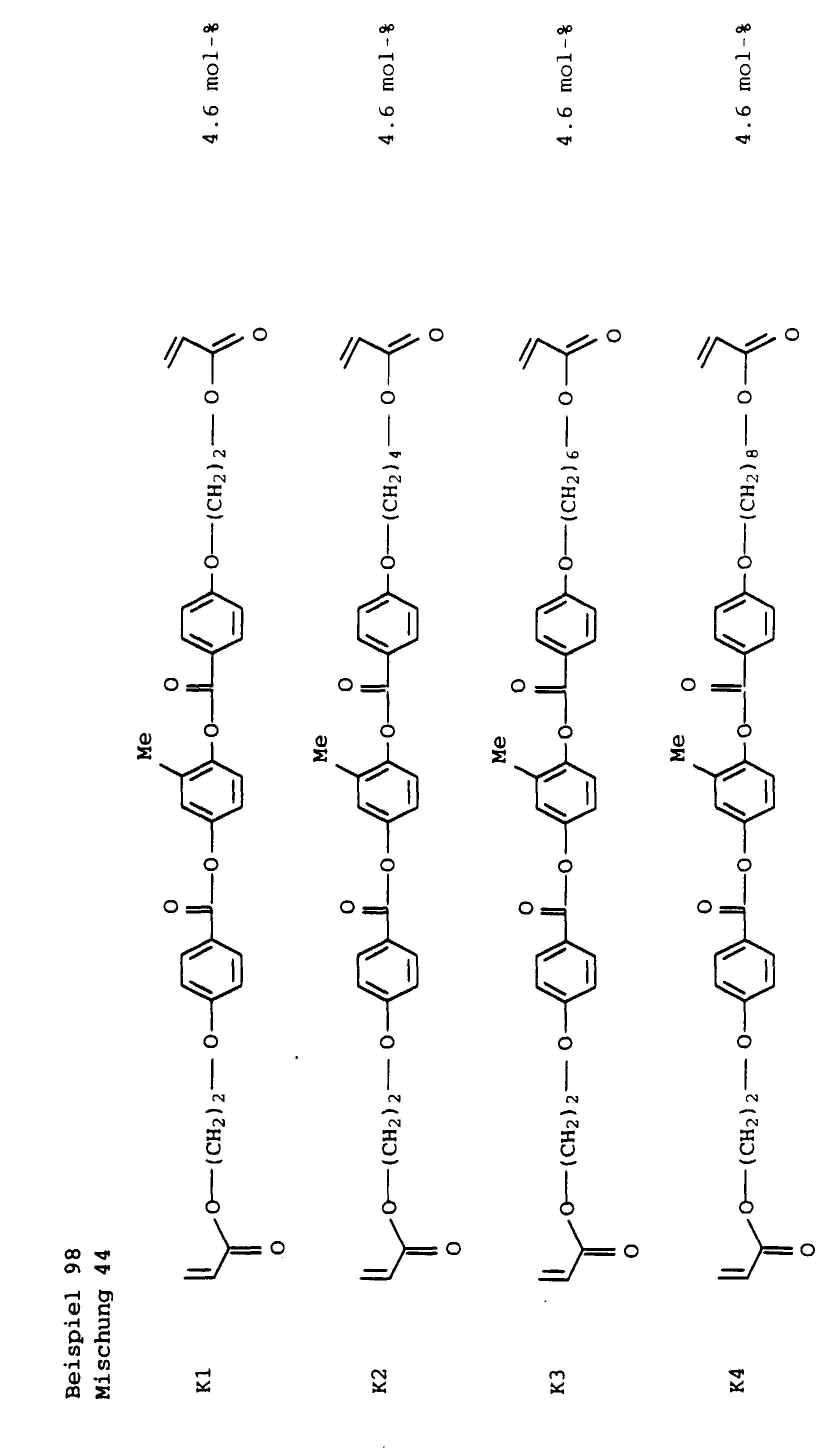

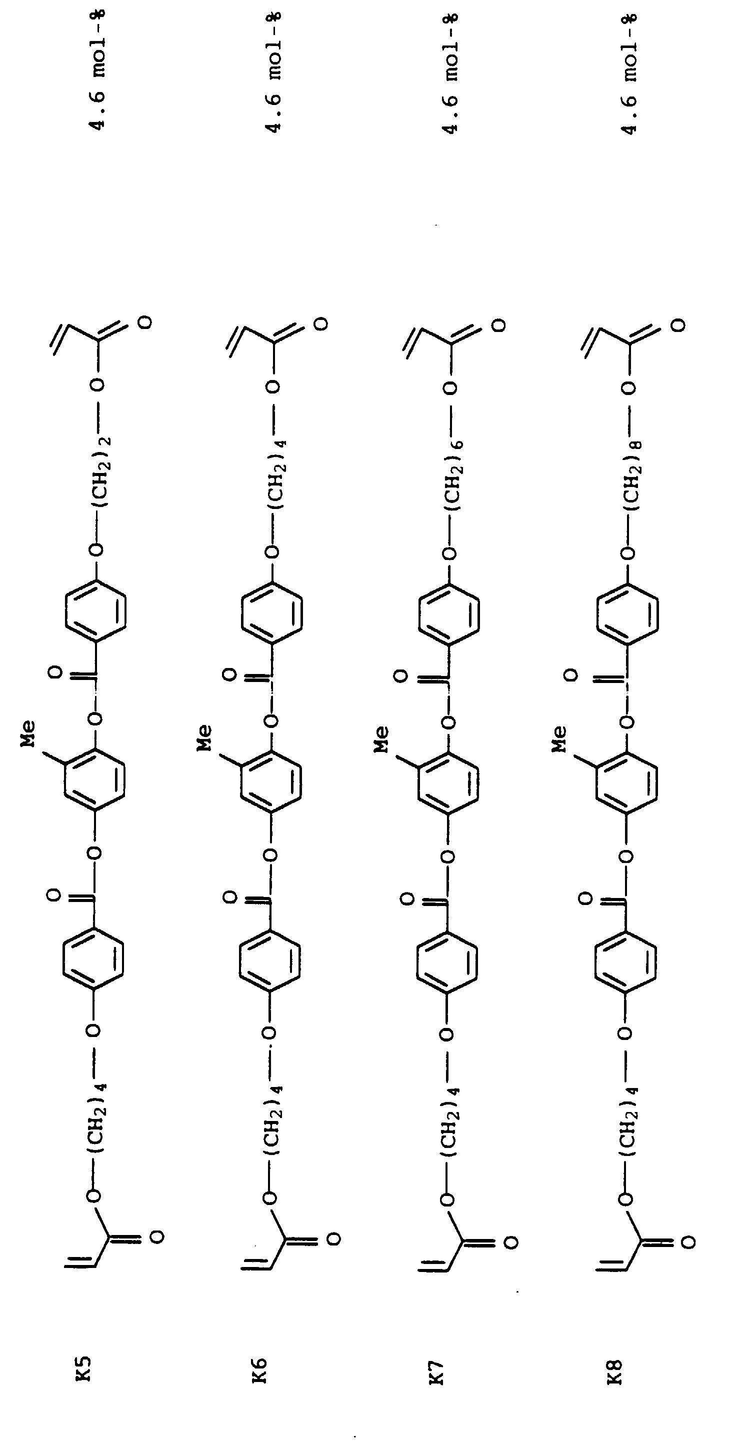

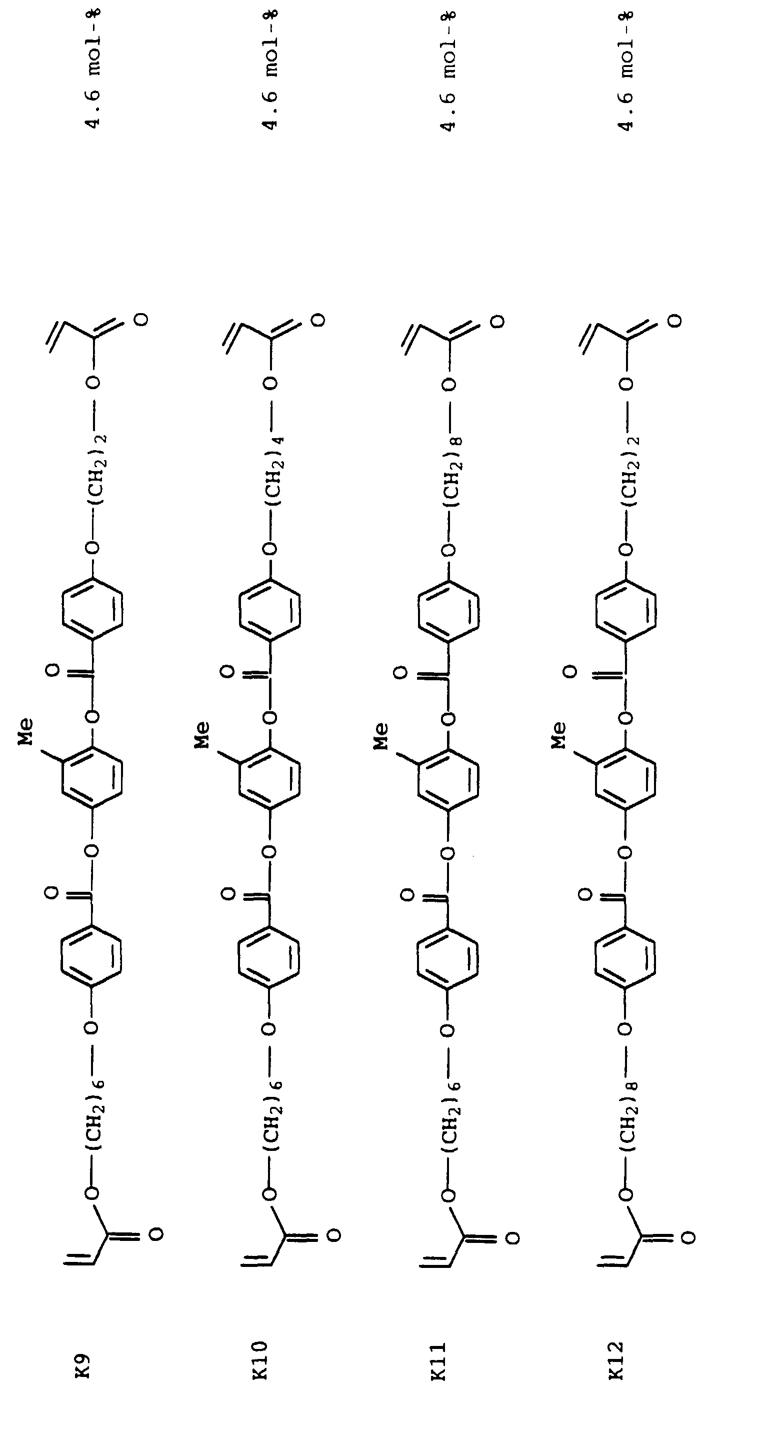

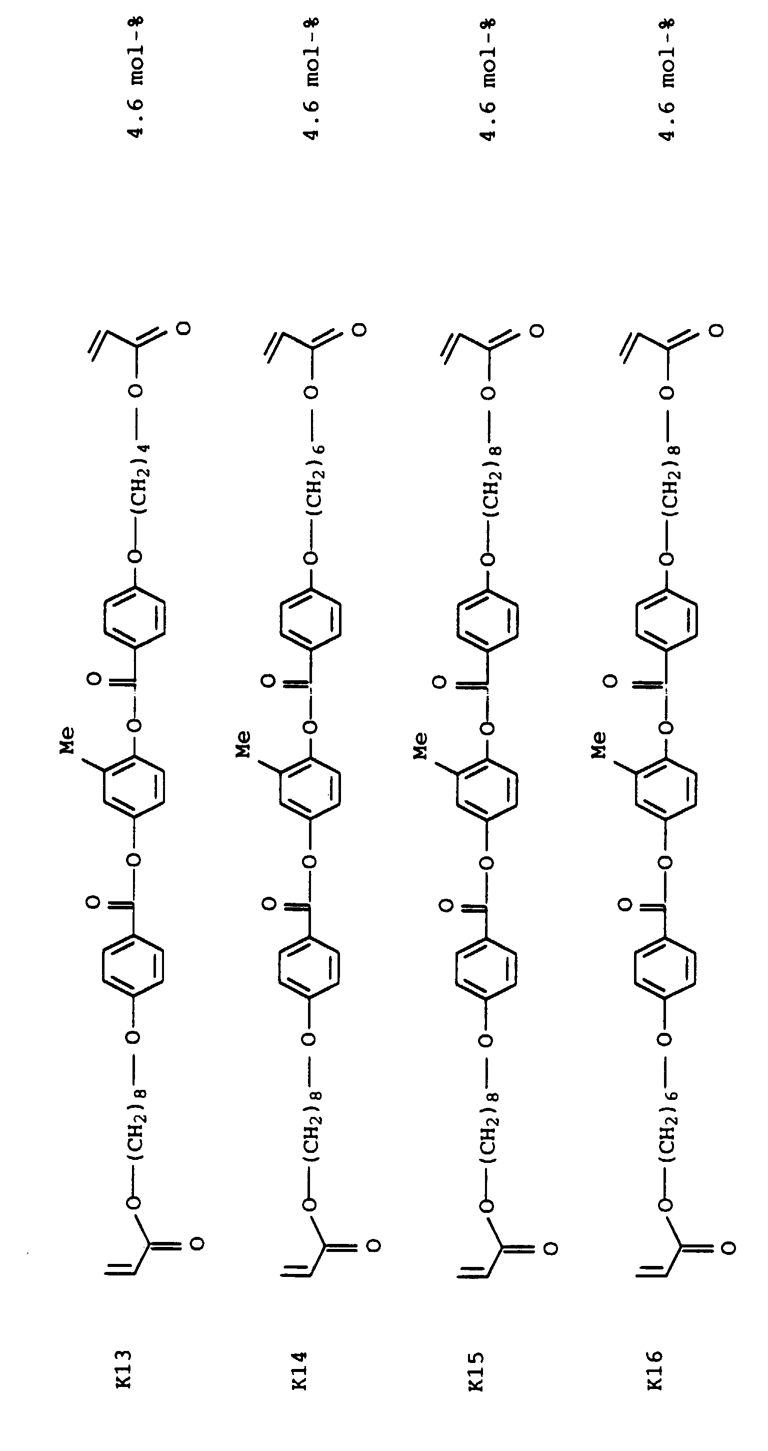

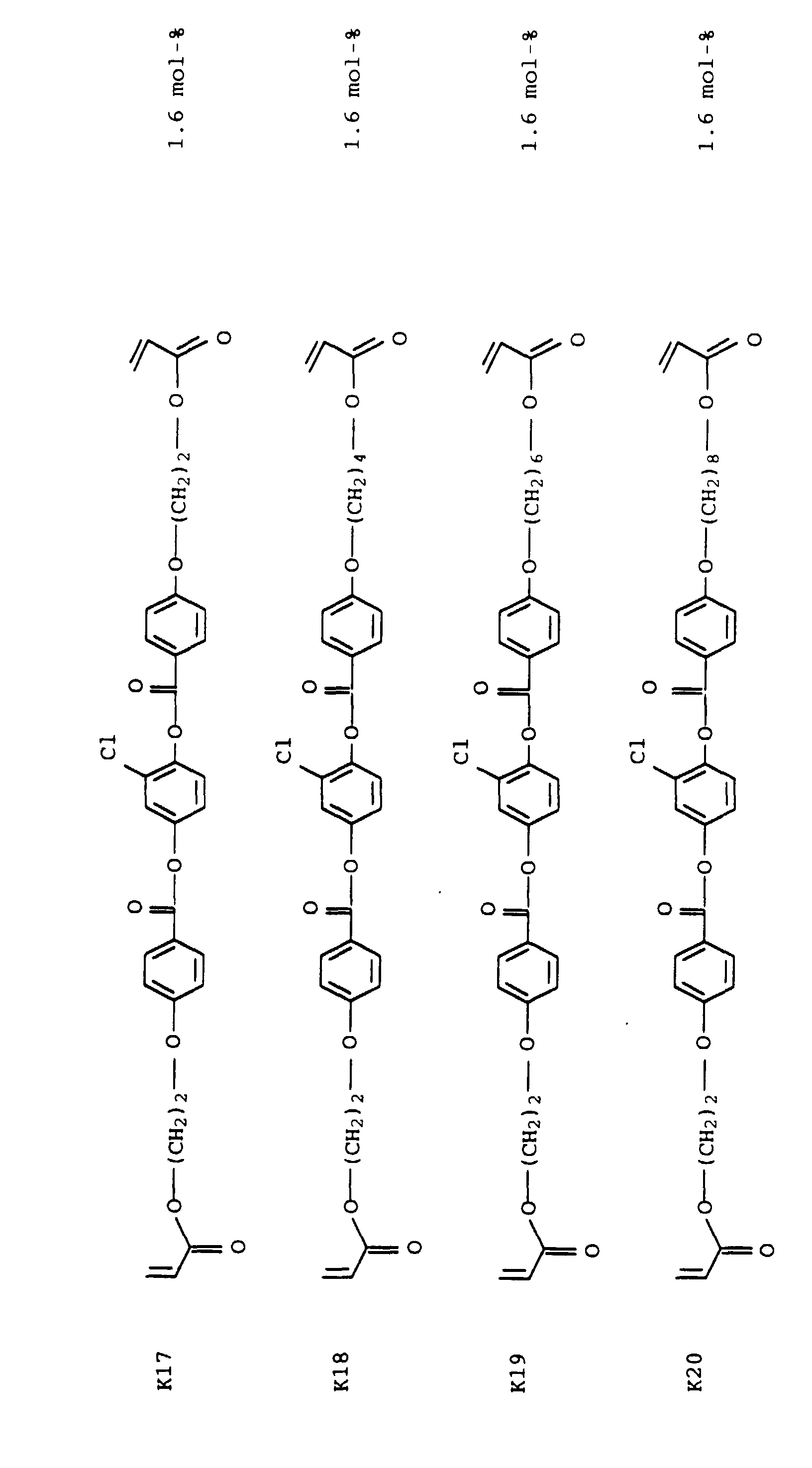

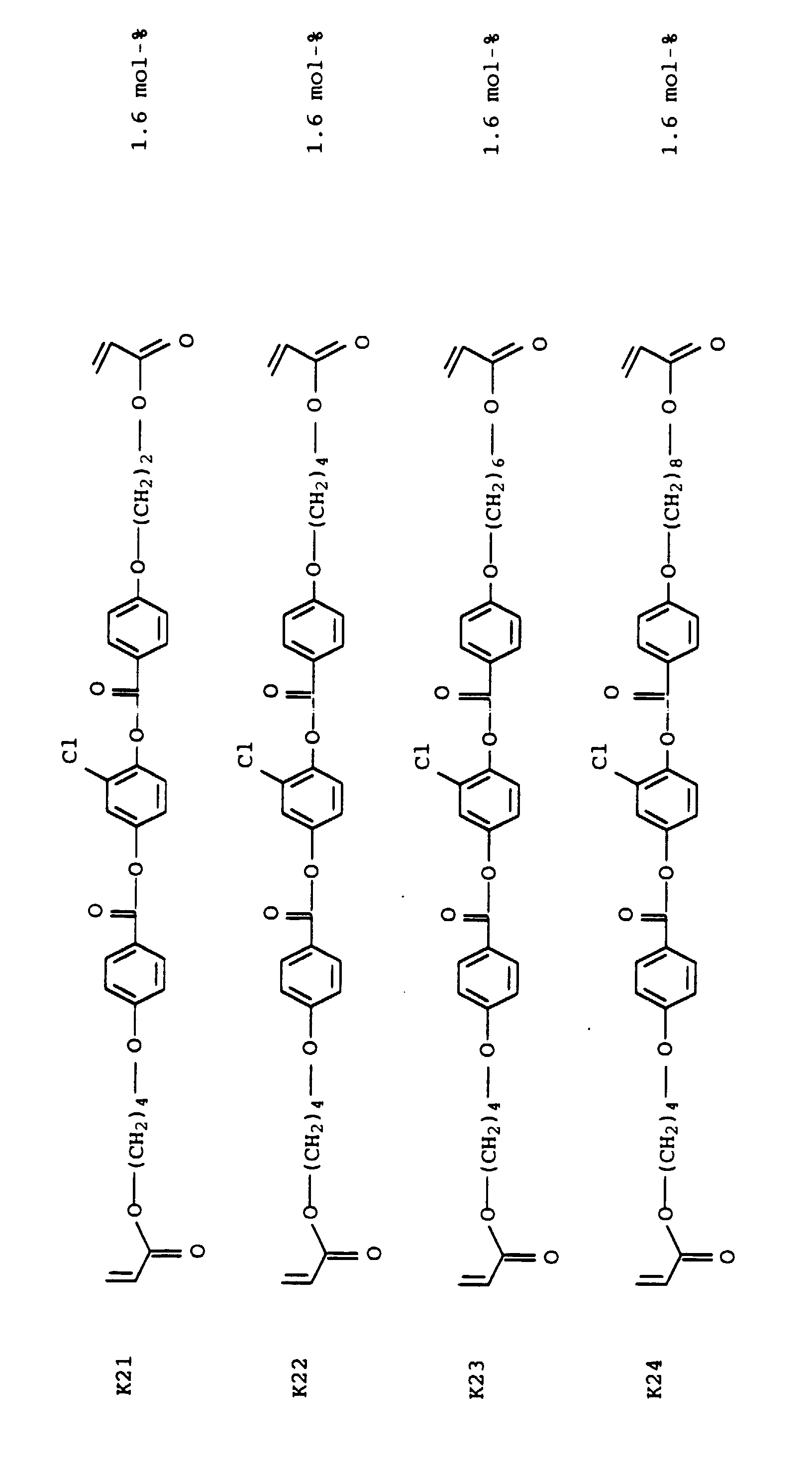

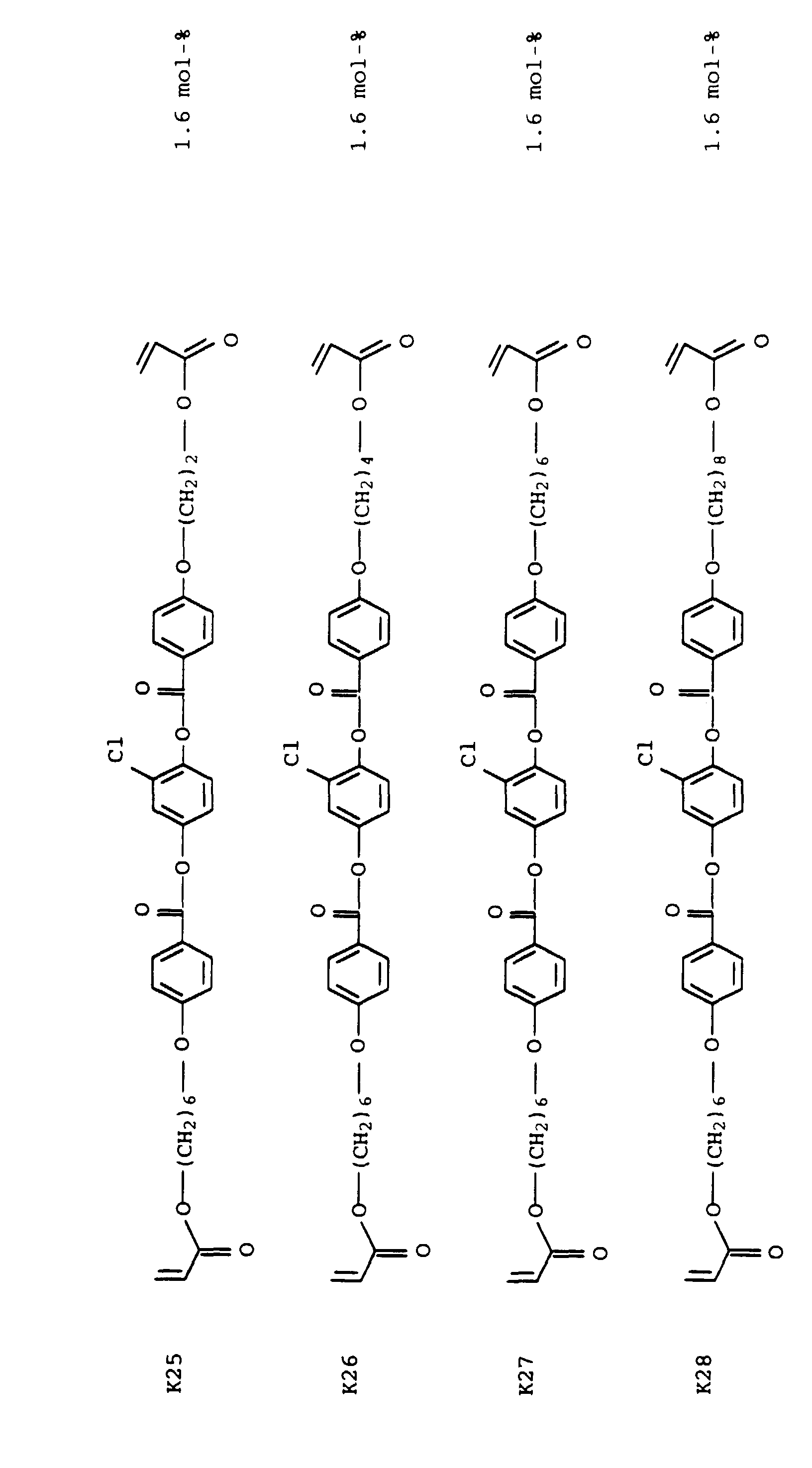

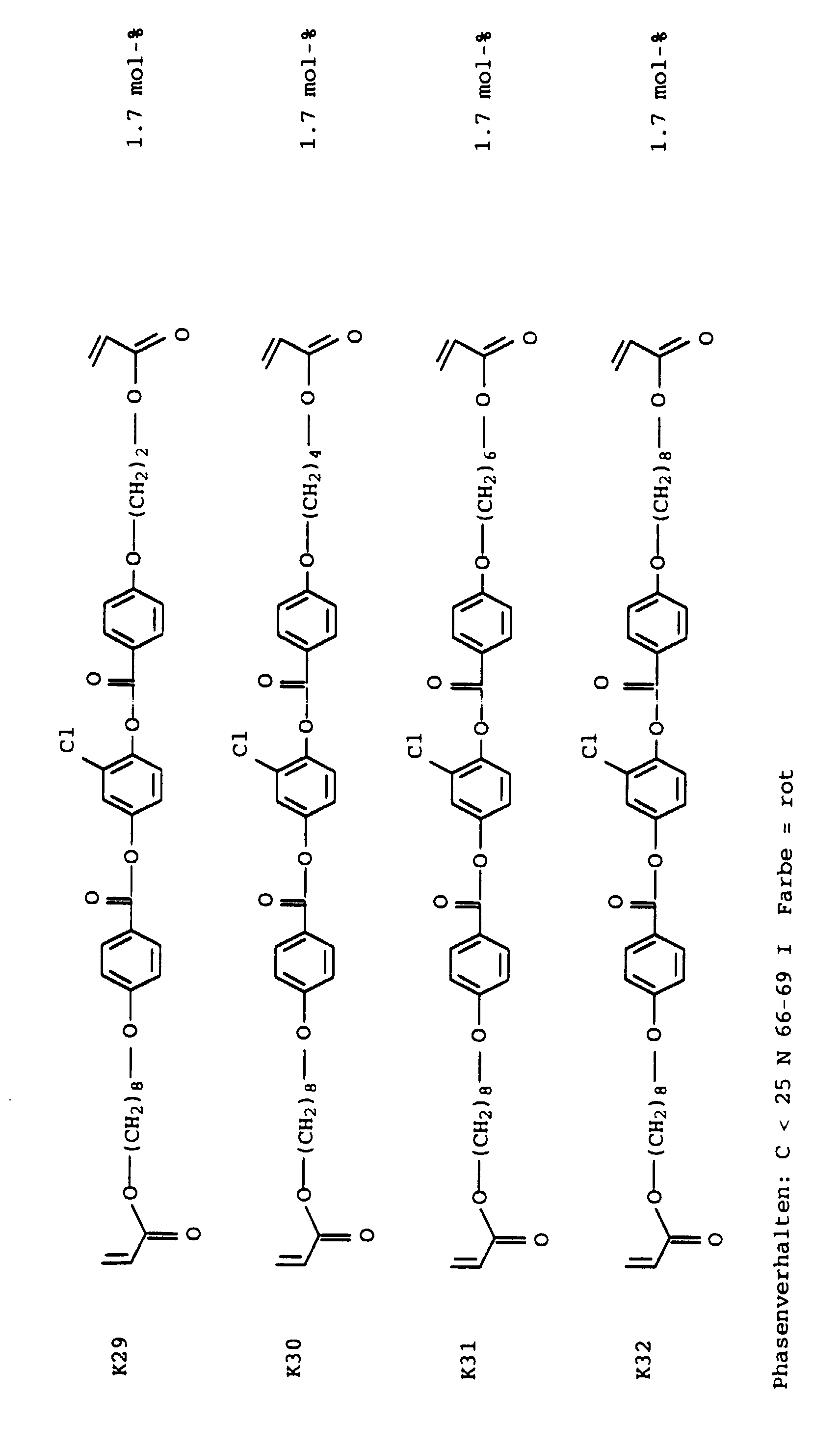

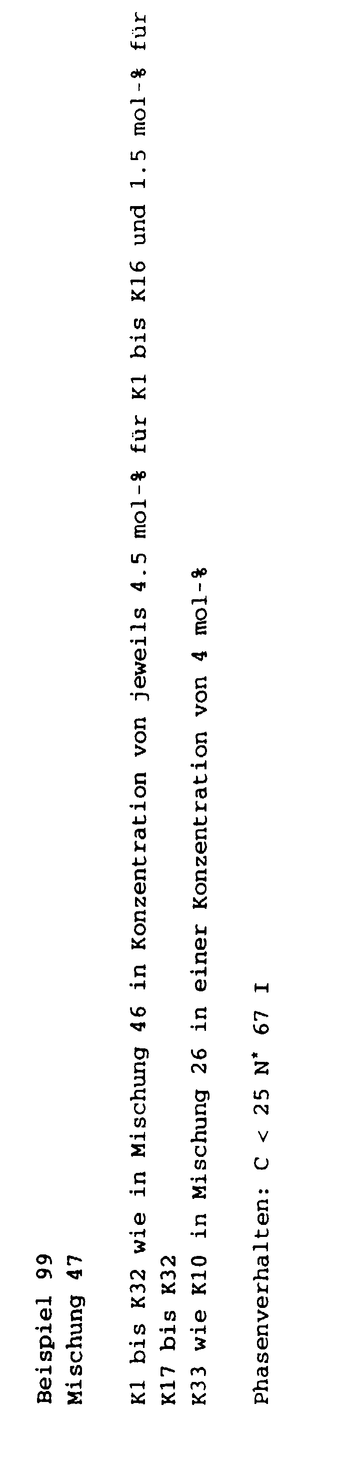

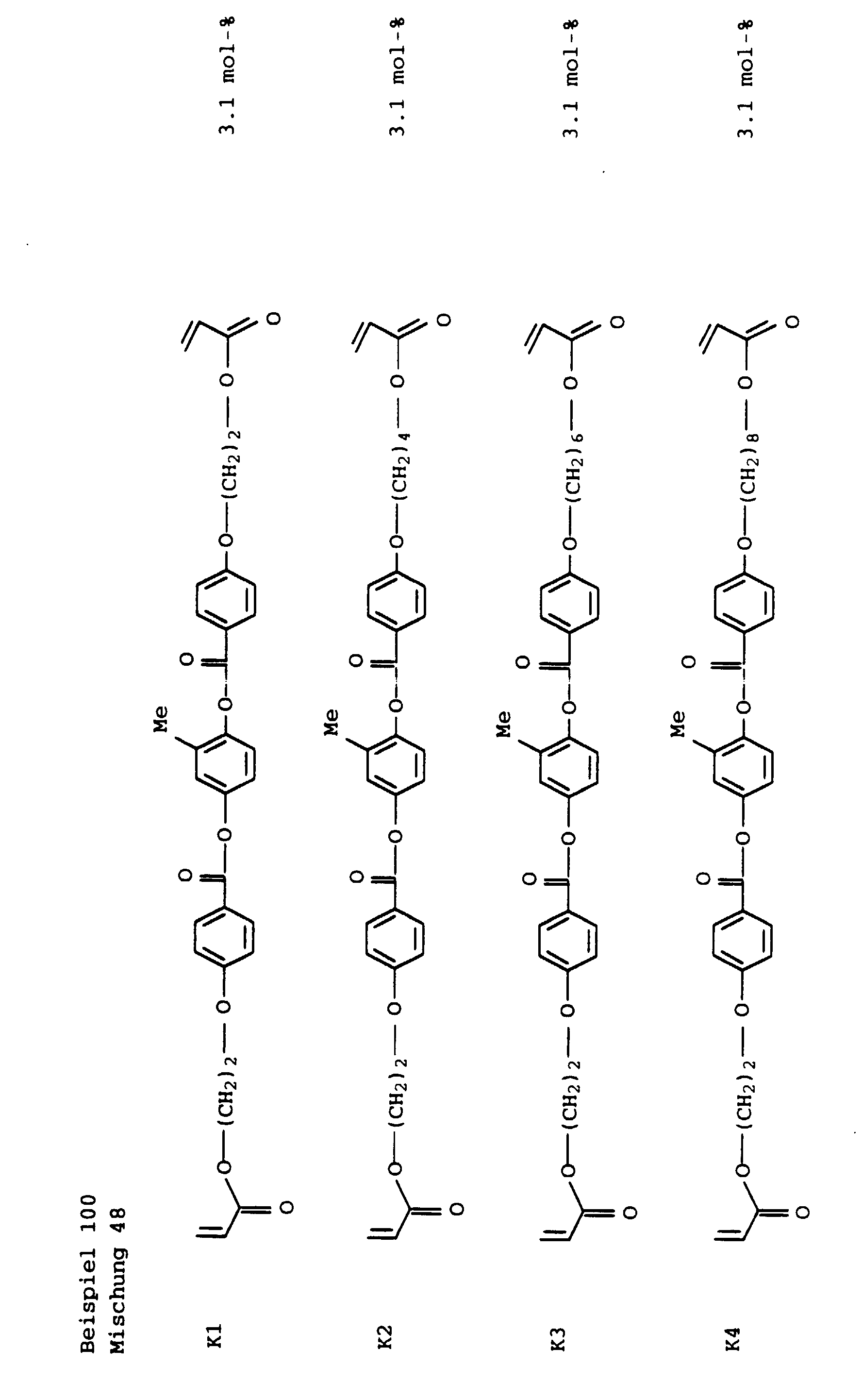

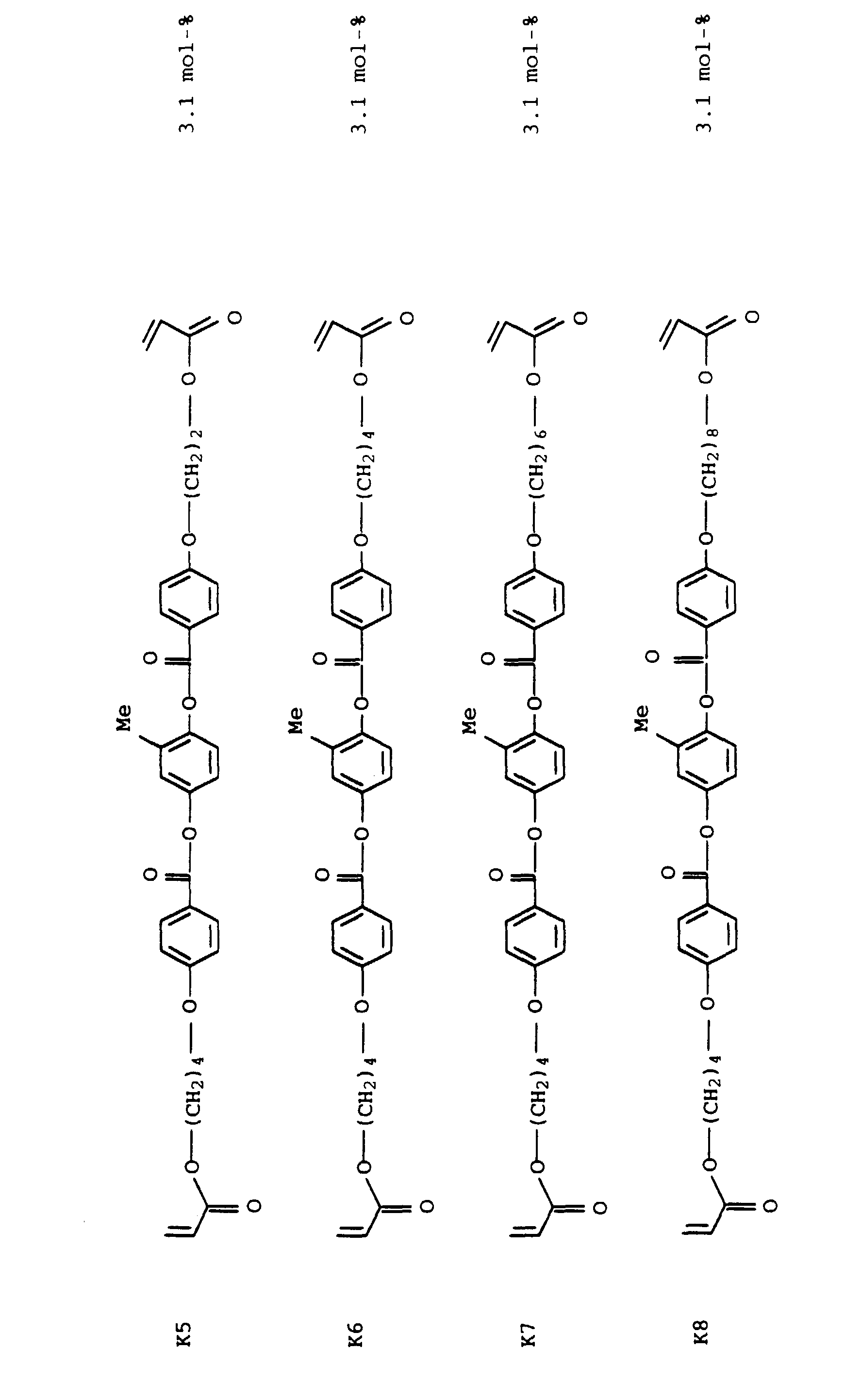

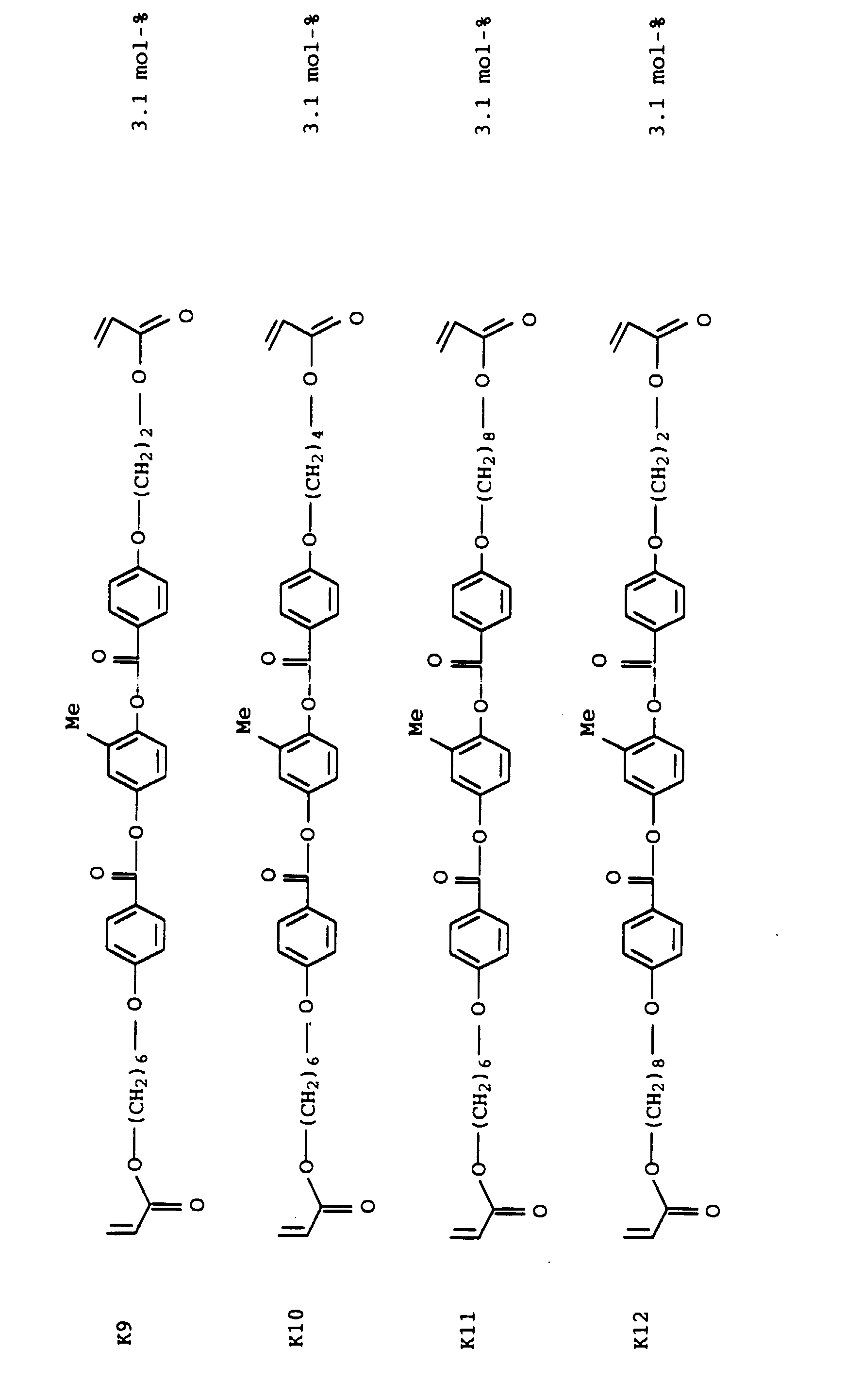

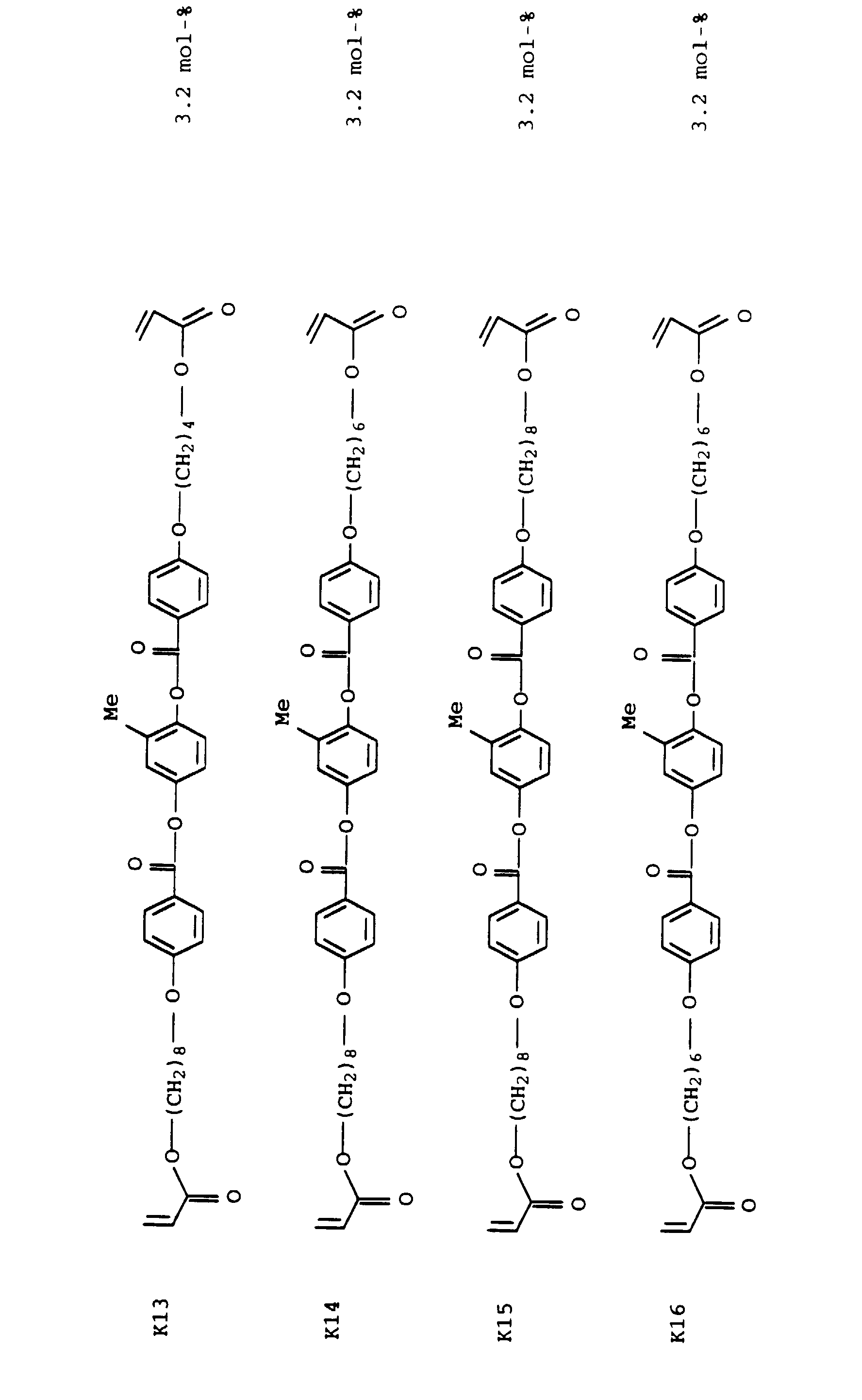

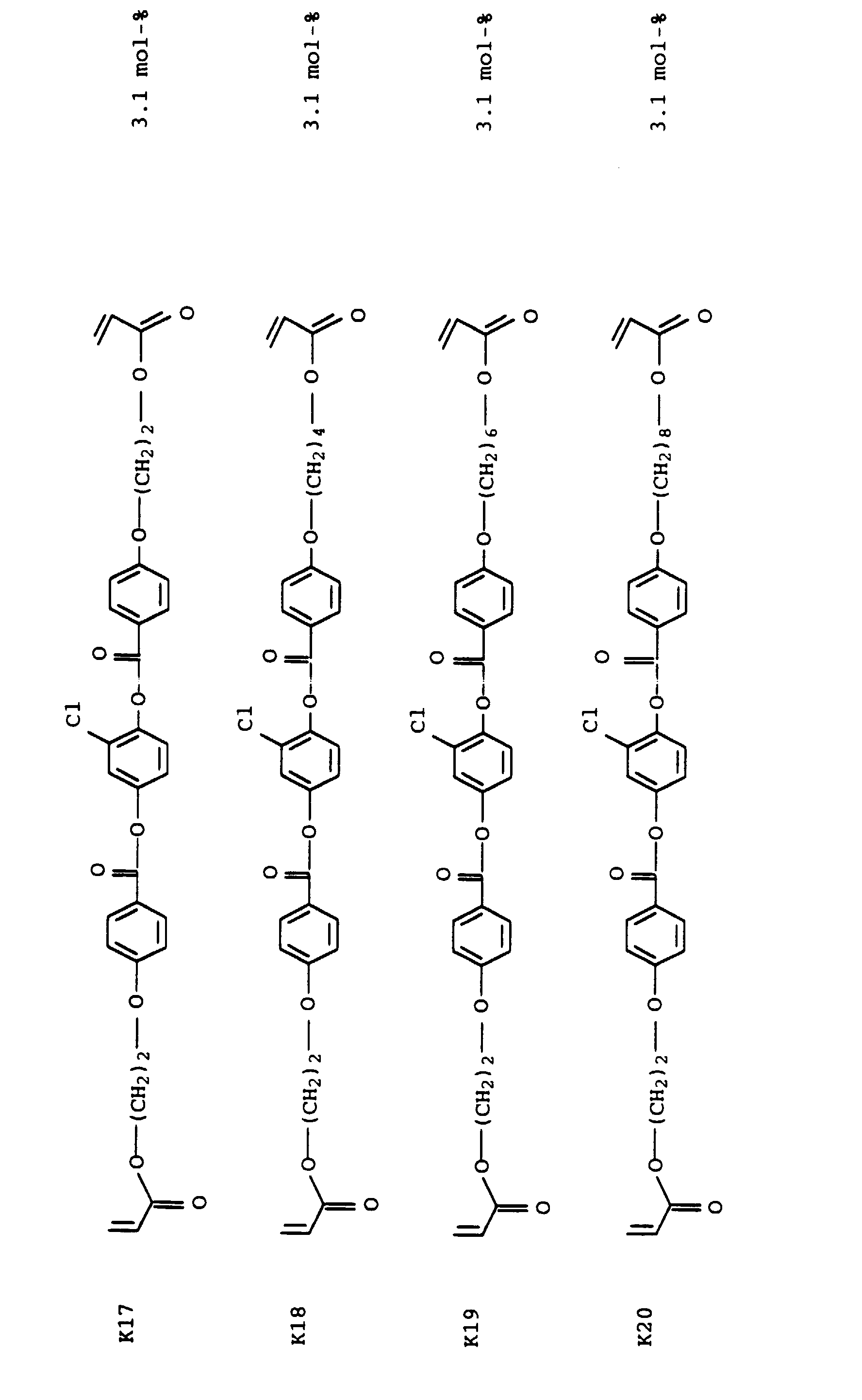

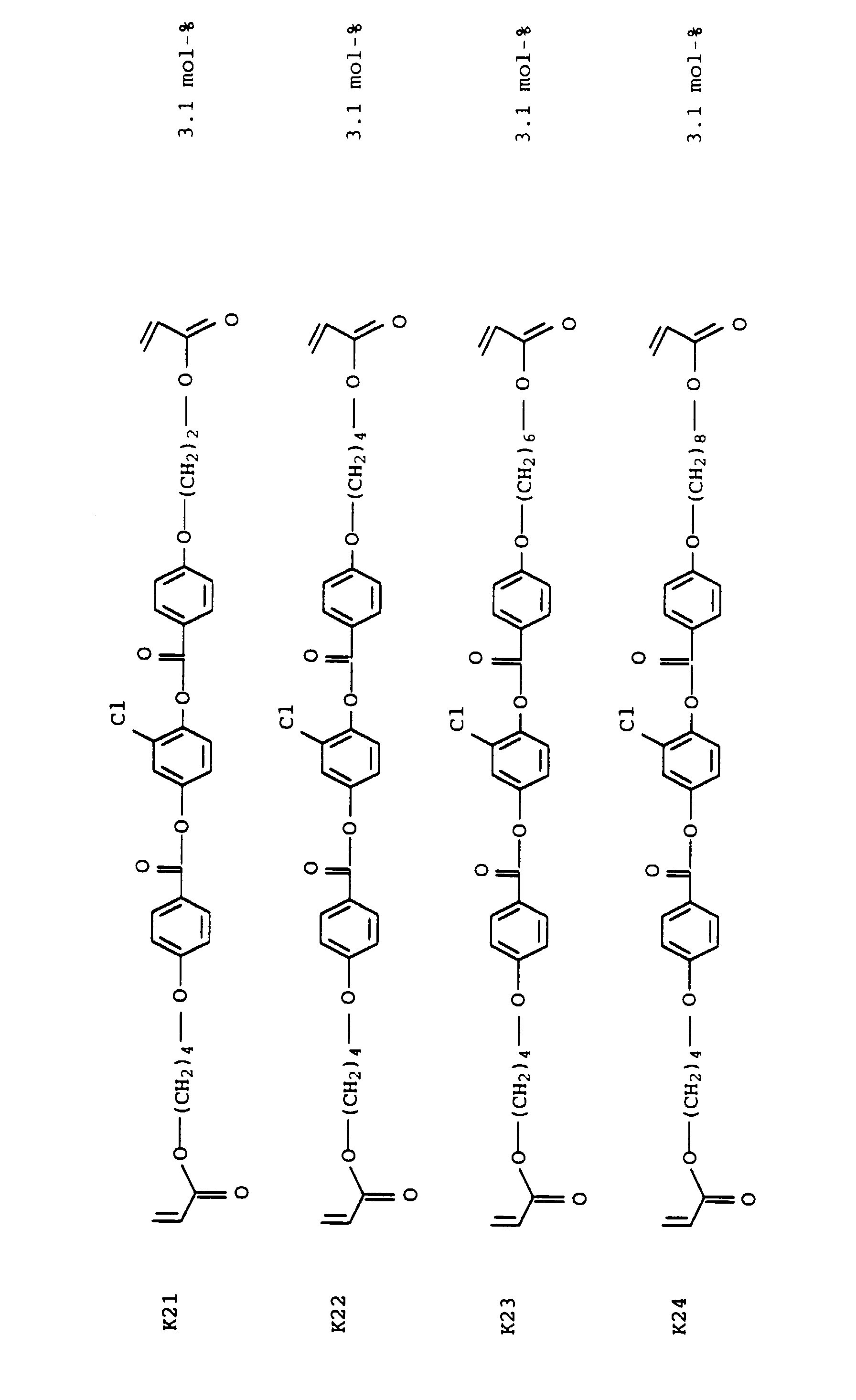

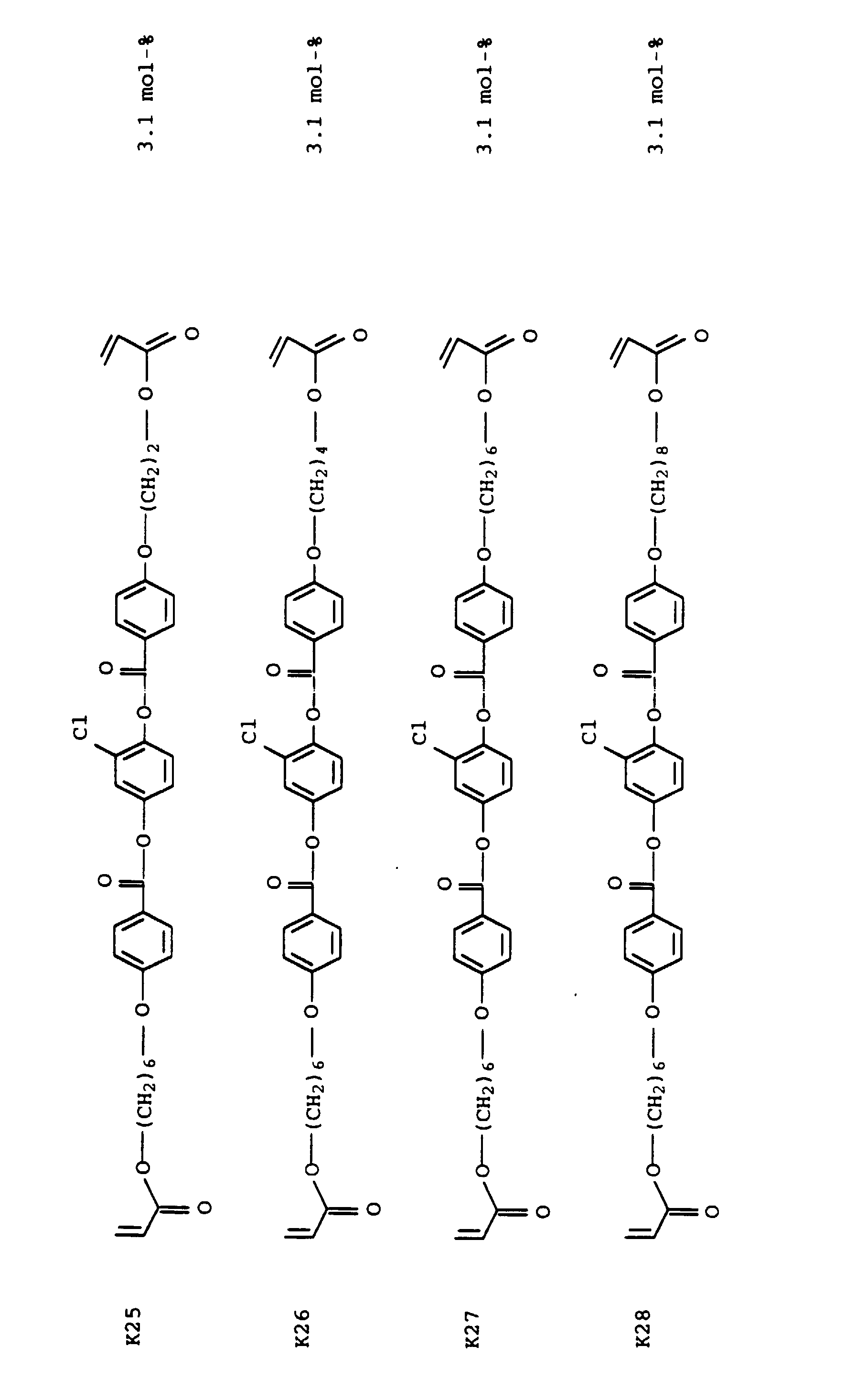

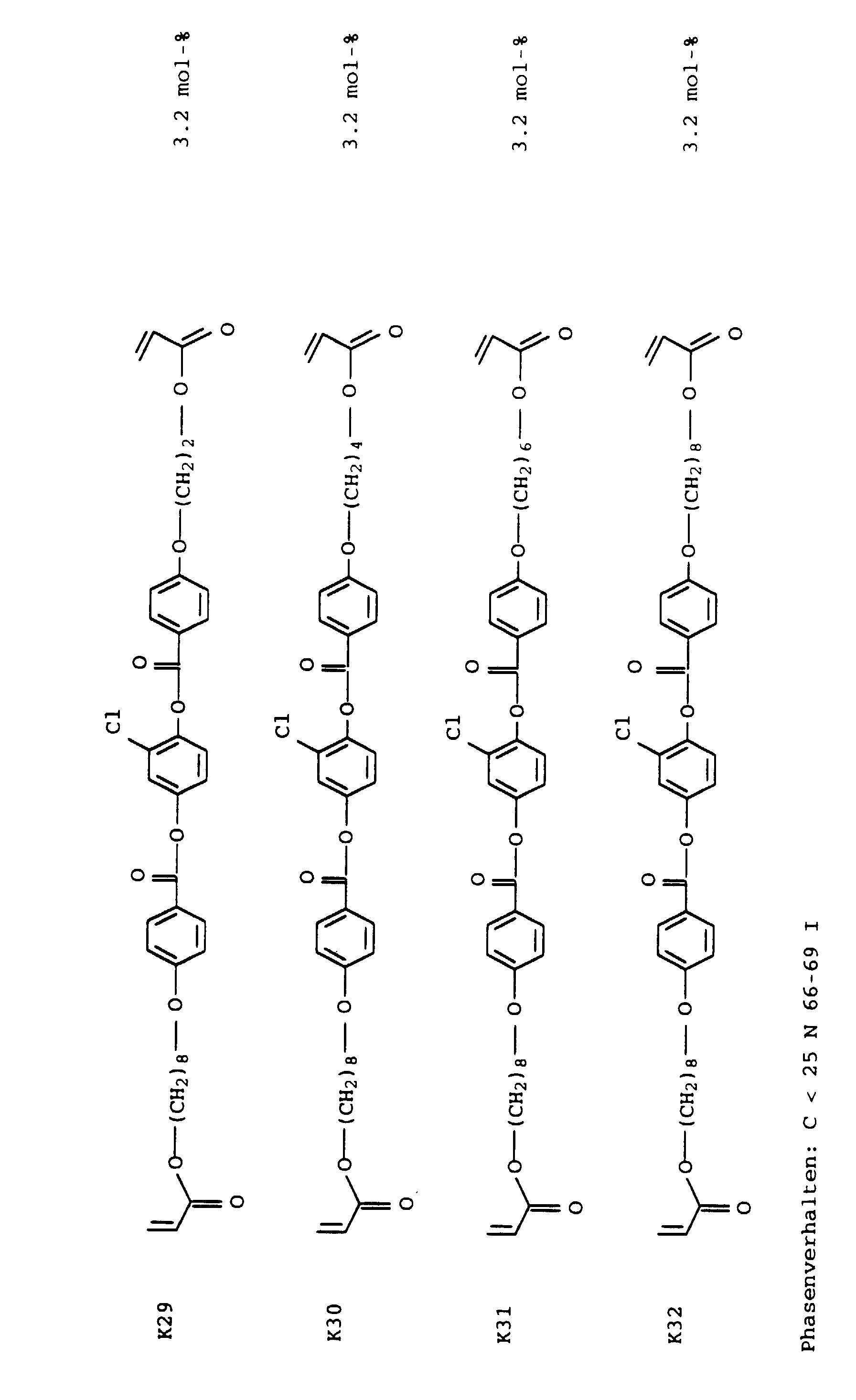

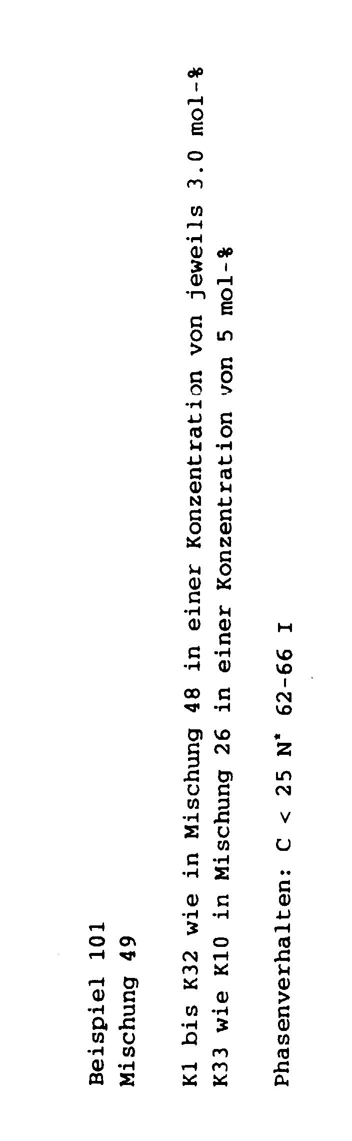

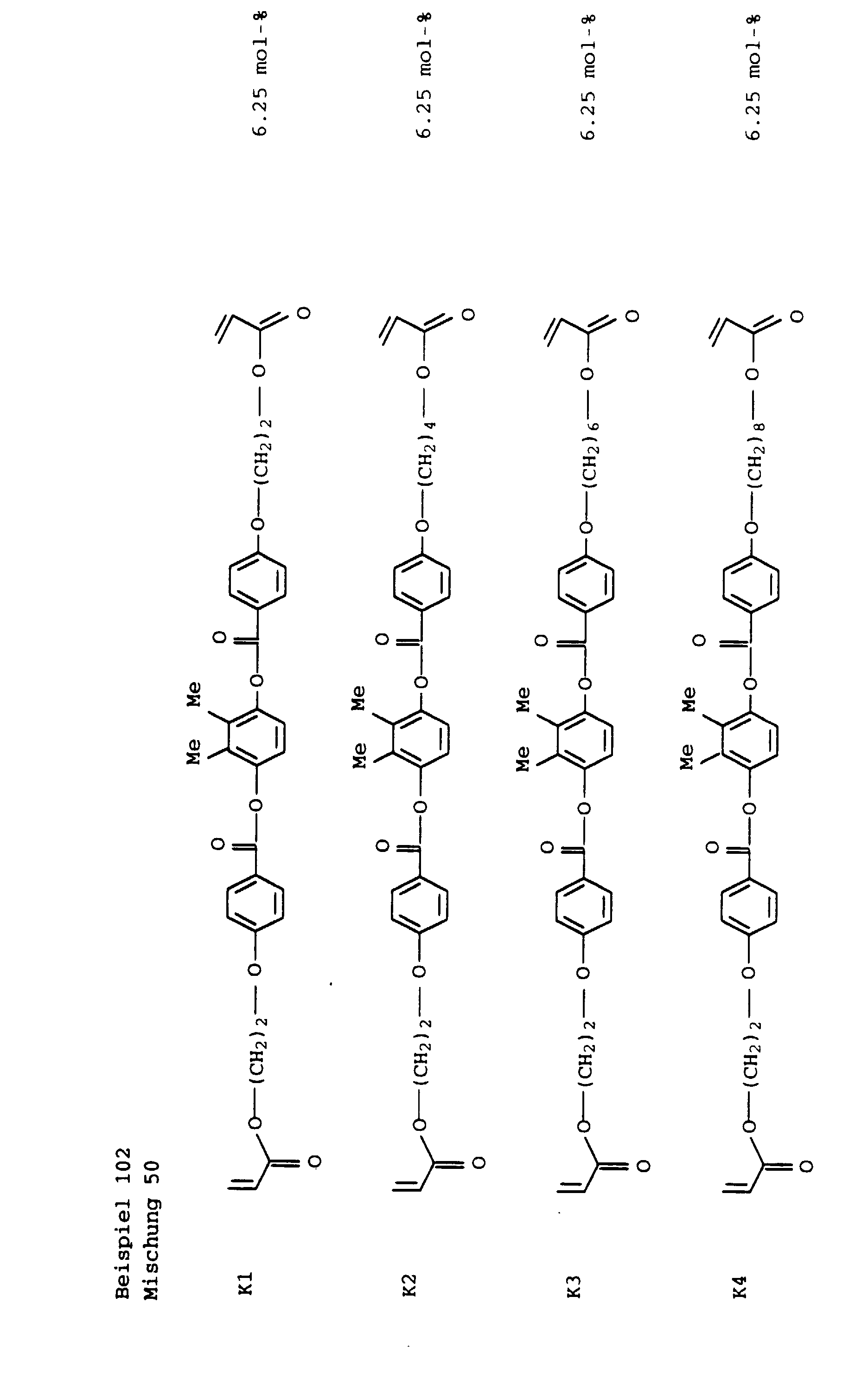

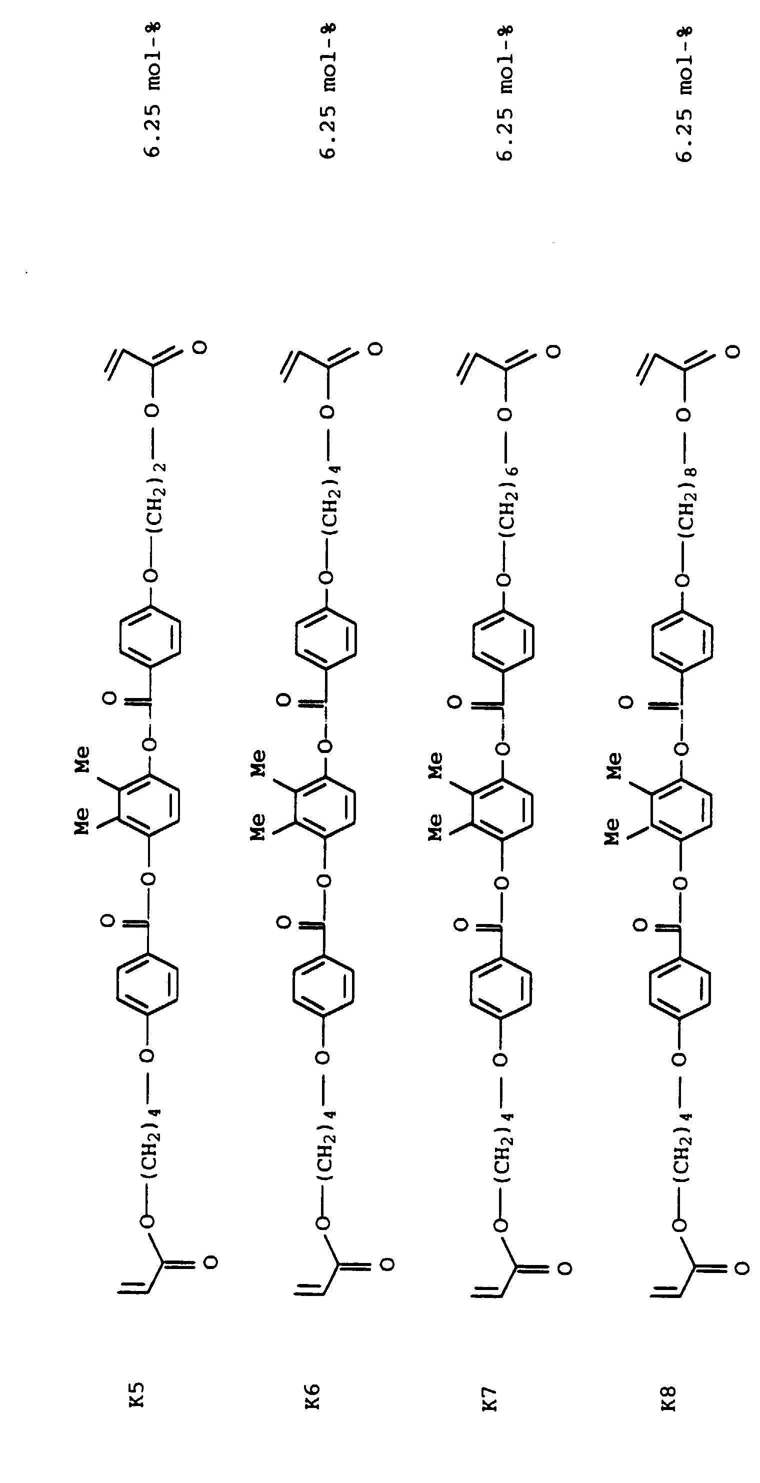

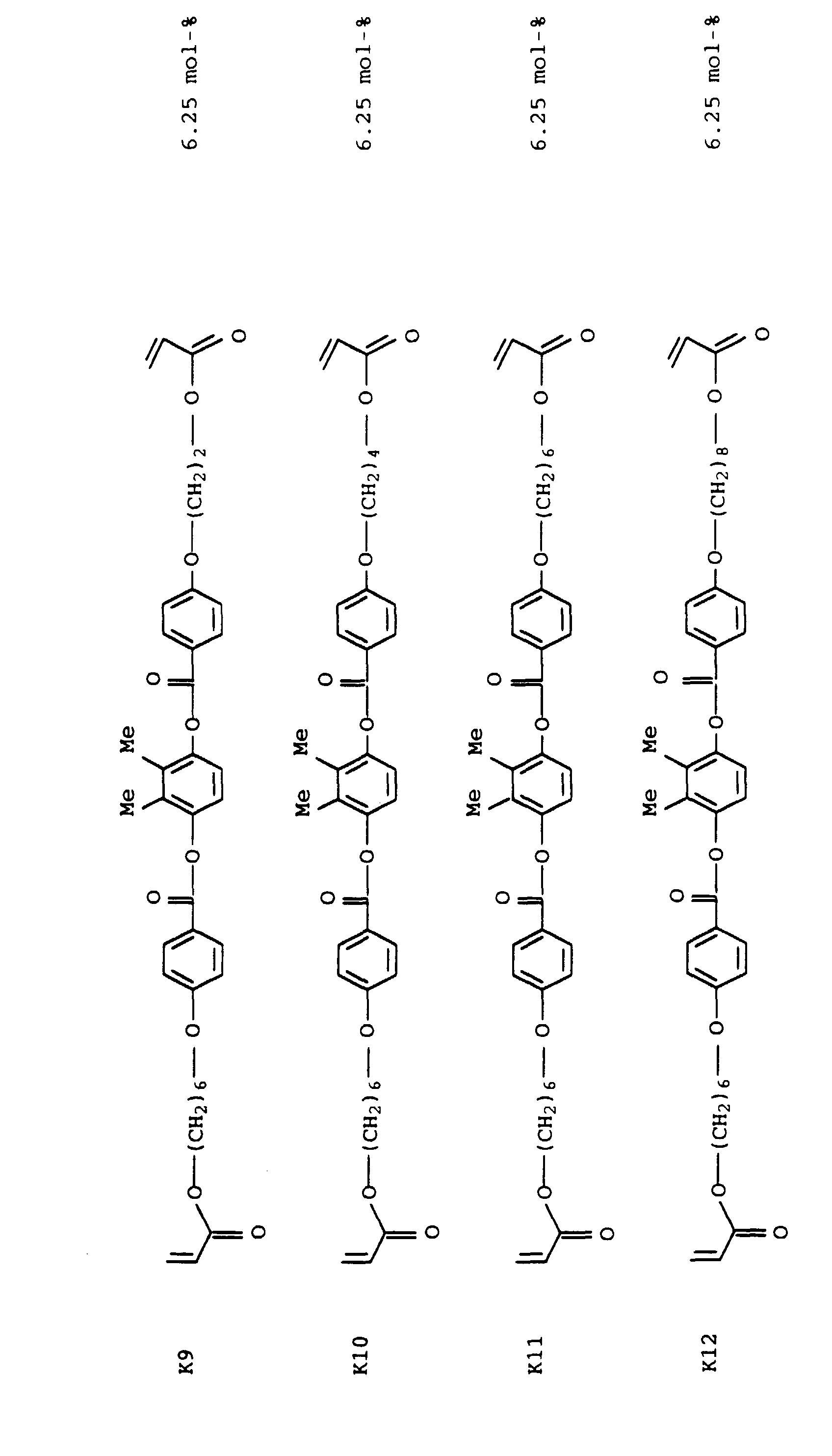

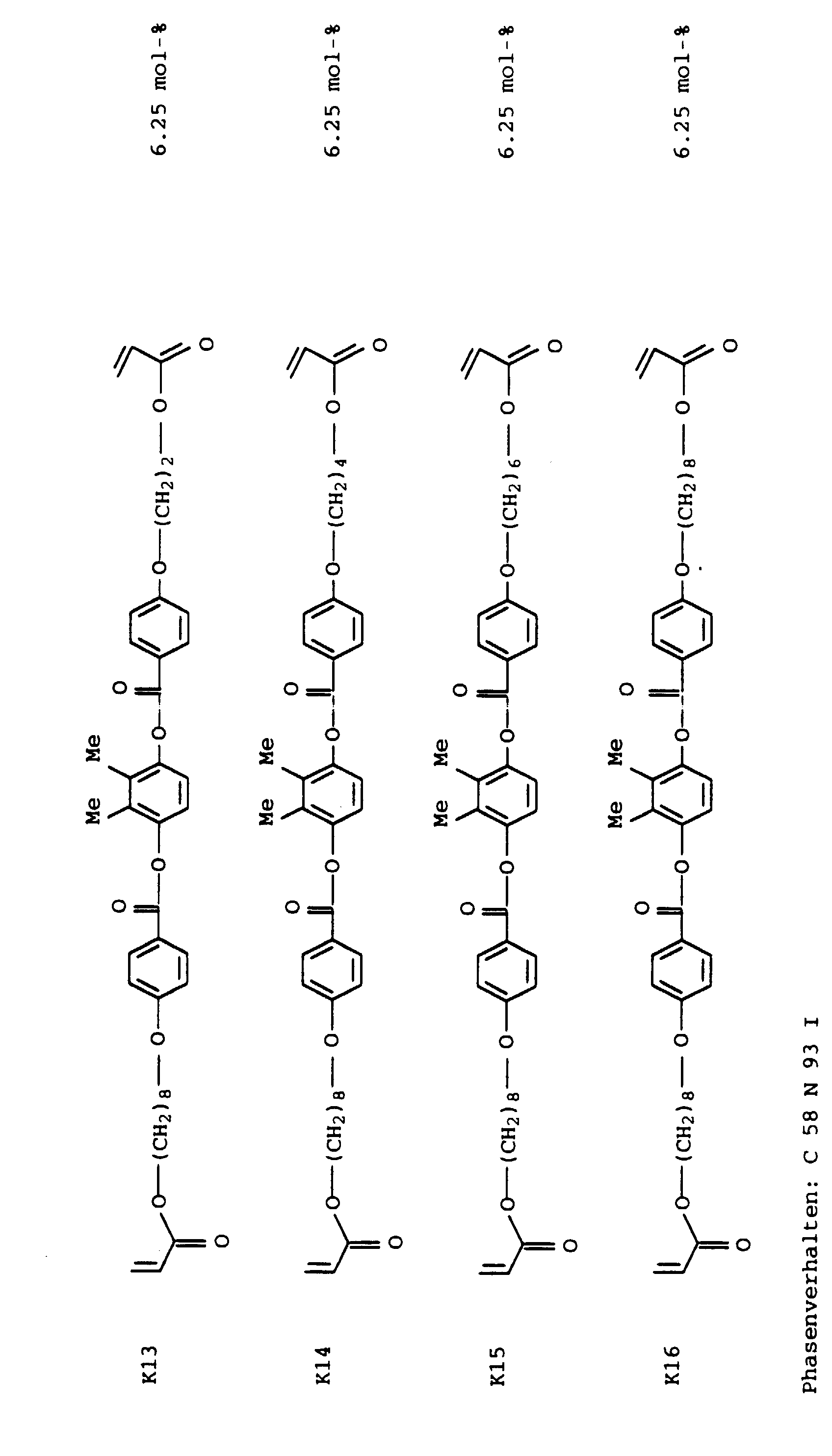

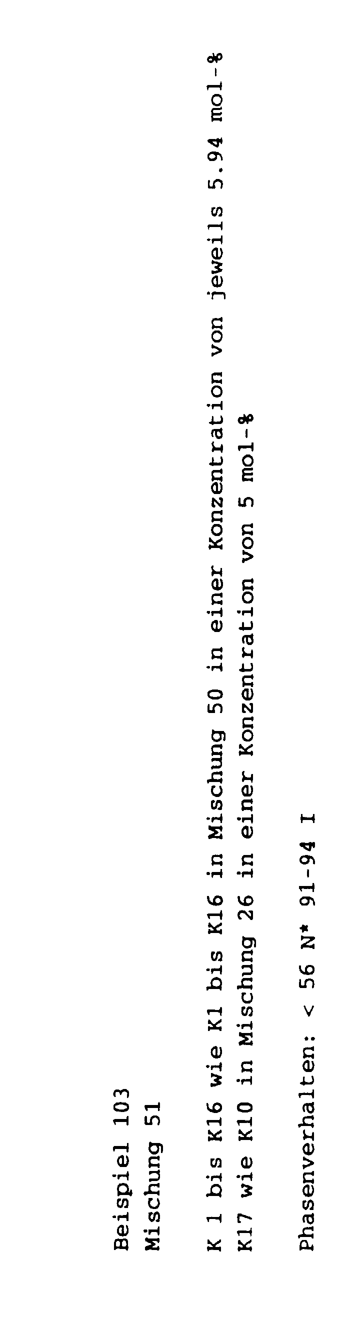

### Beispiel 104

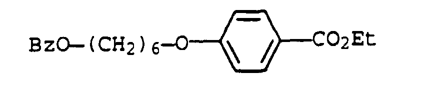

a) 4-[ω-Benzyloxyhexoxy]-benzoesäureethylester
   Zu einer Suspension von 4,8 g Natriumhydrid (60 %ige Dispersion in Öl) werden langsam bei 20°C 16,6 g 4-Hydroxybenzoesäureethylester gegeben bis die H₂-Entwicklung abklingt. Dann werden nach einstündigem Nachrühren bei Raumtemperatur 19,9 g 6-Benzyloxy-1-chlorhexan hinzugegeben. Nach 16-stündigem Erhitzen unter Rückfluß wird der ausgefallene Niederschlag abfiltriert und das Lösungsmittel entfernt. Durch Umkristallisation aus Toluol erhält man 15,2 g der obigen Verbindung. NMR, MS und IR stimmen mit der Struktur überein. 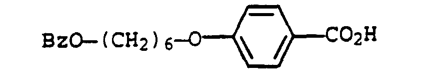
b) 4-[ω-Benzyloxyhexoxy]-benzoesäure
   Zu einer Lösung von 10 g des Produktes aus a) in 300 ml Ethanol gibt man 2 Äquivalente KOH und kocht 4 h unter Rückfluß. Das Reaktionsgemisch wird dann auf Wasser gegossen und mit konz. Salzsäure sauer gestellt. Der ausgefallene Niederschlag wird abfiltriert und über Nacht im Vakuum bei 50°C getrocknet. Man erhält 8,5 g der obigen Verbindung. NMR, MS und IR stimmen mit der Struktur überein. 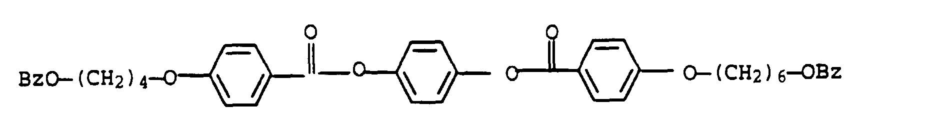
c) 1,4-[4'-(ω-Benzyloxyhexoxy]-benzoyloxy]-[4'-(ω-benzyloxybutoxy)-benzoyloxy]-benzol
   Entsprechend der Vorschrift aus Beispiel 1 werden 4 g 4-[ω-Benzyloxyhexoxy]-benzoesäure, 3,9 g 4-[4-Benzyloxybutoxy]-benzoesäure und 2 g Hydrochinon umgesetzt und ergeben 7,2 g der obigen Verbindung. NMR, IR und MS stimmen mit der Struktur überein. 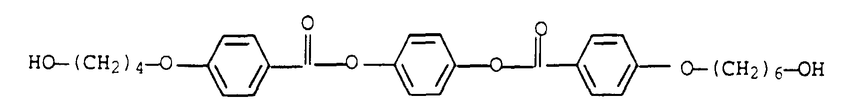
d) 1-[4'-(ω-Hydroxyhexoxyl-benzoyloxy]-4-[4"-(ω-hydroxybutoxy)benzoyloxy]-benzol
   12 g 1-[4'-(ω-Benzoyloxyhexoxy)benzoyloxy-4-]-[4"-(ω-Benzoyloxybutoxy)benzoyloxy]benzol werden in 200 ml Ethanol gelöst. mit 2 g Pd/C (10 %) versetzt und 6 h bei Raumtemperatur unter Wasserstoffatmosphäre bis zum Ende der Wasserstoffaufnahme gerührt. Dann wird vom Katalysator abfiltriert, das Lösungsmittel entfernt und der Rückstand aus Toluol umkristallisiert. Man erhält 8,5 g der obigen Verbindung. NMR, IR und MS stimmen mit der Struktur überein. 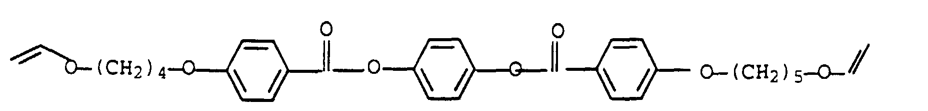
e) 1-[4'-(ω-Vinyloxyhexoxyl-benzoyloxy] -4-[4"-(ω-vinyloxybutyloxy)benzoyloxy]-benzol
   8,5 g des Produktes d) werden mit 1,5 g Hg(OAc)₂ in 500 ml Ethylvinylether gelöst und 24 h unter Rückfluß erhitzt. Dann werden 5 g K₂CO₃ hinzugegeben und der Überschuß an Ethylvinylether abdestilliert. Der Rückstand wird filtriert und das K₂CO₃ mit Petrolether gewaschen. Filtrat und Waschflüssigkeit werden eingeengt und nach Umkristallisation aus Petrolether/ Essigester (9:1) erhält man 8,1 g der obigen Verbindung. NMR, MS und IR stimmen mit der Struktur überein.

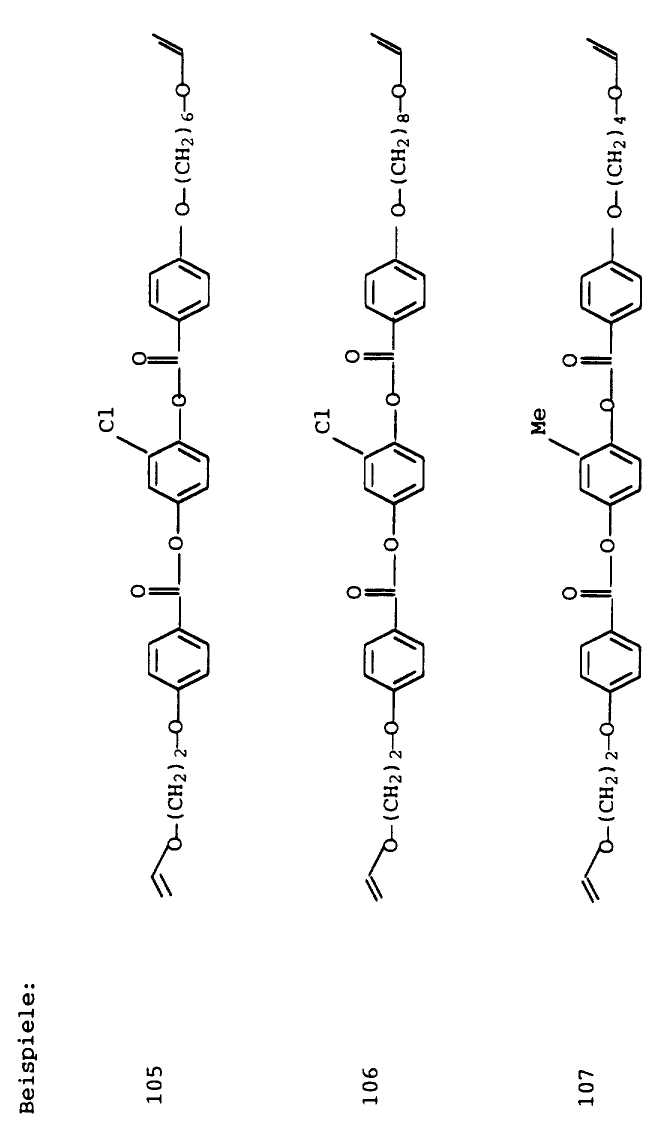

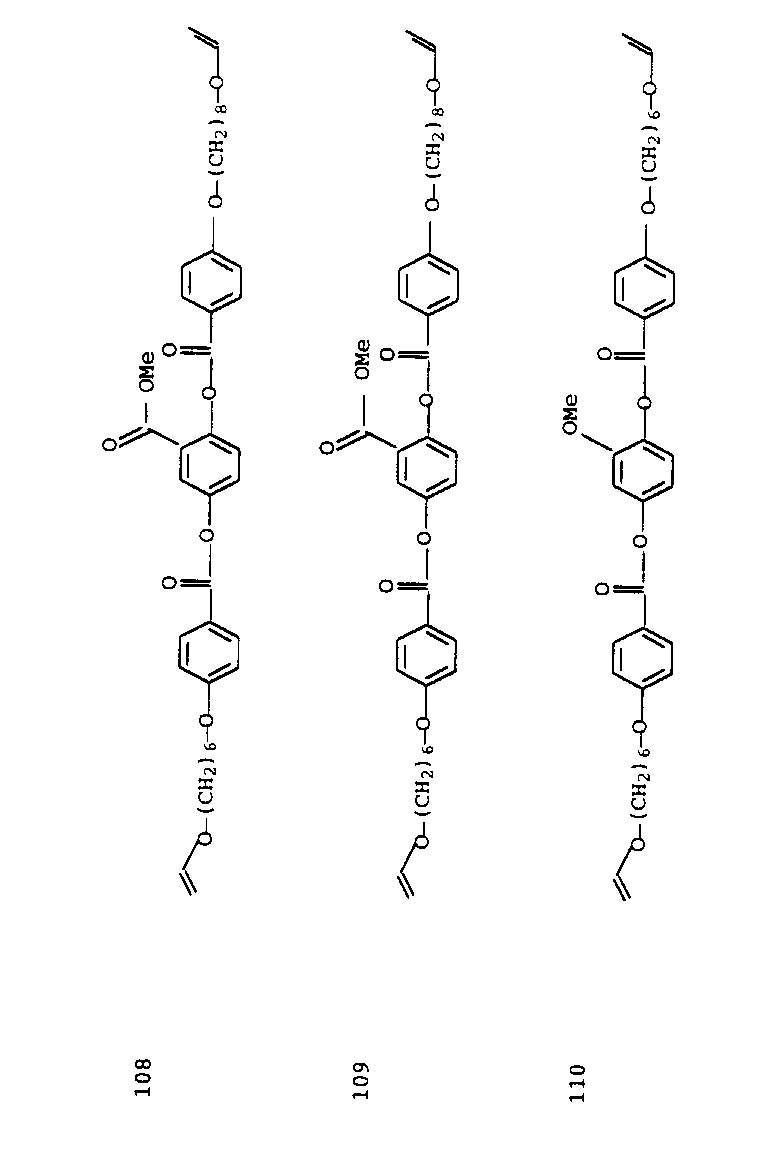

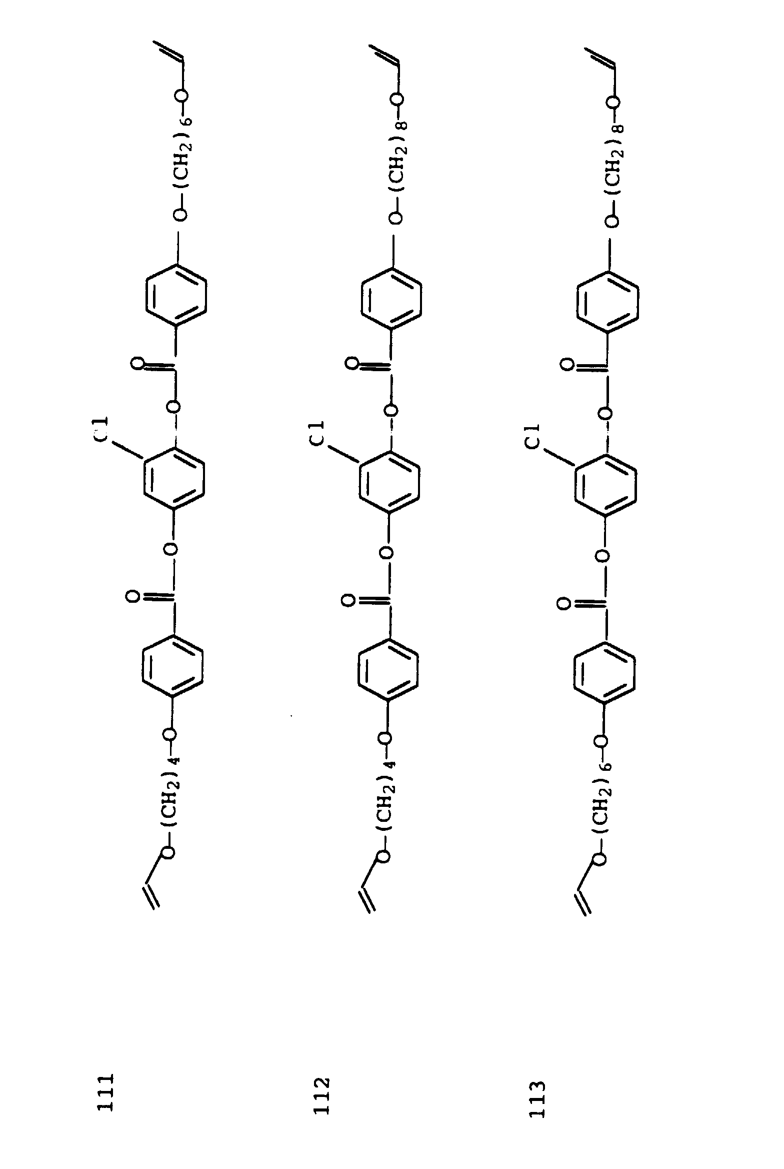

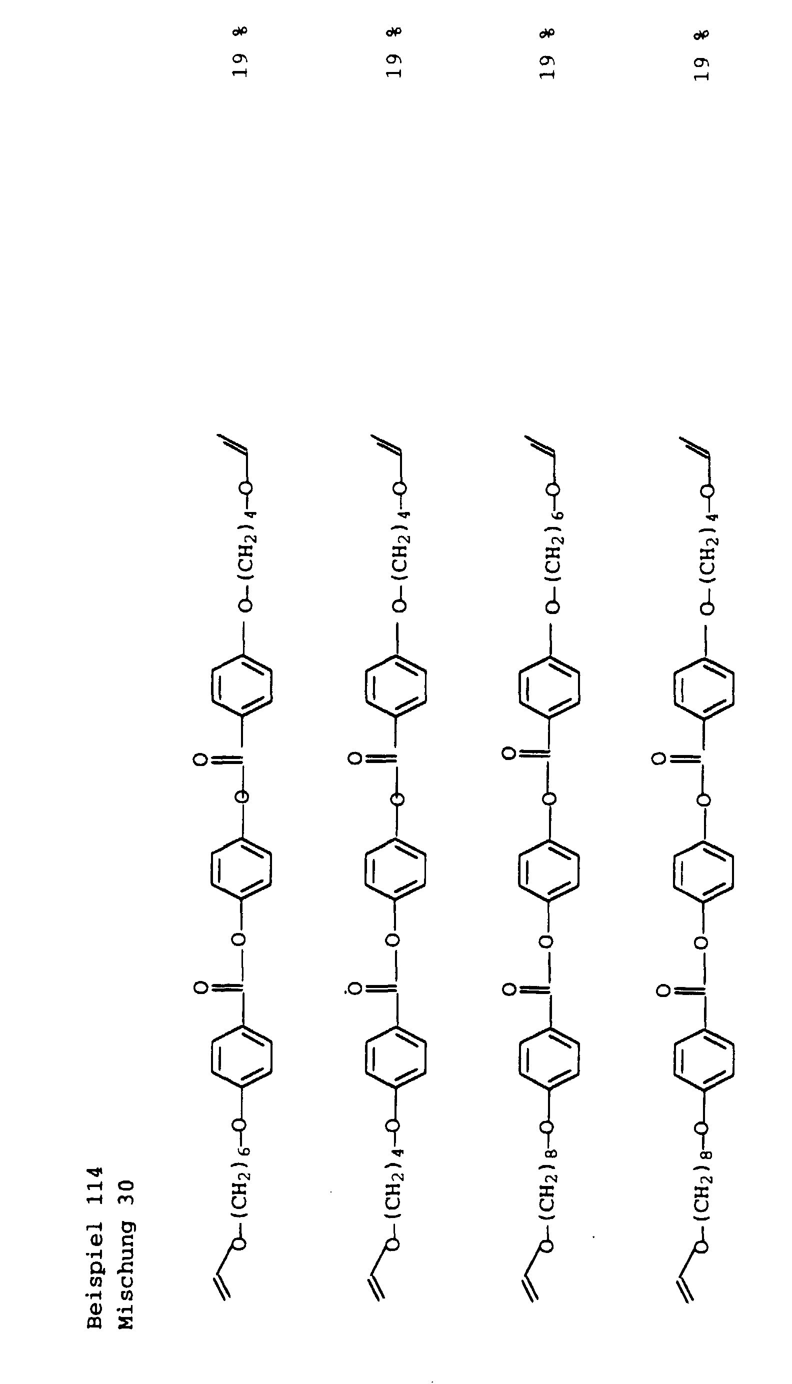

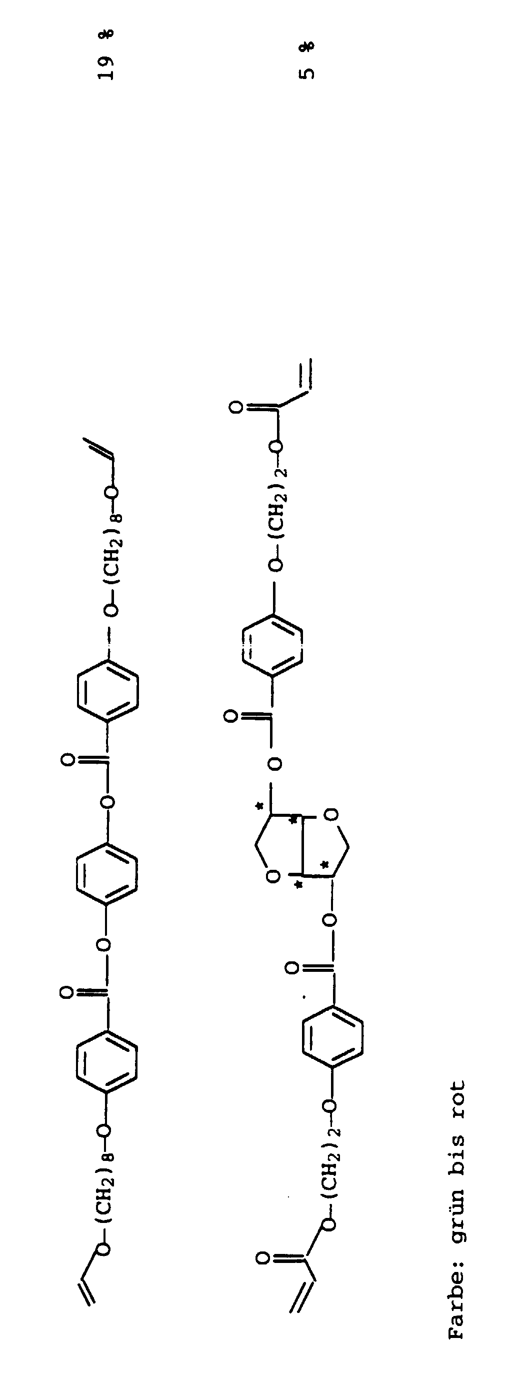

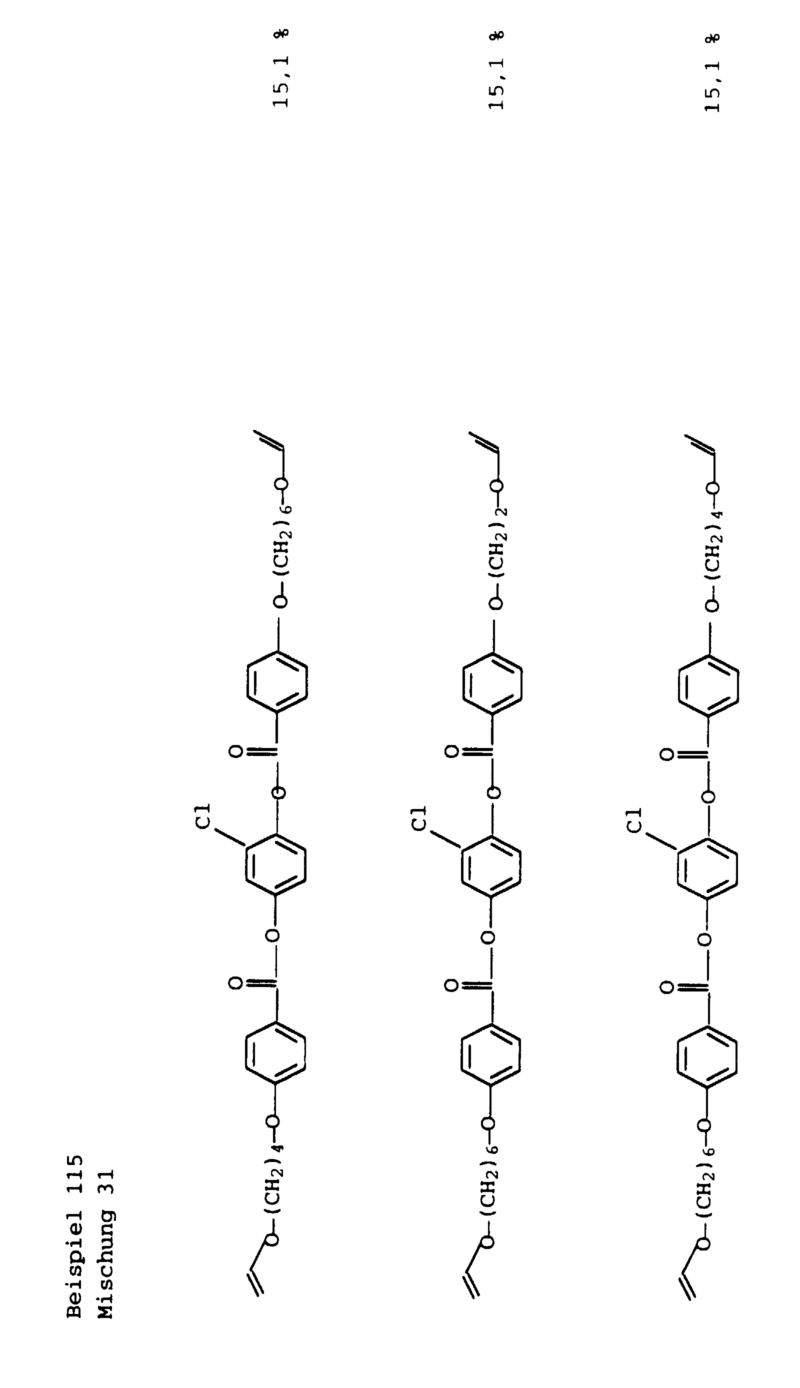

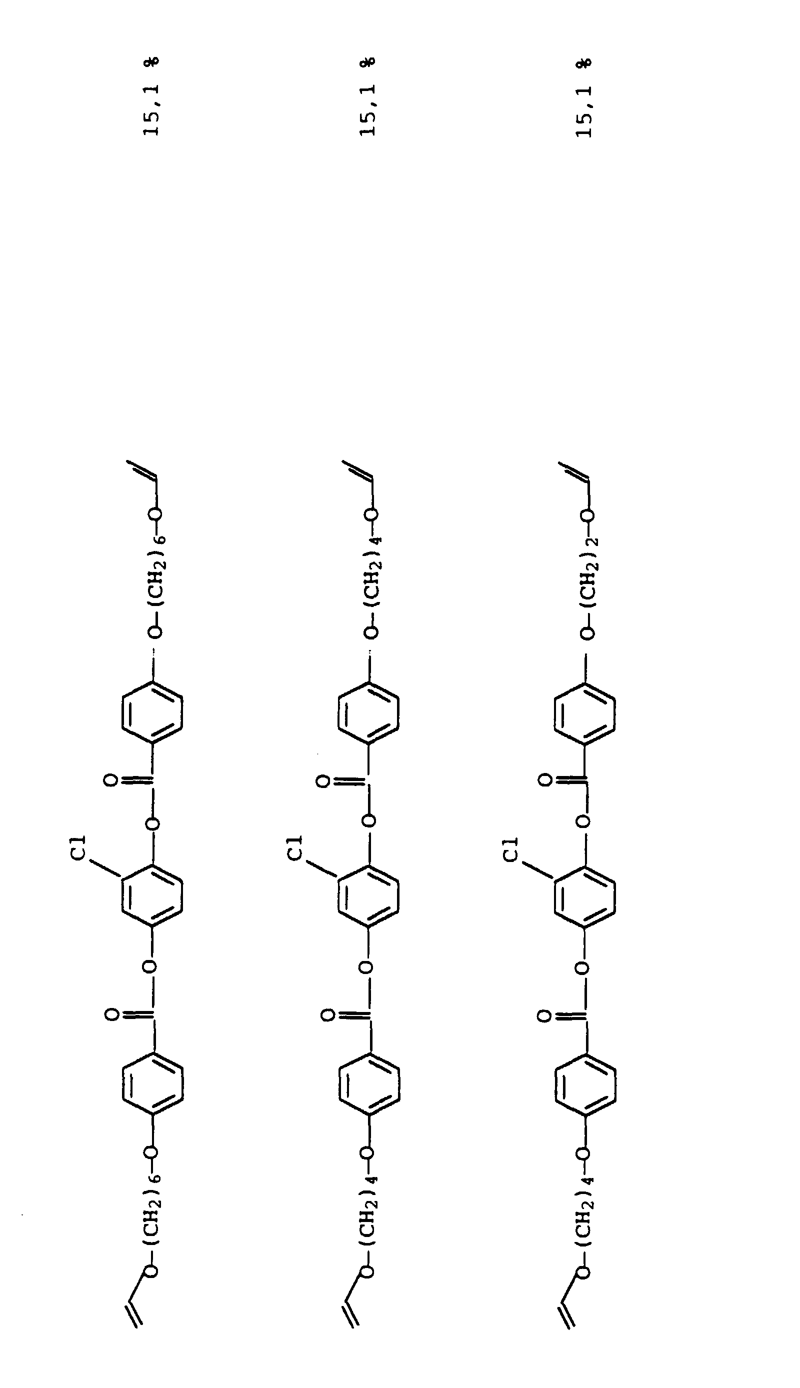

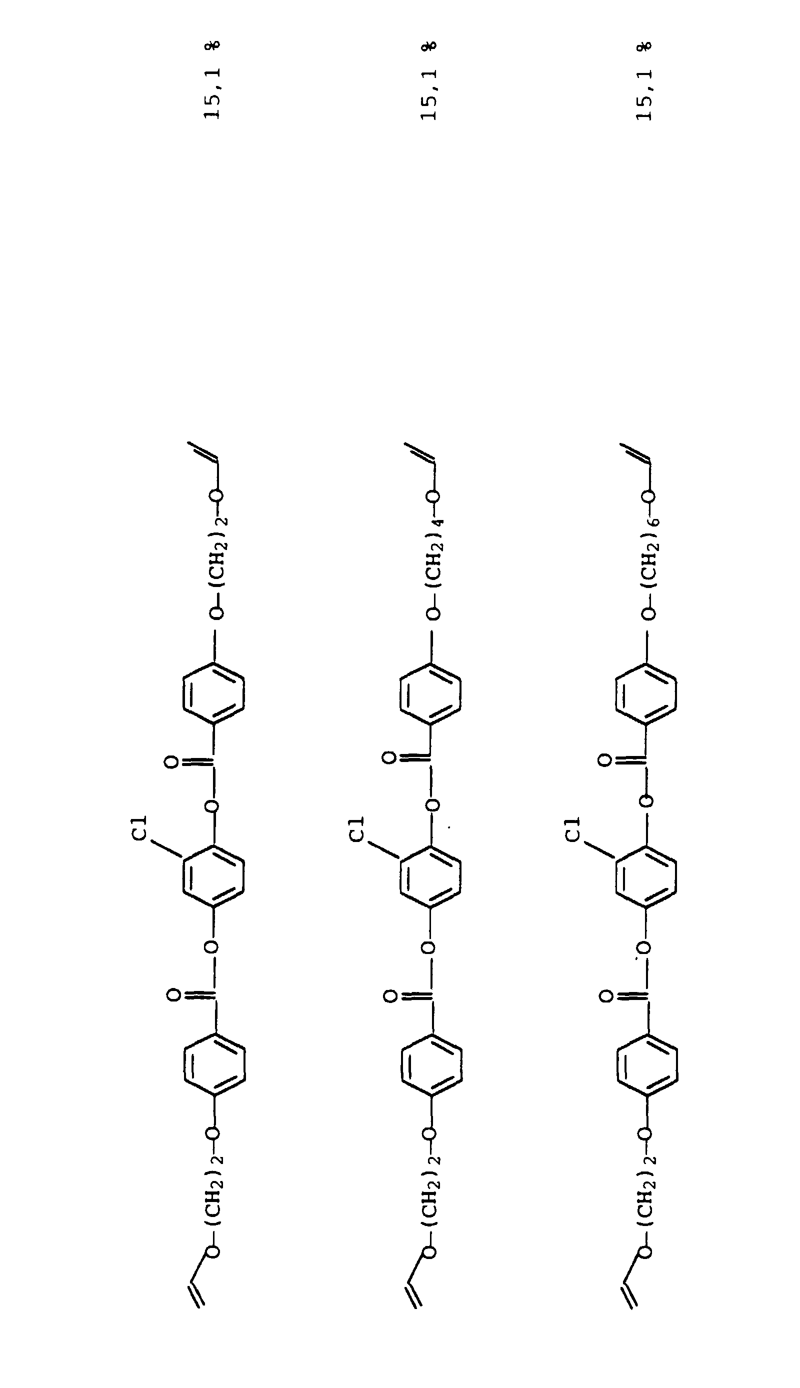

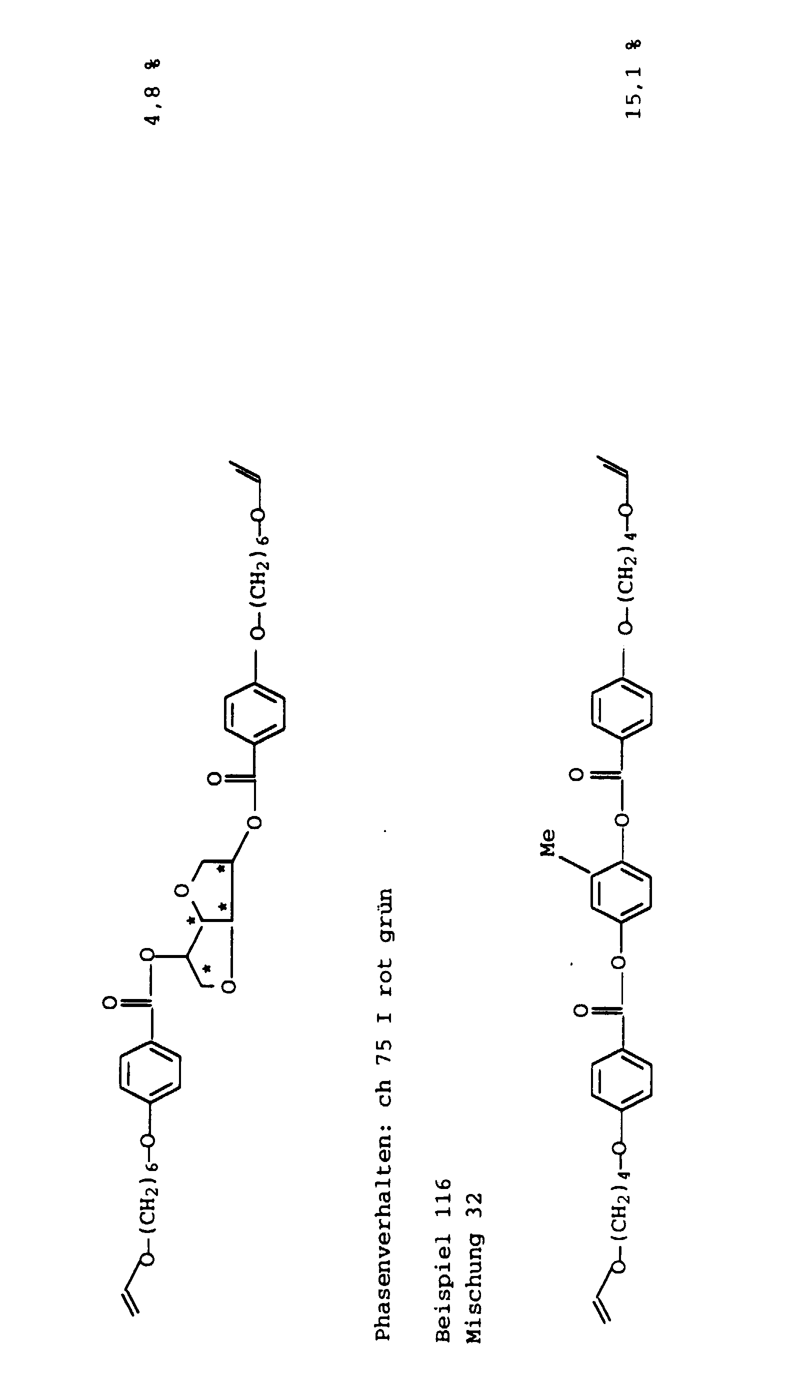

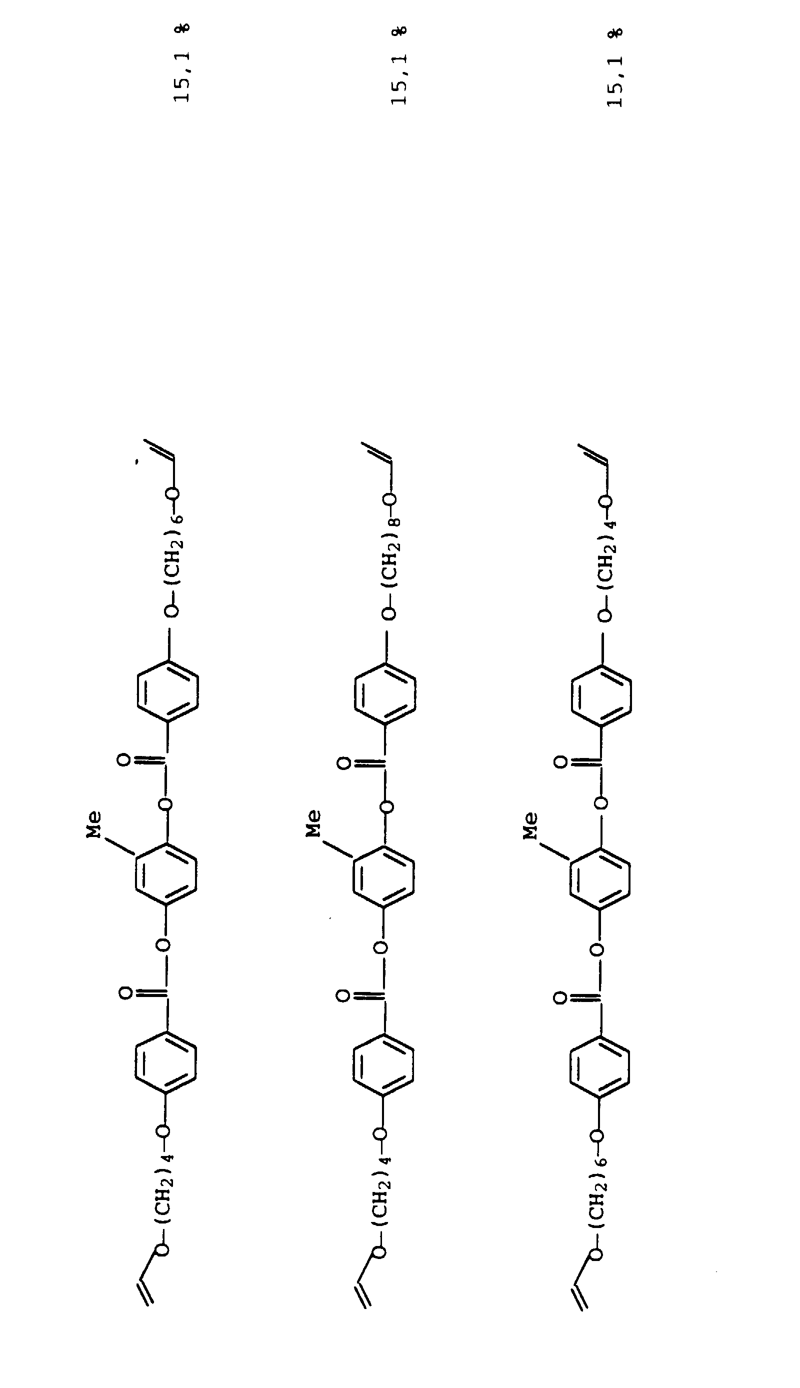

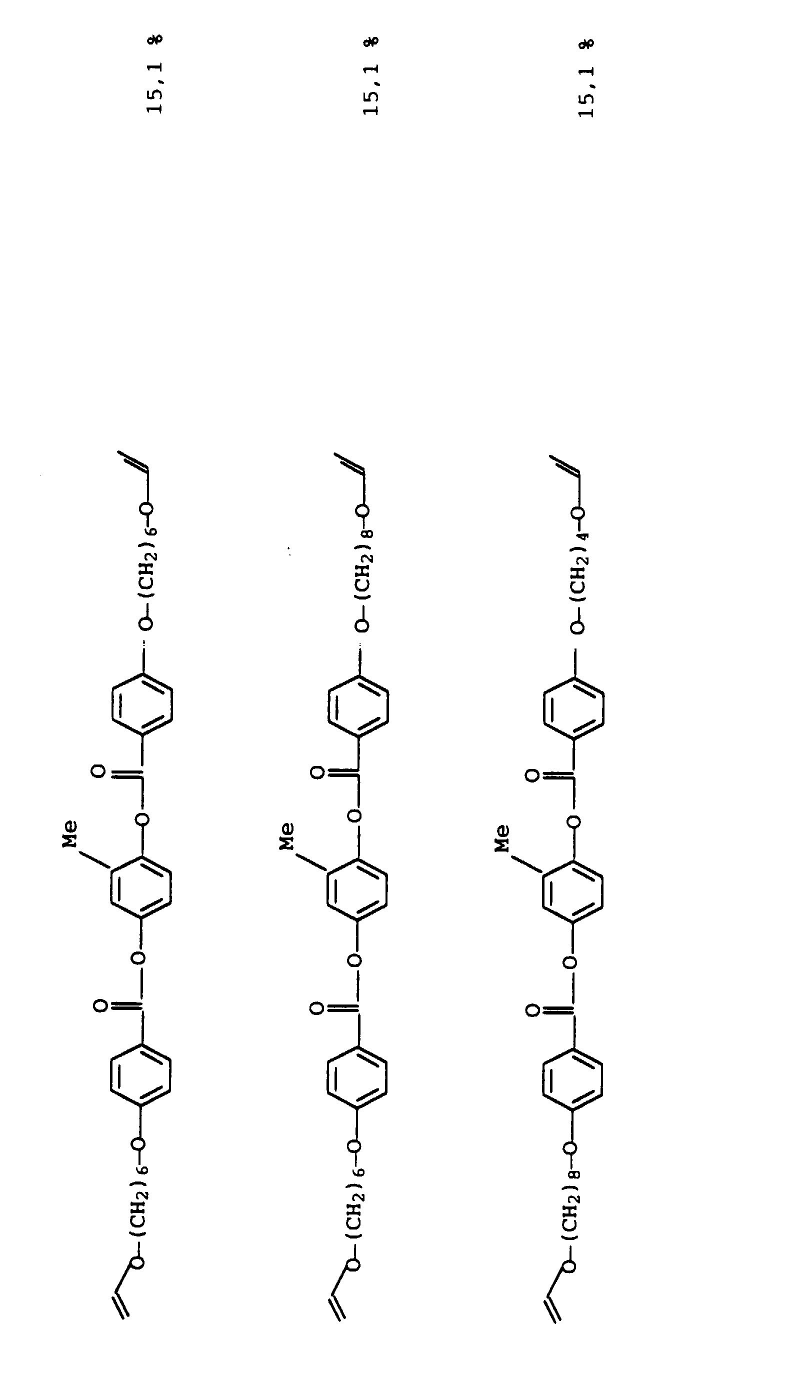

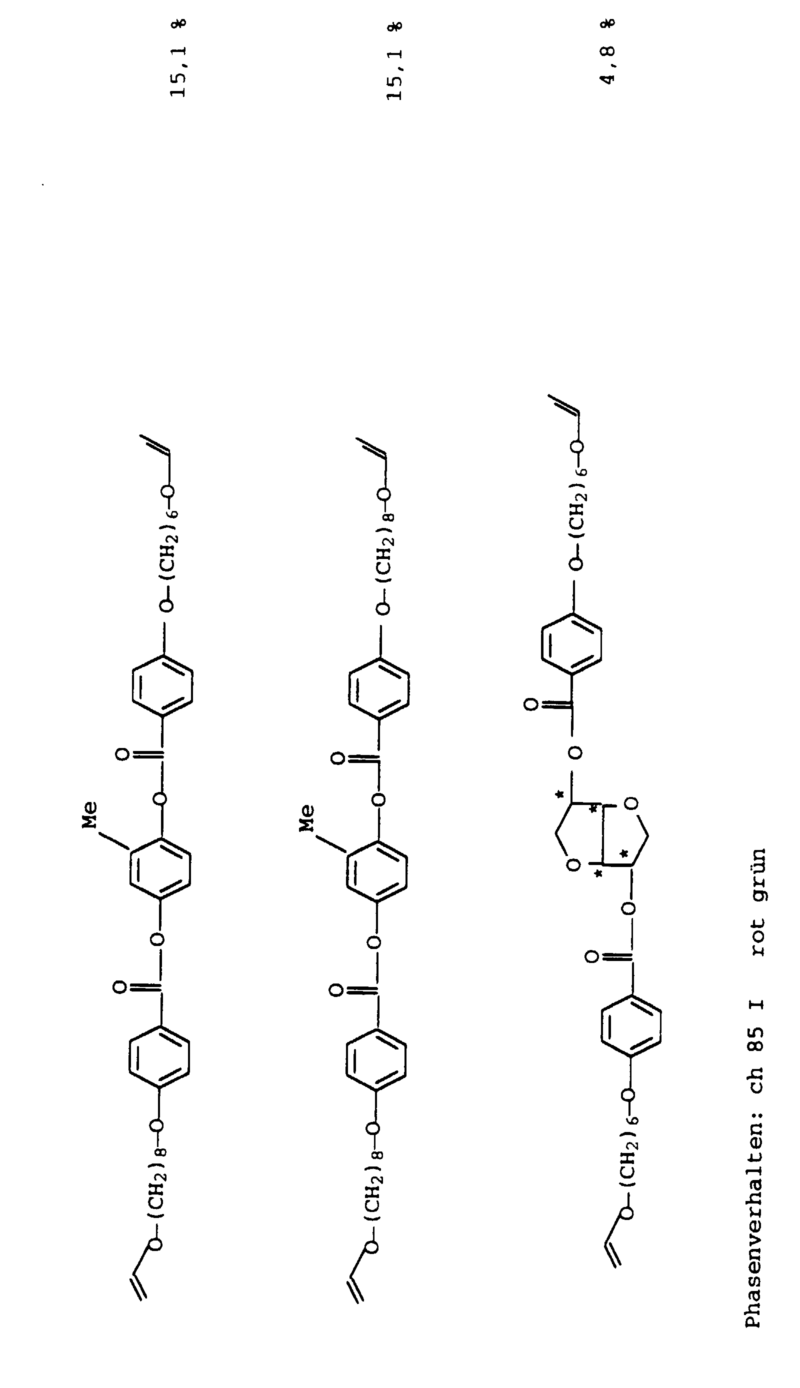

### Beispiel 117

Synthese von 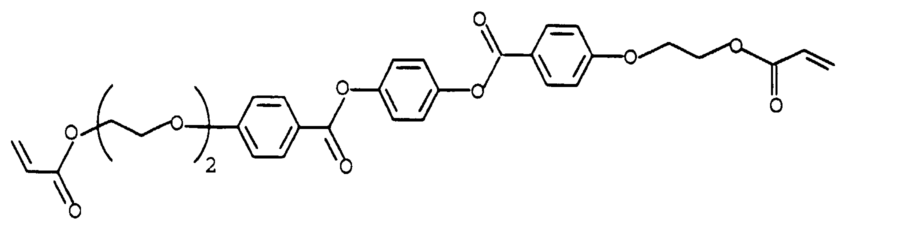
a. Veretherung von 4-Hydroxybenzoesäureethylester mit Diethylenglycol
   3,65 g (0,022 mol) 4-Hydroxybenzoesäureethylester werden zusammen mit 11,66 g (0,11 mol) Diethylenglycol in 35 ml abs. Tetrahydrofuran gelöst und mit 8,7 g (0,033 mol) Diethylazodicarboxylat versetzt. Die Reaktionsmischung wird 24 h bei RT gerührt. Anschließend wird das Gemisch am Rotationsverdampfer eingeengt, der Rückstand durch Säulenchromatographie (Kieselgel, Laufmittel: Toluol/Essigsäureethylester 5:1) gereinigt.
   Ausbeute: 4,36 g, 78 %.
b. Darstellung der 4-(Diethylenglycoloxy)-benzoesäure
   4,36 g (0,017 mol) 4-(2-Diethylenglycoloxyl-benzoesäureethylester werden in 80 ml Ethanol gelöst und mit 1,46 g (0,026 mol) KOH versetzt. Das Gemisch wird 3 h unter Rückfluß erhitzt; anschließend wird die Lösung mit konz. Salzsäure sauer gestellt, Essigsäureethylester zugegeben und der ausgefällte Niederschlag abgesaugt. Der Rückstand wird verworfen, das Filtrat ergibt nach Einengen das gewünschte Produkt.
   Ausbeute: 4,7 g, 98 %.
   Die weiteren Schritte zur Herstellung der Verbindung des Beispiels 117 entsprechen denen von Beispiel 1.

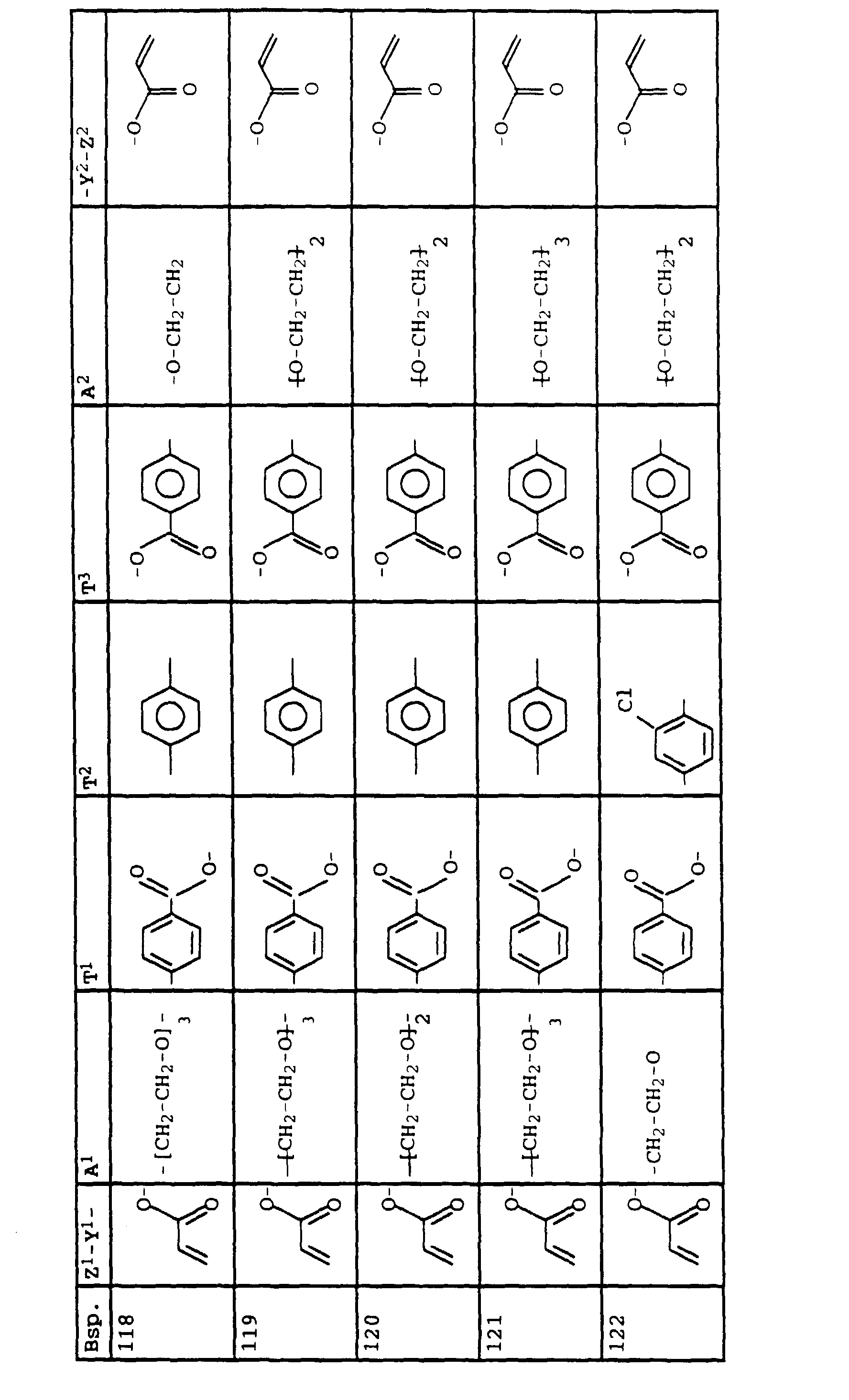

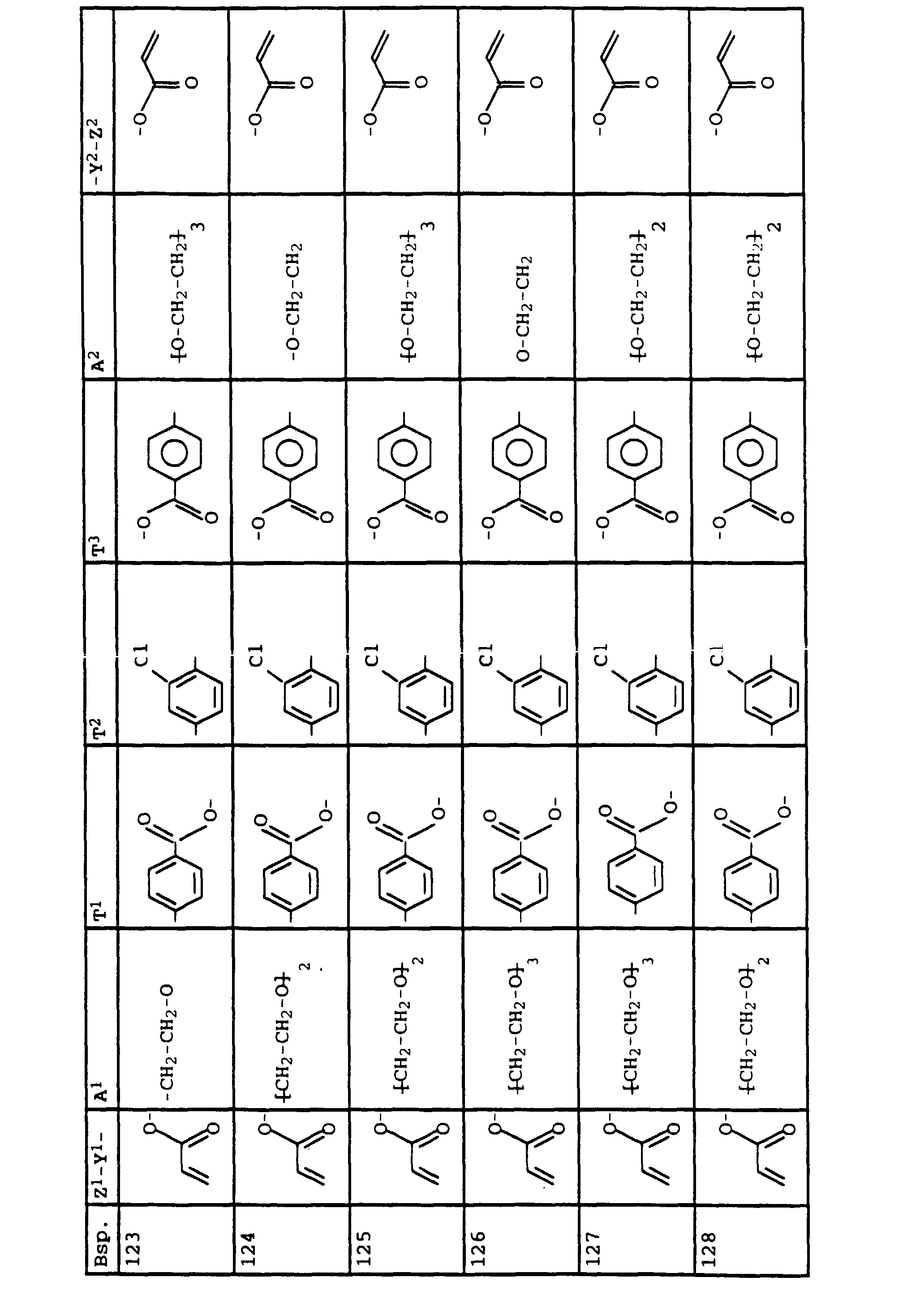

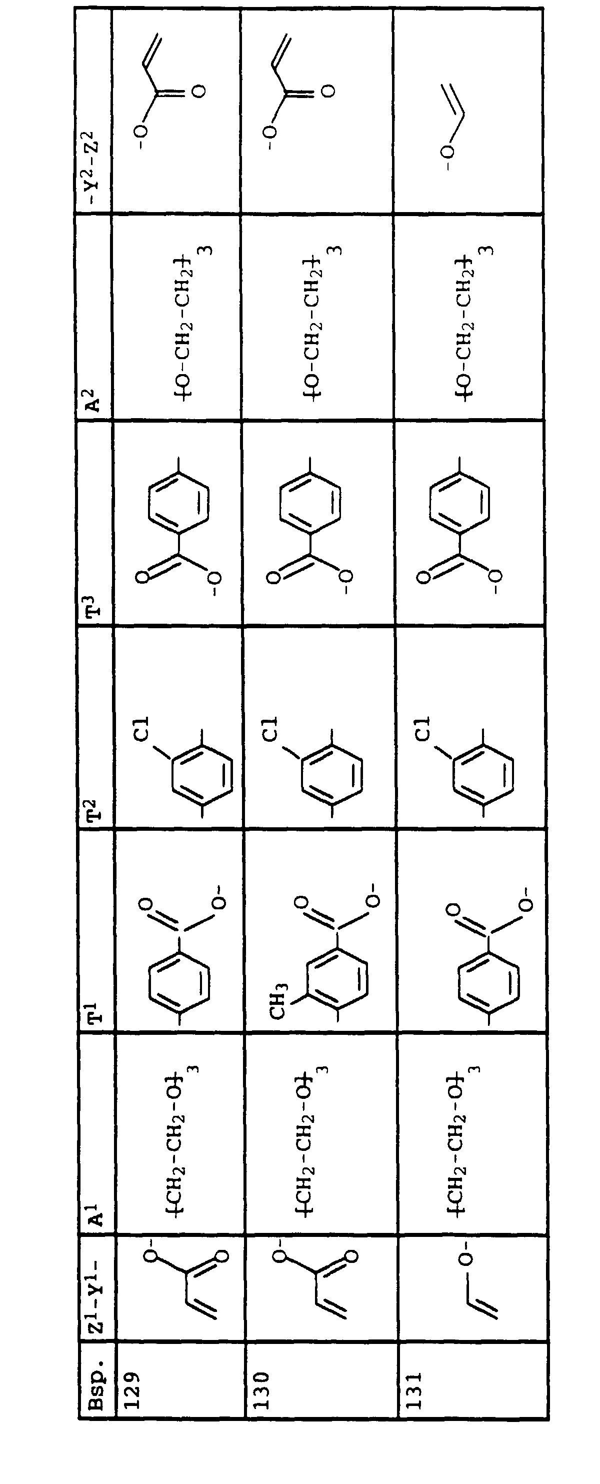

### Beispiel 132

### Mischung 33

K1; Konzentration 25 mol% 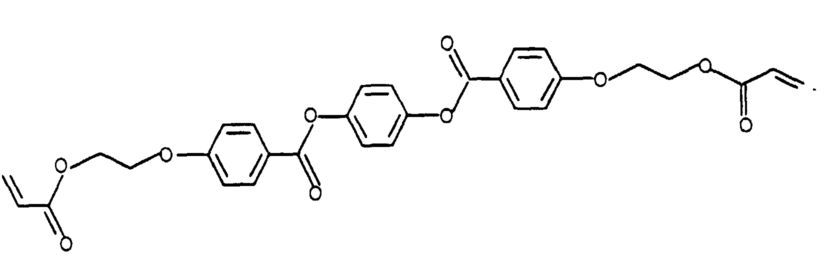
K2; Konzentration 45 mol% 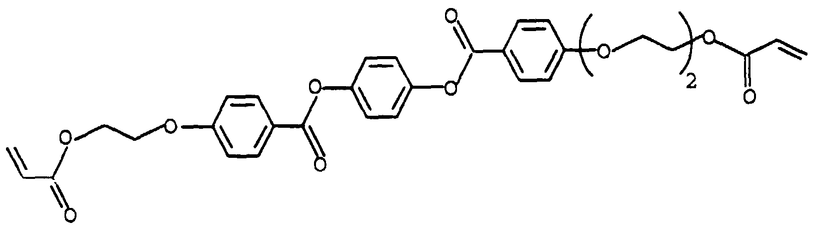
K3; Konzentration 5 mol% 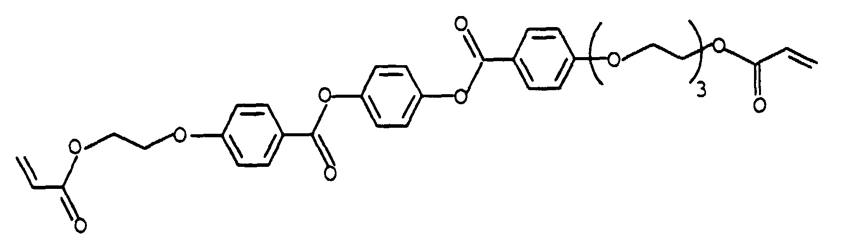
K4; Konzentration 20,25 mol% 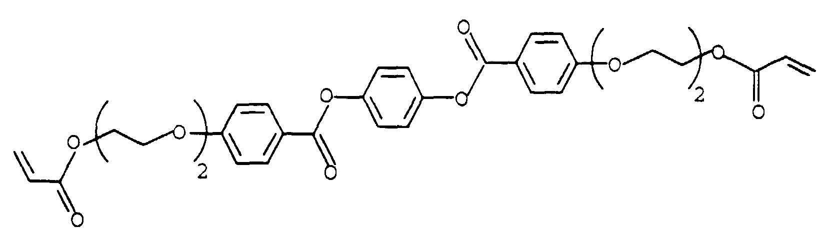
K5; Konzentration 4,5 mol% 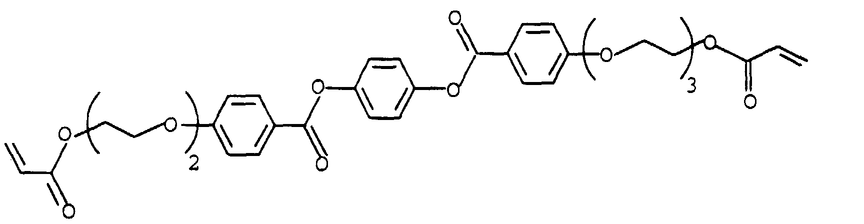
K6; Konzentration 0,25 mol% 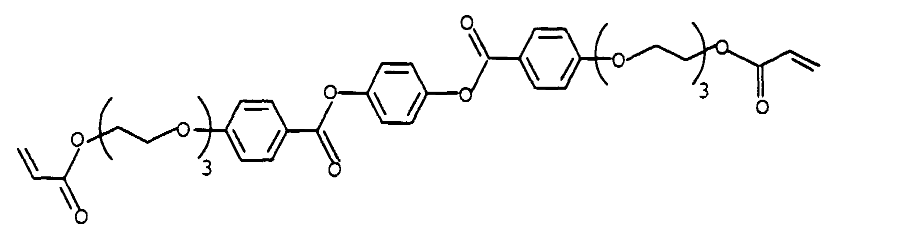
Phasenverhalten: S 45 N 83 I

### Beispiel 133

### Mischung 34

K1 wie K1 Beispiel 132; Konzentration 23,75 mol%
K2 wie K2 Beispiel 132; Konzentration 42,75 mol%
K3 wie K3 Beispiel 132; Konzentration 4,75 mol%
K4 wie K4 Beispiel 132; Konzentration 19,2375 mol%
K5 wie K5 Beispiel 132; Konzentration 4,275 mol%
K6 wie K6 Beispiel 132; Konzentration 0,002375 mol%
K7; Konzentration 5 mol% 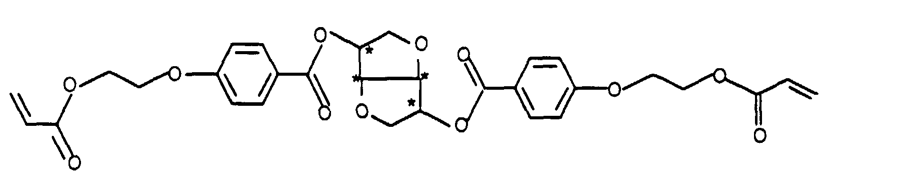
Phasenverhalten: S 41 Ch 81 I
Farbe: rot/grün

### Beispiel 134

### Mischung 35

K1 wie K1 Beispiel 132; Konzentration 23,25 mol%
K2 wie K2 Beispiel 132; Konzentration 41,85 mol%
K3 wie K3 Beispiel 132; Konzentration 4,65 mol%
K4 wie K4 Beispiel 132; Konzentration 18,8325 mol%
K5 wie K5 Beispiel 132; Konzentration 4,185 mol%
K6 wie K6 Beispiel 132; Konzentration 0,002325 mol%
K7; wie K7 Beispiel 133; Konzentration 7 mol%
Phasenverhalten: S 40 Ch 80 I
Farbe: grün/blau

### Beispiel 135

### Mischung 36

K1; Konzentration 25 mol% 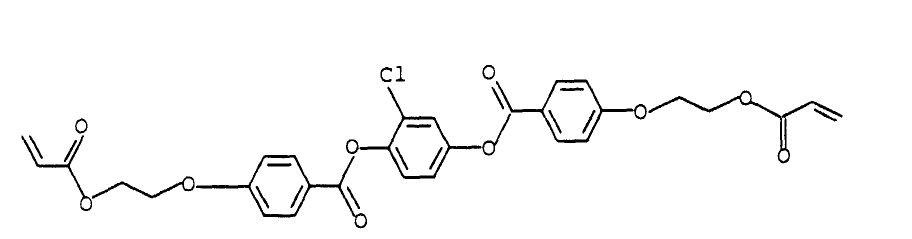
K2; Konzentration 22,5 mol% 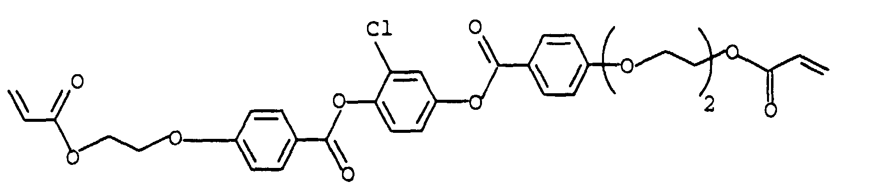
K3; Konzentration 2,5 mol% 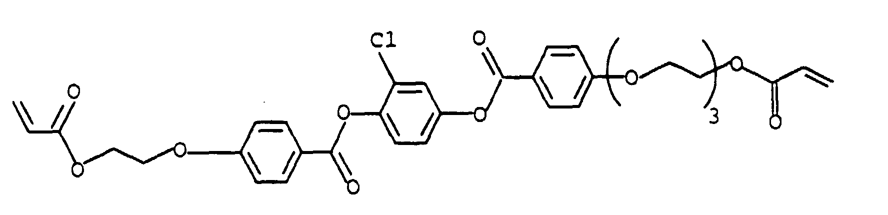
K4; Konzentration 22,5 mol% 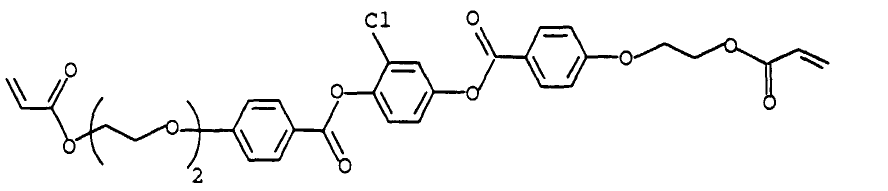
K5; Konzentration 20,25 mol% 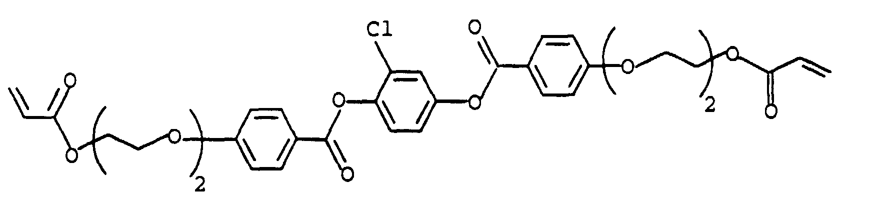
K6; Konzentration 2,25 mol% 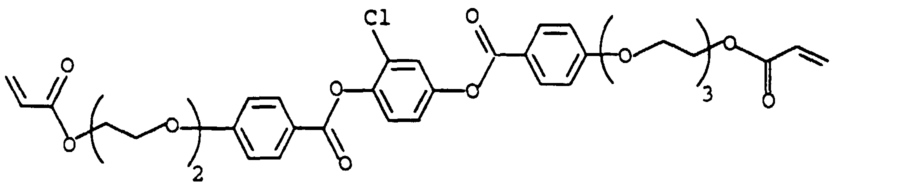
K7; Konzentration 2,5 mol% 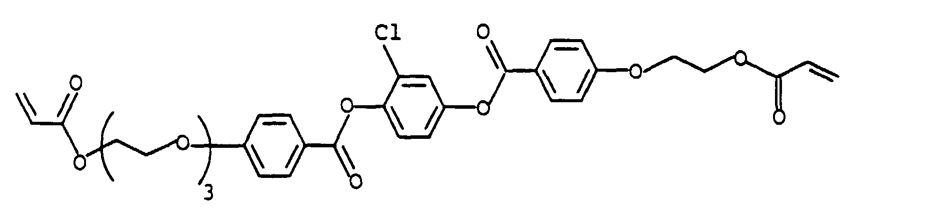
K8; Konzentration 2,25 mol% 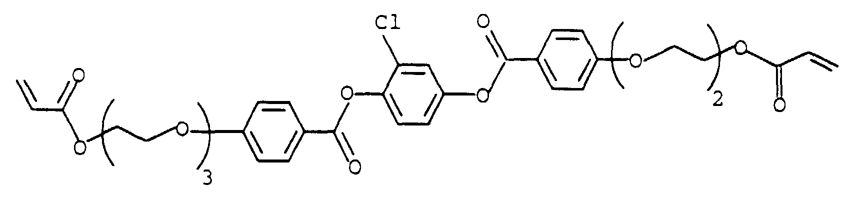
K9; Konzentration 0,25 mol% 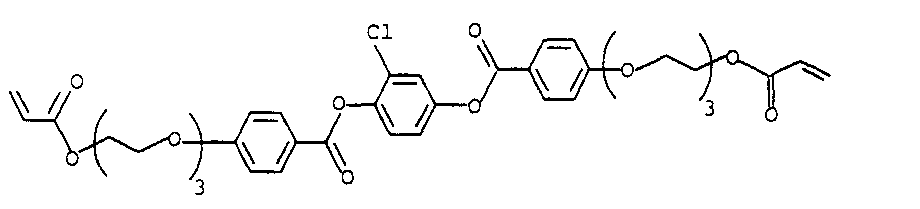
Phasenverhalten: S 45 N 78 I

### Beispiel 136

### Mischung 37

K1 wie K1 Beispiel 135; Konzentration 23,75 mol%
K2 wie K2 Beispiel 135; Konzentration 21,375 mol%
K3 wie K3 Beispiel 135; Konzentration 2,375 mol%
K4 wie K4 Beispiel 135; Konzentration 21,375 mol%
K5 wie K5 Beispiel 135; Konzentration 19,2375 mol%
K6 wie K6 Beispiel 135; Konzentration 2,1375 mol%
K7 wie K7 Beispiel 135; Konzentration 2,375 mol%
K8 wie K8 Beispiel 135; Konzentration 2,1375 mol%
K9 wie K9 Beispiel 135; Konzentration 0,2375 mol%
K10 wie K7 Beispiel 133; Konzentration 5 mol%
Phasenverhalten: S 43 Ch 70 I
Farbe: rot

### Beispiel 137

### Mischung 38

K1 wie K1 Beispiel 135; Konzentration 23,25 mol%
K2 wie K2 Beispiel 135; Konzentration 230,925 mol%
K3 wie K3 Beispiel 135; Konzentration 2,325 mol%
K4 wie K4 Beispiel 135; Konzentration 20,925 mol%
K5 wie K5 Beispiel 135; Konzentration 18,8325 mol%
K6 wie K6 Beispiel 135; Konzentration 2,0925 mol%
K7 wie K7 Beispiel 135; Konzentration 2,325 mol%
K8 wie K8 Beispiel 135; Konzentration 2,0925 mol%
K9 wie K9 Beispiel 135; Konzentration 0,2325 mol%
K10 wie K7 Beispiel 133; Konzentration 7 mol%
Phasenverhalten: S 41 Ch 70 I
Farbe: grün

## Patentansprüche

1. Mischungen flüssigkristalliner Verbindungen, die mindestens zwei verschiedene Substanzen der allgemeinen Formel I 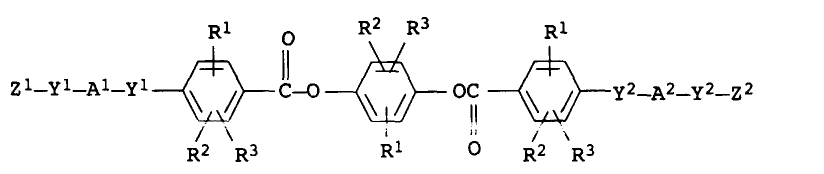 enthalten, in der die Reste
Z¹, Z² unabhängig voneinander eine polymerisierbare Gruppe,
Y¹, Y² jeweils unabhängig voneinander eine direkte Bindung, -O-, -COO-, -OCO- oder -S-,
A¹, A² unabhängig voneinander einen Spacer und
R¹, R² und R³ unabhängig voneinander Wasserstoff, C₁- bis C₂₀-Alkyl, C₁- bis C₂₀-Alkoxy, C₁- bis C₂₀-Alkoxycarbonyl, C₁-bis C₂₀-Monoalkylaminocarbonyl, Formyl, C₁- bis C₂₀-Alkylcarbonyl, Fluor, Chlor, Brom, cyan, C₁- bis C₂₀-Alkylcarbonyloxy, C₁-bis C₂₀-Alkylcarbonylamino, Hydroxy oder Nitro bedeuten, mit der Maßgabe, daß bei mindestens einer der Substanzen I die Gruppen -A¹-Y¹- und -Y²-A²- verschiedene O-alkylen-Reste sind.

2. Flüssigkristalline Mischungen gemäß Anspruch 1, bei denen Z¹ und/oder Z² ein Rest der Formel CH₂=CH-, CH₂=CCl-, CH₂=C(CH₃)-oder Vinylphenylyl sind.

3. Flüssigkristalline Mischungen gemäß Anspruch 1, bei denen die Reste Y¹ und Y² unabhängig voneinander eine direkte Bindung, -O-, -COO- oder -OCO- sind.

4. Flüssigkristalline Mischungen gemäß Anspruch 1, bei denen die 40 Reste A¹ und A² unabhängig voneinander gegebenenfalls durch Ethersauerstoff oder Estergruppen unterbrochenes C₂- bis C₂₀-Alkylen sind, wobei die Sauerstoffatome oder Estergruppen in der Kette dritte C-Atome ersetzen können.

5. Flüssigkristalline Mischungen gemäß Anspruch 1, bei denen R¹, R² und R³ unabhängig voneinander Wasserstoff, C₁- bis C₁₅-Alkyl, C₁- bis C₁₅-Alkoxy, C₁- bis C₁₅-Alkoxycarbonyl, C₁-bis C₁₅-Monoalkylaminocarbonyl, Formyl, C₁- bis C₁₅-Alkylcarbonyl, Fluor, Chlor, Brom, Cyan, C₁- bis C₁₅-Alkylcarbonyloxy, C₁- bis C₁₅-Alkylcarbonylamino, Hydroxy oder Nitro bedeuten.

6. Flüssigkristalline Mischungen gemäß Anspruch 5, bei denen R¹, R² und R³ unabhängig voneinander Wasserstoff, Methyl, Ethyl, C₈- bis C₁₅-Alkyl, Methoxy, Ethoxy, C₈- bis C₁₅-Alkoxy, Methoxycarbonyl, Ethoxycarbonyl, C₈- bis C₁₅-Alkoxycarbonyl, Formyl, Acetyl, C₈- bis C₁₅-Alkylcarbonyl, Fluor, Chlor, Brom, Cyan, Acetoxy, Hydroxy oder Nitro sind.

7. Flüssigkristalline Mischungen gemäß Anspruch 6, bei denen R¹, R² und R³ unabhängig voneinander Wasserstoff, Methyl, Ethyl, Methoxy, Ethoxy, Methoxycarbonyl, Formyl, Acetyl, Fluor, Chlor, Brom, Cyan, Acetoxy, Hydroxy oder Nitro sind.

8. Flüssigkristalline Mischungen gemäß Anspruch 1, dadurch gekennzeichnet, daß sie bis zu 50 %, vorzugsweise 1 bis 30 %, polymerisierbare chirale Verbindungen enthalten, wobei diese chiralen Verbindungen flüssigkristallin oder nicht flüssigkristallin sein können.

9. Verbindungen der allgemeinen Formel I gemäß Anspruch 1, bei denen die Reste R¹ bis R³, Z¹, Z², Y¹, Y², A¹ und A² die angegebene Bedeutung haben und die Gruppen -A¹-Y¹- und -Y²-A² nicht gleichzeitig gleiche -O-alkylen-Reste sind.

10. Verbindungen gemäß Anspruch 9, bei denen die Reste A¹ und A² verschieden sind.

11. Verbindungen gemäß Anspruch 9, bei denen die Reste Z¹ und Z² verschieden sind.

12. Verbindungen gemäß Anspruch 9, bei denen mindestens 2 der Reste Y¹ und/oder Y² verschieden sind.

13. Verbindungen gemäß der Formel in Anspruch 1, bei denen der Molekülteil 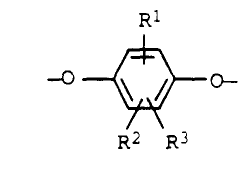 der Formel 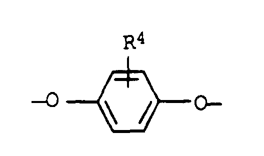 entspricht, wobei
R⁴ C₂- bis C₁₅-Alkyl, C₂- bis C₁₅-Alkoxy, C₂- bis C₁₅-Alkanoyl, C₂- C₁₅-Alkanoyloxy oder C₂- bis C₁₅-Alkoxycarbonyl ist.

14. Verbindungen gemäß Anspruch 13, bei denen R⁴ C₈- bis C₁₅-Alkyl, C₈- bis C₁₅-Alkoxy, C₈- bis C₁₅-Alkanoyl, C₈- bis C₁₅-Alkanoyloxy oder C₈- bis C₁₅-Alkoxycarbonyl ist.

15. Verwendung der Mischungen gemäß Anspruch 1 als Orientierungsschichten für flüssigkristalline Materialien, als photovernetzbare Kleber, als Monomere zur Herstellung flüssigkristalliner Polymere, als Basismaterial zur Herstellung von chiral dotierbaren polymerisierbaren Flüssigkristallsystemen, als polymerisierbare Matrixmonomere für polymer dispergierte Displays, als Basismaterial für polymerisierbare, flüssigkristalline Materialien für optische Bauelemente.

16. Verwendung der Mischungen gemäß Anspruch 1 dotiert mit polymerisierbaren chiralen Verbindungen als Basismaterial für Farbeffekt- und Piezomaterialien.

## Claims

1. A mixture of liquid-crystalline compounds comprising at least two different substances of the formula I 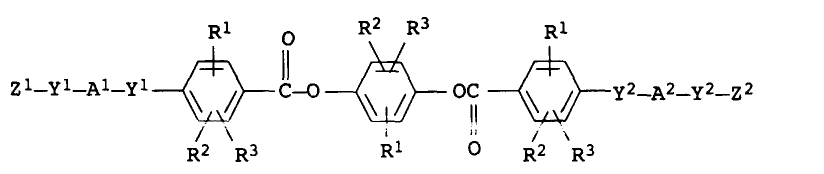 where
Z¹ and Z², independently of one another, are polymerizable groups,
Y¹ and Y², independently of one another, are each a direct bond, -O-, -COO-, -OCO- or -S-,
A¹ and A², independently of one another, are spacers, and
R¹, R² and R³, independently of one another, are hydrogen, C₁- to C₂₀-alkyl, C₁- to C₂₀-alkoxy, C₁- to C₂₀-alkoxycarbonyl, C₁- to C₂₀-monoalkylaminocarbonyl, formyl, C₁- to C₂₀-alkylcarbonyl, fluorine, chlorine, bromine, cyano, C₁- to C₂₀-alkylcarbonyloxy, C₁- to C₂₀-alkylcarbonylamino, hydroxyl or nitro, with the proviso that -A¹-Y¹- and -Y²-A²- in at least one of the substances I are different O-alkylene radicals.

2. A liquid-crystalline mixture as claimed in claim 1, where Z¹ and/or Z² is a radical of the formula CH₂=CH-, CH₂=CCl-, CH₂=C(CH₃)- or vinylphenyl.

3. A liquid-crystalline mixture as claimed in claim 1, where Y¹ and Y², independently of one another, are a direct bond, -O-, -COO- or -OCO-.

4. A liquid-crystalline mixture as claimed in claim 1, where A¹ and A², independently of one another, are C₂- to C₂₀-alkylene, which may be interrupted by ether oxygen or ester groups, where the oxygen atoms or ester groups can replace third carbon atoms in the chain.

5. A liquid-crystalline mixture as claimed in claim 1, where R¹, R² and R³, independently of one another, are hydrogen, C₁- to C₁₅-alkyl, C₁- to C₁₅-alkoxy, C₁- to C₁₅-alkoxycarbonyl, C₁- to C₁₅-monoalkylaminocarbonyl, formyl, C₁- to C₁₅-alkylcarbonyl, fluorine, chlorine, bromine, cyano, C₁- to C₁₅-alkylcarbonyloxy, C₁- to C₁₅-alkylcarbonylamino, hydroxyl or nitro.

6. A liquid-crystalline mixture as claimed in claim 5, where R¹, R² and R³, independently of one another, are hydrogen, methyl, ethyl, C₈- to C₁₅-alkyl, methoxy, ethoxy, C₈- to C₁₅-alkoxy, methoxycarbonyl, ethoxycarbonyl, C₈- to C₁₅-alkoxycarbonyl, formyl, acetyl, C₈- to C₁₅-alkylcarbonyl, fluorine, chlorine, bromine, cyano, acetoxy, hydroxyl or nitro.

7. A liquid-crystalline mixture as claimed in claim 6, where R¹, R² and R³, independently of one another, are hydrogen, methyl, ethyl, methoxy, ethoxy, methoxycarbonyl, formyl, acetyl, fluorine, chlorine, bromine, cyano, acetoxy, hydroxyl or nitro.

8. A liquid-crystalline mixture as claimed in claim 1, which comprises up to 50 %, preferably from 1 to 30 %, of polymerizable chiral compounds, which can be liquid-crystalline or non-liquid-crystalline.

9. A compound of the formula I as claimed in claim 1, where R¹ to R³, Z¹, Z², Y¹, Y², A¹ and A² are as defined above, and -A¹-Y¹- and -Y²-A²- are not simultaneously identical -O-dialkylene radicals.

10. A compound as claimed in claim 9, where A¹ and A² are different from one another.

11. A compound as claimed in claim 9, where Z¹ and Z² are different from one another.

12. A compound as claimed in claim 9, where at least 2 of the radicals Y¹ and/or Y² are different from one another.

13. A compound of the formula in claim 1, where the moiety 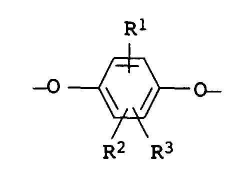 conforms to the formula 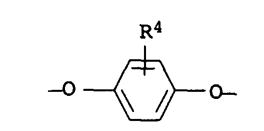 where
R⁴ is C₂- to C₁₅-alkyl, C₂- to C₁₅-alkoxy, C₂- to C₁₅-alkanoyl, C₂- to C₁₅-alkanoyloxy or C₂- to C₁₅-alkoxycarbonyl.

14. A compound as claimed in claim 13, where R⁴ is C₈- to C₁₅-alkyl, C₈- to C₁₅-alkoxy, C₈- to C₁₅-alkanoyl, C₈- to C₁₅-alkanoyloxy or C₈- to C₁₅-alkoxycarbonyl.

15. The use of a mixture as claimed in claim 1 as alignment layers for liquid-crystalline materials, as photocrosslinkable adhesives, as monomers for the preparation of liquid-crystalline polymers, as base material for the production of polymerizable liquid-crystal systems which can be doped by chiral compounds, as polymerizable matrix monomers for polymer-dispersed displays, and as base material for polymerizable liquid-crystalline materials for optical components.

16. The use of a mixture as claimed in claim 1 doped with polymerizable chiral compounds as base material for color-effect materials and piezomaterials.

## Revendications

1. Mélanges de composés cristaux liquides contenant au moins deux substances différentes de formule générale I: 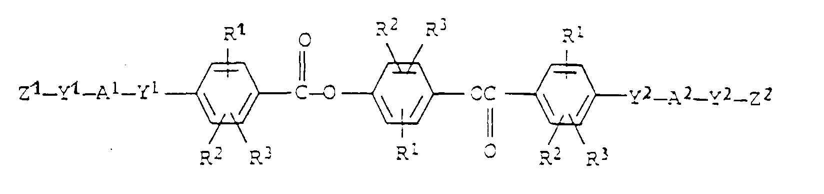 dans laquelle les restes signifient
Z¹, Z² indépendamment l'un de l'autre un groupement polymérisable,
Y¹, Y² à chaque fois indépendamment l'un de l'autre une liaison directe, -O-, -COO-, -OCO- ou -S-,
A¹, A² indépendamment l'un de l'autre un groupement écarteur et
R¹. R² et R³ indépendamment l'un de l'autre un atome d'hydrogène, un groupement alkyle en C₁-C₂₀, alcoxy en C₁-C₂₀, alcoxycarbonyle en C₁-C₂₀, monoalkylaminocarbonyle en C₁-C₂₀, formyle, alkylcarbonyle en C₁-C₂₀, un atome de fluor, de chlore, de brome, un groupement cyano, alkylcarbonyloxy en C₁-C₂₀, alkylcarbonylamino en C₁-C₂₀, hydroxy ou nitro, étant spécifié que les groupements -A¹-Y¹- et -Y²-A²- sont des restes O-alkylène pour au moins une des substances I.

2. Mélanges de cristaux liquides selon la revendication 1, pour lesquels Z¹ et/ou Z² sont un reste de formule CH₂=CH-, CH₂=CCl-, CH₂=C(CH₃)-ou vinylphénylyle.

3. Mélanges de cristaux liquides selon la revendication 1, pour lesquels les restes Y¹ et Y² sont indépendamment l'un de l'autre une liaison directe, -O-, -COO- ou -OCO-.

4. Mélanges de cristaux liquides selon la revendication 1, pour lesquels les restes A¹ et A² sont indépendamment l'un de l'autre des groupements alkylène en C₂-C₂₀ éventuellement interrompus par des atomes d'oxygène en fonction éther ou par des groupements ester, les atomes d'oxygène ou les groupements ester pouvant remplacer dans la chaîne le tiers des atomes de carbone.

5. Mélanges de cristaux liquides selon la revendication 1, pour lesquels R¹, R² et R³ signifient indépendamment les uns des autres, un atome d'hydrogène, un groupement alkyle en C₁-C₁₅, alcoxy en C₁-C₁₅, alcoxycarbonyle en C₁-C₁₅, monoalkylaminocarbonyle en C₁-C₁₅, formyle, alkylcarbonyle en C₁-C₁₅, un atome de fluor, de chlore, de brome, un groupement cyano, alkylcarbonyloxy en C₁-C₁₅, alkylcarbonylamino en C₁-C₁₅, hydroxy ou nitro.

6. Mélanges de cristaux liquides selon la revendication 5, pour lesquels R¹, R² et R³ sont, indépendamment les uns des autres, un atome d'hydrogène, un groupement méthyle, éthyle, alkyle en C₈-C₁₅, méthoxy, éthoxy, alcoxy en C₈-C₁₅, méthoxycarbonyle, éthoxycarbonyle, alcoxycarbonyle en C₈-C₁₅, formyle, acétyle, alkylcarbonyle en C₈-C₁₅, un atome de fluor, de chlore, de brome, un groupement cyano, acétoxy, hydroxy ou nitro.

7. Mélanges de cristaux liquides selon la revendication 6, pour lesquels R¹, R² et R³ sont, indépendamment les uns des autres, un atome d'hydrogène, un groupement méthyle, éthyle, méthoxy, éthoxy, méthoxycarbonyle, formyle, acétyle, un atome de fluor, de chlore, de brome, un groupement cyano, acétoxy, hydroxy ou nitro.

8. Mélanges de cristaux liquides selon la revendication 1, caractérisés en ce qu'ils contiennent jusqu'à 50 %, de préférence de 1 à 30 %, de composés chiraux polymérisables, ces composés chiraux pouvant être des cristaux liquides ou non.

9. Composés de formule générale I selon la revendication 1, pour lesquels les restes R¹ à R³, Z¹, Z², Y¹, Y², A¹ et A² ont les significations mentionnées et les groupements -A¹-Y¹- et -Y²-A² ne sont pas simultanément des restes O-alkylène.

10. Composés selon la revendication 9 pour lesquels les restes A¹ et A² sont différents.

11. Composés selon la revendication 9 pour lesquels les restes Z¹ et Z² sont différents.

12. Composés selon la revendication 9 pour lesquels au moins deux des restes Y¹ et/ou Y² sont différents.

13. Composés selon la formule de la revendication 1, pour lesquels la partie de molécule 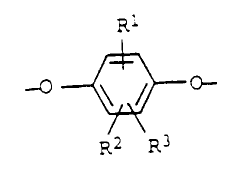 correspond à la formule 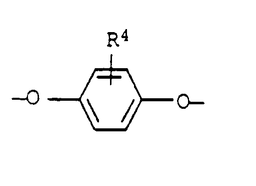 dans laquelle R⁴ est un groupement alkyle en C₂-C₁₅, alcoxy en C₂-C₁₅, alcanoyle en C₂-C₁₅, alcanoyloxy en C₂-C₁₅ ou alcoxycarbonyle en C₂-C₁₅,

14. Composés selon la revendication 13, pour lesquels R⁴ est un groupement alkyle en C₈-C₁₅, alcoxy en C₈-C₁₅, alcanoyle en C₈-C₁₅, alcanoyloxy en C₈-C₁₅ ou alcoxycarbonyle en C₈-C₁₅.

15. Utilisation de mélanges selon la revendication 1 en tant que couches d'orientation pour cristaux liquides, en tant qu'adhésif photoréticulable, en tant que monomères pour la fabrication de cristaux liquides polymères, en tant que matériau de base pour la fabrication de systèmes de cristaux liquides polymérisables pouvant être chiraux, en tant que monomère de matrice polymérisable pour des écrans à polymères dispersés, en tant que matériau de base pour cristaux liquides polymérisables pour éléments de structure optiques.

16. Utilisation de mélanges selon la revendication 1, dotés de composés chiraux polymérisables en tant que matériau de base pour matériau à effet coloré ou pour matériau piézo-électrique.
